# EUROPÄISCHE PATENTSCHRIFT

(11) **EP 1 470 136 B1**
(45) Veröffentlichungstag und Bekanntmachung des Hinweises auf die Patenterteilung: **28.03.2007**
(21) Anmeldenummer: 03704443.5
(22) Anmeldetag: 21.01.2003
(51) Int. Cl.: C07F 9/22, C07B 63/00

(54) **VERFAHREN ZUR ABTRENNUNG VON SÄUREN AUS CHEMISCHEN REAKTIONSGEMISCHEN MIT HILFE VON IONISCHEN FLÜSSIGKEITEN**
METHOD FOR THE SEPARATION OF ACIDS FROM CHEMICAL REACTION MIXTURES BY MEANS OF IONIC FLUIDS
PROCEDE DE SEPARATION D'ACIDES A PARTIR DE MELANGES REACTIONNELS CHIMIQUES AU MOYEN DE LIQUIDES IONIQUES

(30) Priorität: 24.01.2002 DE 10202838; 04.07.2002 DE 10230222; 18.10.2002 DE 10248902; 31.10.2002 DE 10251140
(43) Veröffentlichungstag der Anmeldung: 27.10.2004
(73) Patentinhaber: BASF Aktiengesellschaft, 67056 Ludwigshafen (DE)
(72) Erfinder: VOLLAND, Martin, 69117 Heidelberg (DE); SEITZ, Verena, 67061 Ludwigshafen (DE); MAASE, Matthias, 67346 Speyer (DE); FLORES, Miguel Angel, 28300 Aranjuez (Madrid) (ES); PAPP, Rainer, 67346 Speyer (DE); MASSONNE, Klemens, 67098 Bad Dürkheim (DE); STEGMANN, Veit, 68165 Mannheim (DE); HALBRITTER, Klaus, 69124 Heidelberg (DE); NOE, Ralf, 68163 Mannheim (DE); BARTSCH, Michael, 67433 Neustadt (DE); SIEGEL, Wolfgang, 67117 Limburgerhof (DE); BECKER, Michael, 67063 Ludwigshafen (DE); HUTTENLOCH, Oliver, 68809 Neulussheim (DE)
(86) Internationale Anmeldenummer: PCT/EP2003/000549
(87) Internationale Veröffentlichungsnummer: WO 2003/062251

(56) Entgegenhaltungen:
- EP-A- 1 142 898
- DE-A- 19 724 884
- DE-A- 19 826 936
- US-A- 5 710 344

## Beschreibung

Die vorliegende Erfindung beschreibt ein Verfahren zur vereinfachten Abtrennung von Säuren aus Reaktionsgemischen mittels einer ionischen Flüssigkeit.

Der chemische Fachmann hat oft das Problem, während einer chemischen Reaktion freigesetzte Säuren abzufangen oder Säuren aus Reaktionsgemischen abzutrennen. Beispiele für Reaktionen, in denen Säuren im Reaktionsverlauf freigesetzt werden sind die Silylierung von Alkoholen oder Aminen mit Halogensilanen, die Phosphorylierung von Aminen oder Alkoholen mit Phosphorhalogeniden, die Bildung von Sulfonsäureestem oder -amiden aus Alkoholen oder Aminen und Sulfonsäurechloriden oder - anhydriden, Eliminierungen oder Substitutionen.

Bei diesen Reaktionen werden Säuren freigesetzt, weshalb zusätzlich eine Hilfsbase zugesetzt wird, die in der Regel nicht als Reaktant an der eigentlichen Reaktion teilnimmt. In der Regel ist es notwendig, die freigesetzten Säuren mit dieser Base unter Salzbildung zu binden, um Neben- und Folgereaktionen zu unterbinden oder aber einfach um die Säure aus dem gewünschten Reaktionsprodukt zu entfernen und ggf. in den Prozeß zurückzuführen. Werden die Salze der verwendeten Basen zunächst nicht abgetrennt, so können sie auch in Gegenwart des Wertproduktes, z.B. durch Zugabe einer weiteren, stärkeren Base, wie wäßrigen Laugen, z.B. Natronlauge oder Kalilauge, aufgearbeitet werden. Dabei entsteht das Salz der in diesem Schritt hinzugefügten stärkeren Base. Außerdem wird die ursprünglich verwendete Base in Freiheit gesetzt. Diese beiden Komponenten, d.h. das Salz der stärkeren Base und die in Freiheit gesetzte zuerst verwendete Base (Hilfsbase) müssen in aller Regel ebenfalls vom Wertprodukt abgetrennt werden. Bei dieser Vorgehensweise ist es oft von Nachteil, daß das Wertprodukt, das bei der Aufarbeitung zugegen ist, durch die hinzugefügte stärkere Base selbst oder weitere Stoffe in dieser Base, z.B. dem Wasser in einer wässrigen Lauge, zersetzt werden kann.

Die Salze der Hilfsbase mit der Säure sind in der Regel in organischen Lösemitteln nicht löslich und weisen hohe Schmelzpunkte auf, so daß sie in organischen Medien Suspensionen bilden, die schwieriger zu handhaben sind als beispielsweise Flüssigkeiten. Es wäre also wünschenswert, die Salze der Hilfsbasen in flüssiger Form abtrennen zu können. Zudem würden die bekannten verfahrenstechnischen Nachteile von Suspensionen eliminiert. Diese sind z.B. die Bildung von Verkrustungen, Verringerung des Wärmeüberganges, schlechte Durchmischung und Rührbarkeit sowie die Bildung von lokalen Über- oder Unterkonzentrationen und sogenannten hot spots.

Der Stand der Technik beeinhaltet für industriell durchgeführte Verfahren demnach folgende Nachteile:
1) Zugabe von zwei Hilfsstoffen, der Hilfsbase sowie einer weiteren starken Base und der daraus erwachsenden Aufgabe, zwei Hilfsstoffe vom Wertprodukt und voneinander abzutrennen,
2) Handhabung von Suspensionen
3) Abtrennung des Salzes der starken Base als Feststoff.

Anzustreben ist jedoch eine verfahrenstechnisch einfache Phasentrennung mittels einer flüssig-flüssig-Phasentrennung.

Aus DE-A 197 24 884 und DE-A 198 26 936 sind Verfahren zur Herstellung von Carbonyldümidazolen durch Phosgenierung von Imidazolen bekannt, bei denen das gebildete Hydrochlorid des als Edukt eingesetzten Imidazols als Schmelze aus dem Reaktionsgemisch abgetrennt wird. In der DE-A 198 26 936 wird auf S. 3, Zeile 5 darauf hingewiesen, daß das Hydrochlorid des Imidazols überraschenderweise bei Temperaturen von 110-130°C flüssig ist und bereits deutlich unterhalb des in der Literatur beschriebenen Schmelzpunktes von 158-161 °C schmilzt. Als Grund dafür geben die Erfinder entweder die Bildung eines eutektischen Gemisches aus dem Imidazol Hydrochlorid mit dem Wertprodukt Carbonyldiimidazol oder die Bildung eines ternären Gemisches aus dem Imidazol Hydrochlorid, dem Wertprodukt Carbonyldiimidazol und Lösemittel Chlorbenzol an. Obwohl das Imidazol Hydrochlorid nicht hätte flüssig vorliegen dürfen, war das in diesem speziellen Fall doch überraschenderweise der Fall. Die Anwendbarkeit dieses Konzepts für andere Reaktionen als die Phosgenierung von Imidazolen wird nicht beschrieben.

Es bestand also die Aufgabe, auch für andere chemische Reaktionen oder für die Abtrennung von Säuren, die in Gemischen enthalten sind, aber nicht während einer chemischen Reaktion abgespalten werden, ein vereinfachtes Verfahren zur Abtrennung von Säuren zu finden, bei dem ein aus einer eingesetzten Hilfsbase und einer Säure gebildetes Salz über eine verfahrenstechnisch einfache flüssig-flüssig-Phasentrennung abgetrennt werden kann.

Die Aufgabe wurde erfindungsgemäß gelöst durch ein Verfahren zur Abtrennung von Säuren aus Reaktionsgemischen mittels einer Hilfsbase, in dem die Hilfsbase
b) ein Salz mit der Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet.

Dem Fachmann ist bekannt, daß die Abtrennung einer flüssigen Phase von einer zweiten flüssigen Phase verfahrenstechnisch erheblich einfacher zu gestalten ist als eine Feststoffabtrennung.

Der technische Nutzen des erfindungsgemäßen Verfahrens besteht darin, daß die Abtrennung des Hilfsstoffes durch eine einfache Flüssig-Flüssig-Phasentrennung erfolgen kann, so daß der verfahrenstechnisch aufwendige Umgang mit Feststoffen wegfällt.

Die Aufarbeitung der Hilfsstoffe kann in Abwesenheit des Wertproduktes erfolgen, so daß letzteres weniger belastet wird.

Die Aufgabenstellung wird durch die hier beschriebene Erfindung gelöst. Dies geschieht dadurch, daß Hilfsbasen in Reaktionsgemischen enthalten sind oder nachträglich zugesetzt werden, deren Salze mit im Reaktionsverlauf abgespaltenen oder zugesetzten, d.h. nicht während der Reaktion abgespaltenen Säuren unter den Reaktionsbedingungen und/oder Auf-arbeitungsbedingungen flüssig sind und eine mit dem gegebenenfalls gelösten Wertprodukt nicht mischbare Phase bilden. Derartige flüssige Salze werden oft als ionische Flüssigkeiten bezeichnet. Die zu bindenden Säuren können entweder frei in der Reaktionsmischung vorliegen oder einen Komplex oder ein Addukt mit dem Wertprodukt oder einem anderen in der Reak-tionsmischung anwesenden Stoff bilden. Insbesondere Lewis-Säuren neigen dazu, mit Stoffen wie Ketonen Komplexe zu bilden. Diese Komplexe können durch die Hilfsbase aufgebrochen werden, wobei sich im Sinne dieser Erfindung das Salz aus der Hilfsbase und der abzutrennenden Lewis-Säure bildet.

Die Hilfsbasen können anorganische oder organische Basen sein, bevorzugt organische.

Weiterhin können Gemische oder Lösungen von Hilfsbasen eingesetzt werden, um die Aufgabenstellung zu erfüllen.

Nicht mischbar bedeutet, daß sich mindestens zwei, durch eine Phasengrenzfläche getrennte flüssige Phasen ausbilden.

Wenn das reine Wertprodukt mit dem Salz aus der Hilfsbase und der Säure gänzlich oder zu einem größeren Teil mischbar ist, kann dem Wertprodukt auch ein Hilfsstoff, z.B. ein Lösemittel zugesetzt werden, um eine Entmischung oder Löslichkeitsverringerung zu erreichen. Dies ist beispielsweise dann sinnvoll, wenn die Löslichkeit des Salzes im Wertprodukt oder umgekehrt 20 Gew.-% oder mehr beträgt, bevorzugt 15 Gew.-% oder mehr, besonders bevorzugt 10 Gew-% oder mehr und ganz besonders bevorzugt 5 Gew.-% oder mehr beträgt. Die Löslichkeit wird bestimmt unter den Bedingungen der jeweiligen Trennung. Bevorzugt wird die Löslichkeit bestimmt bei einer Temperatur, die oberhalb des Schmelzpunktes des Salzes liegt und unterhalb der niedrigsten der folgenden Temperaturen, besonders bevorzugt 10 °C unterhalb der niedrigsten und ganz besonders bevorzugt 20 °C unterhalb der niedrigsten:
- Siedepunkt des Wertproduktes
- Siedepunkt des Lösemittels
- Temperatur der signifikanten Zersetzung des Wertproduktes

Das Lösungsmittel ist dann als geeignet anzusehen, wenn das Gemisch aus Wertprodukt und Lösungsmittel das Salz bzw. das Salz das Wertprodukt oder eine Mischung aus Wertprodukt und Lösemittel weniger als die oben angegebenen Mengen zu lösen vermag. Als Lösungsmittel verwendbar sind beispielsweise Benzol, Toluol, o-, m- oder p-Xylol, Mesitylen, Cyclohexan, Cyclopentan, Pentan, Hexan, Heptan, Oktan, Petrolether, Aceton, Isobutylmethylketon, Diethylketon, Diethylether, tert-Butylmethylether, teri-Butylethylether, Tetrahydrofuran, Dioxan, Essigester, Methylacetat, Dimethylformamid, Dimethylsulfoxid, Acetonitril, Chloroform, Dichlormethan, Methylchloroform oder Gemische davon.

Bei dem Wertprodukt handelt es sich in der Regel um unpolare organische oder anorganische Verbindungen.

Als chemische Reaktionen, die der Erfindung zugrundeliegen, kommen alle Reaktion in Betracht, bei denen Säuren freigesetzt werden, mit Ausnahme von Phosgenierungen, besonders bevorzugt mit Ausnahme von Acylierungen, d.h. Reaktionen von Säurehalogeniden und Carbonsäureanhydriden.

Reaktionen, bei denen das erfindungsgemäße Verfahren angewendet werden kann sind beispielsweise
- Alkylierungen mit Alkyl- oder Aralkylhalogeniden, wie z.B. Methylchlorid, Methyliodid, Benzylchlorid, 1,2-Dichlorethan oder 2-Chlorethanol,
- Acylierungen, d.h. Reaktionen von Säurehalogeniden und Carbonsäureanhydriden, von beliebigen Substraten, beispielsweise Alkoholen oder Aminen,
- Silylierungen, also Umsetzungen mit Verbindungen, die mindestens eine Si-Hal-Bindung enthalten, wie z.B. SiCl₄, (H₃C)₂SiCl₂ oder Trimethylsilylchlorid,
- Phosphorylierungen, also Umsetzungen mit Verbindungen, die mindestens eine P-Hal-Bindung enthalten, wie z.B. PCl₃, PCl₅, POCl₃, POBr₃, Dichlorphenylphosphin oder Diphenylchlorphosphin, wie sie beispielsweise ebenfalls von Chojnowski et al., a.a.O. beschrieben sind,
- Sulfurierungen, i.e. Sulfidierungen, Sulfierungen, Sulfonierungen und Sulfatierungen, mit beispielsweise Sulfurylchlorid (SO₂Cl₂), Thionylchlorid (SOCl₂), Chlorsulfonsäure (ClSO₃H), Sulfonsäurehalogeniden, wie z.B. p-Toluolsulfonsäurechlorid, Methansulfonsäurechlorid oder Trifluormethansulfonsäurechlorid, oder Sulfonsäureanhydriden, wie sie z.B. von Dobrynin, V.N. et al. Bioorg. Khim. 9(5), 1983, 706-10 beschrieben ist,
- Eliminierungen, bei denen eine C=C-Doppelbindung unter Abspaltung einer Säure, wie beispielsweise HCl, HBr, Essigsäure oder para-Toluolsulfonsäure, gebildet wird oder
- Deprotonierungen, bei denen ein acides Wasserstoffatom von der Hilfsbase abstrahiert wird.

Bevorzugt unter den genannten Reaktionstypen sind Alkylierungen, Silylierungen, Phosphorylierungen, Sulfurierungen, Acylierungen mit Ausnahme der Phosgenierungen und Eliminierungen und besonders bevorzugt sind Silylierungen, Phosphorylierungen und Sulfurierungen.

Weiterhin kann erfindungsgemäß auch eine Säure aus Reaktionsmischungen abgetrennt werden, bei denen eine Säure, die nicht während der Reaktion freigesetzt wurde, hinzugefügt wurde, beispielsweise um den pH-Wert einzustellen oder um eine Reaktion zu katalysieren. So können z.B. Lewis-Säuren, die als Katalysatoren für Friedel-Crafts-Alkylierungen oder -Acylierungen eingesetzt wurden, auf einfache Art abgetrennt werden.

Die im Sinne dieser Erfindung abzutrennenden Säuren können Brönstedsäuren und Lewis-Säuren sein. Welche Säuren als Brönsted und Lewissäuren bezeichnet werden, wird in Hollemann-Wiberg, Lehrbuch der Anorganischen Chemie, 91.-100. Auflage, Walter de Gruyter, Berlin New York 1985, S. 235 bzw. S. 239 beschrieben. Zu den Lewissäuren im Sinne dieser Erfindung zählen auch die als Friedel-Crafts-Katalysatoren verwendeten Lewissäuren, die in George A. Olah, Friedel-Crafts and Related Reactions, Vol. I, 191 bis 197, 201 und 284-90 (1963) beschrieben sind. Als Beispiele genannt seien Aluminiumtrichlorid (AlCl₃), Eisen(III)chlorid (FeCl₃), Aluminiumtribromid (AIBr₃) und Zinkchlorid (ZnCl₂).

Allgemein enthalten die erfindungsgemäß abtrennbaren Lewis-Säuren kationische Formen der Metalle der Gruppen Ib, IIb, IIIa, IIIb, IVa, IVb, Va, Vb, Vlb, VIIb und VIII des Periodensystems der Elemente sowie der seltenen Erden, wie beispielsweise Lanthan, Cer, Praseodym, Neodym, Samarium, Europium, Gadolinium, Terbium, Dysprosium, Holmium, Erbium, Thulium, Ytterbium oder Lutetium.

Genannt seien besonders Zink, Cadmium, Beryllium, Bor, Aluminium, Gallium, Indium, Thallium, Titan, Zirkon, Hafnium, Erbium, Germanium, Zinn, Vanadium, Niob, Skandium, Yttrium, Chrom, Molybden, Wolfram, Mangan, Rhenium, Palladium, Thorium, Eisen, Kupfer und Kobalt. Bevorzugt sind Bor, Zink, Cadmium, Titan, Zinn, Eisen, Kobalt.

Als Gegenionen der Lewis-Säure kommen in Frage F⁻, Cl⁻, ClO⁻, ClO₃⁻, ClO₄⁻, Br⁻, J⁻, JO₃⁻, CN⁻, OCN⁻, SCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, Dithiocarbamat, Salicylat, (OCₙH₂ₙ₊₁)⁻, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht, Methansulfonat (CH₃SO₃⁻), Trifluormethansulfonat (CF₃SO₃⁻). Toluolsulfonat (CH₃C₆H₄SO₃⁻), Benzolsulfonat (C₆H₅SO₃⁻), Hydroxid (OH⁻), Anionen aromatischer Säuren wie Benzoesäure, Phthalsäure, und dergleichen und 1,3-Dicarbonylverbindungen.

Weiterhin genannt seien Carboxylate, insbesondere sind zu erwähnen Formiat, Acetat, Trifluoracetat, Propionat, Hexanoat und 2-Ethylhexanoat, Stearat sowie Oxalat, Acetylacetonat, Tartrat, Acrylat und Methacrylat, bevorzugt Formiat, Acetat, Propionat, Oxalat, Acetylacetonat, Acrylat und Methacrylat.

Weiterhin kommen Borhydride und Organoborverbindungen der allgemeinen Formel BR""₃ und B(OR"")₃ in Betracht, worin R"" jeweils unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte lminogruppen unterbrochenes C₂ - C₁₈-Alkyl, C₆- C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein kann. Die Reste R"" können auch miteinander verbunden sein.

Als bevorzugte Beispiele für Lewis-Säuren seien neben den oben angeführten AlCl₃, FeCl₃, AlBr₃ und ZnCl₂ genannt BeCl₂, ZnBr₂, Znl₂, ZnSO₄, CuCl₂, CuCl, Cu(O₃SCF₃)₂, CoCl₂, Col₂, Fel₂, FeCl₂, FeCl₂(THF)₂, TiCl₄(THF)₂, TiCl₄, TiCl₃, CITi(OiPr)₃, SnCl₂, SnCl₄, Sn(SO₄), Sn(SO₄)₂, MnCl₂, MnBr₂, ScCl₃, BPh₃, BCl₃, BBr₃, BF₃•OEt₂, BF₃•OMe₂, BF₃•MeOH, BF₃•CH₃COOH, BF₃•CH₃CN, B(CF₃COO)₃, B(OEt)₃, B(OMe)₃, B(O*i*Pr)₃, PhB(OH)₂, 3-MeO-PhB(OH)₂, 4-MeO-PhB(OH)₂, 3-F-PhB(OH)₂, 4-F-PhB(OH)₂, (C₂H₅)₃Al, (C₂H₅)₂AlCl, (C₂H₅)AlCl₂, (C₈H₁₇)AlCl₂, (C₈H₁₇)₂AlCl, (iso-C₄H₉)₂AlCl, Ph₂AlCl, PhAlCl₂, Al(acac)₃, Al(O*i*Pr)₃, Al(O*n*Bu)₃, Al(O*sek*Bu)₃, Al(OEt)₃, GaCl₃, ReCl₅, ZrCl₄, NbCl₅, VCl₃, CrCl₂, MoCl₅, YCl₃, CdCl₂, CdBr₂, SbCl₃, SbCl₅, BiCl₃, ZrCl₄, UCl₄, LaCl₃, CeCl₃, Er(O₃SCF₃), Yb(O₂CCF₃)₃, SmCl₃, Sml₂, B(C₆H₅)₃, TaCl₅.

Die Lewis-Säuren können stabilisiert sein durch Alkali- oder Erdalkalimetallhalogenide, beispielsweise LiCl oder NaCl. Dazu werden die (Erd)Alkalimetallhalogenide zur Lewis-Säure im molaren Verhältnis 0 - 100 : 1 gemischt.

Mit Halogen oder Hal ist im Rahmen dieser Schrift Fluor (F), Chlor (Cl), Brom (Br) oder lod (I), bevorzugt Chlor gemeint.

Umgesetzt im Sinne einer Silylierung, Phosphorylierung oder Sulfurierung werden in der Regel Verbindungen, die mindestens eine freie O-H-,. S-H- oder N-H-Bindung aufweisen, gegebenenfalls nach Deprotonierung durch die Hilfsbase.

Als Hilfsbase kann erfindungsgemäß eine solche Verbindung eingesetzt werden, die
b) ein Salz mit der während der Reaktion abgespaltenen Säure bildet, das bei Temperaturen flüssig ist, bei denen das Wertprodukt während der Abtrennung des Flüssigsalzes im wesentlichen nicht zersetzt wird und
c) das Salz der Hilfsbase mit dem Wertprodukt oder der Lösung des Wertproduktes in einem geeigneten Lösungsmittel zwei nicht mischbare flüssige Phasen ausbildet.

Bevorzugt sind solche Hilfsbasen, die
a) nicht als Reaktant an der Reaktion teilnehmen.
   Weiterhin bevorzugt kann diese Hilfsbase zusätzlich
d) gleichzeitig als nucleophiler Katalysator in der Reaktion fungieren, d.h. sie erhöht die Reaktionsgeschwindigkeit der Reaktion gegenüber der Durchführung ohne Anwesenheit einer Hilfsbase um das mindestens 1,5fache, bevorzugt um das mindestens zweifache, besonders bevorzugt um das fünffache, ganz besonders bevorzugt um das mindestens zehnfache und insbesondere um das mindestens zwanzigfache.

Solche als Basen einsetzbaren Verbindungen können Phosphor- Schwefel oder Stickstoffatome enthalten, beispielsweise mindestens ein Stickstoffatom, bevorzugt ein bis zehn Stickstoffatome, besonders bevorzugt ein bis fünf, ganz besonders bevorzugt ein bis drei und insbesondere ein bis zwei Stickstoffatome. Gegebenenfalls können auch weitere Heteroatome, wie z.B. Sauerstoff-, Schwefel- oder Phosphoratome enthalten sein.

Bevorzugt sind solche Verbindungen, die mindestens einen fünf- bis sechsgliedrigen Heterocyclus enthalten, der mindestens ein Stickstoffatom sowie gegebenenfalls ein Sauerstoff- oder Schwefelatom aufweist, besonders bevorzugt solche Verbindungen, die mindestens einen fünf- bis sechsgliedrigen Heterocyclus enthalten, der ein, zwei oder drei Stickstoffatome und ein Schwefel- oder ein Sauerstoffatom aufweist, ganz besonders bevorzugt solche mit zwei Stickstoffatomen.

Besonders bevorzugte Verbindungen sind solche, die ein Molgewicht unter 1000 g/mol aufweisen, ganz besonders bevorzugt unter 500 g/mol und insbesondere unter 250 g/mol.

Weiterhin sind solche als Basen einsetzbaren Verbindungen bevorzugt, die ausgewählt sind aus den Verbindungen der Formeln (la) bis (Ir), 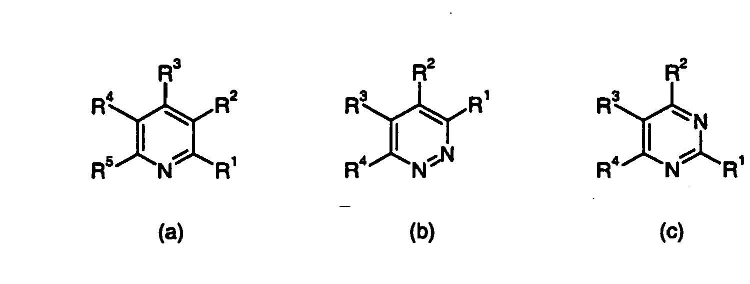 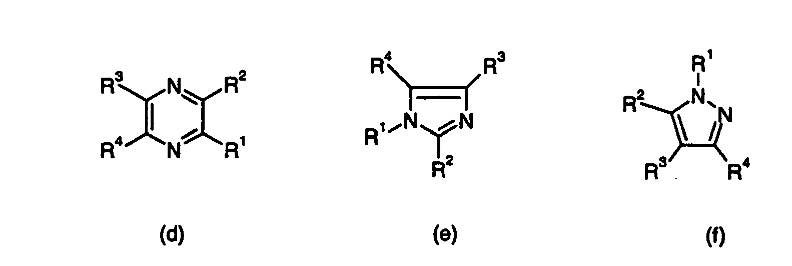 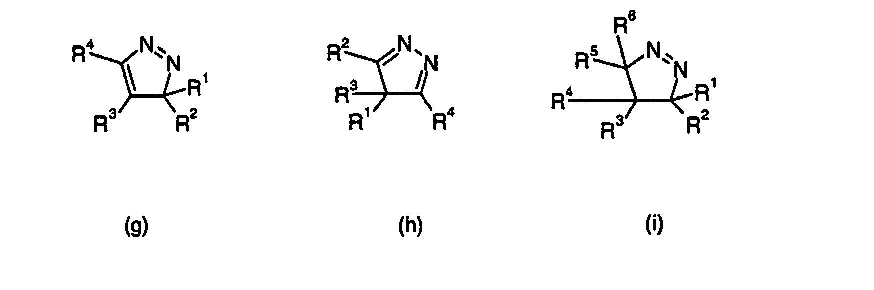 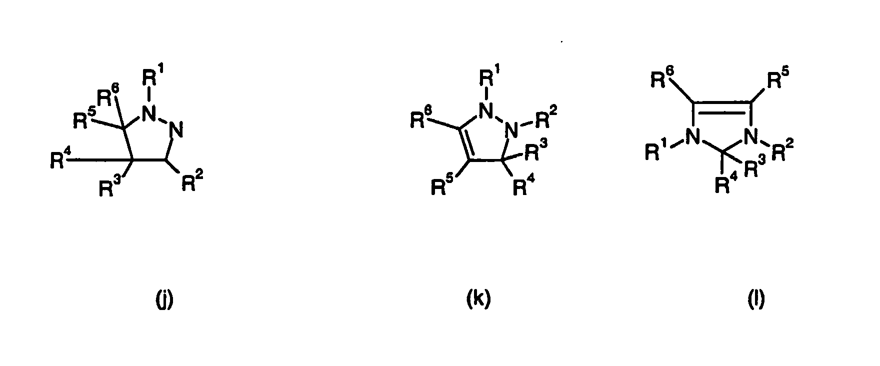 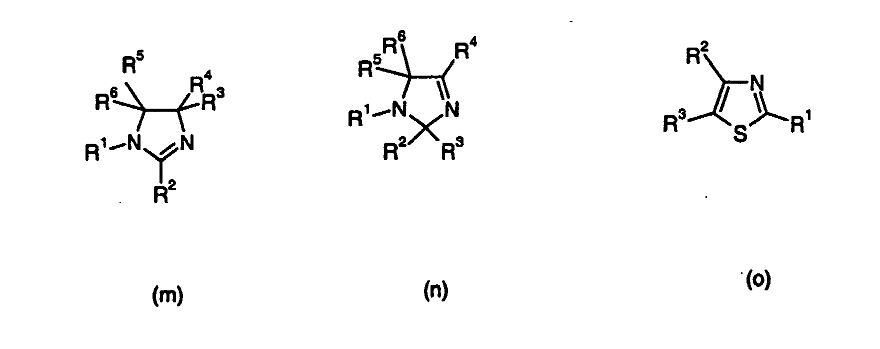 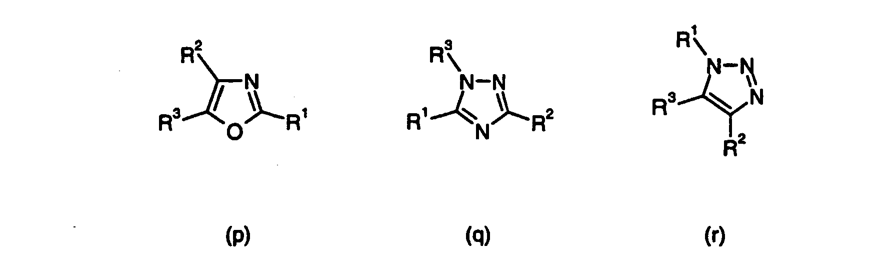 sowie Oligo- bzw. Polymere, die diese Strukturen enthalten,
worin

R¹, R², R³, R⁴, R⁵ und R⁶ unabhängig voneinander jeweils Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₈ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

Darin bedeuten
gegebenenfalls durch funktionelle Gruppen, Aryl, Alkyl, Aryloxy, Alkyloxy, Halogen, Heteroatome und/oder Heterocyclen substituiertes C₁ - C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und,
gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befinden sich zwischen zwei Heteroatomen in der Regel mindestens ein Kohlenstoffatom, bevorzugt mindestens zwei.

Substituierte und unsubstituierte Iminogruppen können beispielsweise Imino-, Methylimino-, *iso*-Propylimino, n-Butylimino oder *tert*-Butylimino sein.

Weiterhin bedeuten
funktionelle Gruppen Carboxy, Carboxamid, Hydroxy, Di-(C₁-C₄-Alkyl)-amino, C₁-C₄-Alkyloxycarbonyl, Cyano oder C₁-C₄-Alkyloxy,
gegebenenfalls durch funktionelle Gruppen, Aryl, Alkyl, Aryloxy, Alkyloxy, Halogen, Heteroatome und/oder Heterocyclen substituiertes C₆ - C₁₂-Aryl beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch funktionelle Gruppen, Aryl, Alkyl, Aryloxy, Alkyloxy, Halogen, Heteroatome und/oder Heterocyclen substituiertes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,
ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, 1-sopropylthiophenyl oder tert.-Butylthiophenyl und
C₁ bis C₄-Alkyl beispielsweise Methyl, Ethyl, Propyl, lsopropyl, n-Butyl, sec-Butyl oder tert.-Butyl.

Bevorzugt sind R¹, R², R³, R⁴, R⁵ und R⁶ unabhängig voneinander Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino und Chlor.

Besonders bevorzugte Pyridine (Ia) sind solche, bei denen einer der Reste R¹ bis R⁵ Methyl, Ethyl oder Chlor ist und alle anderen Wasserstoff sind, oder R³ Dimethylamino und alle anderen Wasserstoff sind oder alle Wasserstoff sind oder R² Carboxy oder Carboxamid und alle anderen Wasserstoff oder R¹ und R² oder R² und R³ 1,4-Buta-1,3-dienylen und alle anderen Wasserstoff sind.

Besonders bevorzugte Pyridazine (Ib) sind solche, bei denen einer der Reste R¹ bis R⁴ Methyl oder Ethyl und alle anderen Wasserstoff oder alle Wasserstoff sind.

Besonders bevorzugte Pyrimidine (lc) sind solche, bei denen R² bis R⁴ Wasserstoff oder Methyl und R¹ Wasserstoff, Methyl oder Ethyl ist, oder R² und R⁴ Methyl, R³ Wasserstoff und R¹ Wasserstoff, Methyl oder Ethyl ist.

Besonders bevorzugte Pyrazine (Id) sind solche, bei denen R¹ bis R⁴ alle Methyl oder alle Wasserstoff sind.

Besonders bevorzugte Imidazole (le) sind solche, bei denen unabhängig voneinander
R¹ ausgewählt ist unter Methyl, Ethyl, n-Propyl, n-Butyl, n-Pentyl, n-Octyl, 2-Hydroxyethyl oder 2-Cyanoethyl und
R² bis R⁴ unabhängig voneinander Wasserstoff, Methyl oder Ethyl bedeuten.

Besonders bevorzugte 1 H-Pyrazole (If) sind solche, bei denen unabhängig voneinander
R¹ unter Wasserstoff, Methyl oder Ethyl,
R², R³ und R⁴ unter Wasserstoff oder Methyl
ausgewählt sind.

Besonders bevorzugte 3H-Pyrazole (Ig) sind solche, bei denen unabhängig voneinander
R¹ unter Wasserstoff, Methyl oder Ethyl,
R², R³ und R⁴ unter Wasserstoff oder Methyl
ausgewählt sind.

Besonders bevorzugte 4H-Pyrazole (1h) sind solche, bei denen unabhängig voneinander
R¹ bis R⁴ unter Wasserstoff oder Methyl,
ausgewählt sind.

Besonders bevorzugte 1-Pyrazoline (li) sind solche, bei denen unabhängig voneinander
R¹ bis R⁶ unter Wasserstoff oder Methyl
ausgewählt sind.

Besonders bevorzugte 2-Pyrazoline (Ij) sind solche, bei denen unabhängig voneinander
R¹ unter Wasserstoff, Methyl, Ethyl oder Phenyl und
R² bis R⁶ unter Wasserstoff oder Methyl
ausgewählt sind.

Besonders bevorzugte 3-Pyrazoline (Ik) sind solche, bei denen unabhängig voneinander
R¹ oder R² unter Wasserstoff, Methyl, Ethyl oder Phenyl und
R³ bis R⁶ unter Wasserstoff oder Methyl
ausgewählt sind.

### Besonders bevorzugte Imidazoline (II) sind solche, bei denen unabhängig voneinander

R¹ oder R² unter Wasserstoff, Methyl, Ethyl, n-Butyl oder Phenyl und
R³ oder R⁴ unter Wasserstoff, Methyl oder Ethyl und
R⁵ oder R⁶ unter Wasserstoff oder Methyl
ausgewählt sind.

Besonders bevorzugte Imidazoline (Im) sind solche, bei denen unabhängig voneinander
R¹ oder R² unter Wasserstoff, Methyl oder Ethyl und
R³ bis R⁶ unter Wasserstoff oder Methyl
ausgewählt sind.

Besonders bevorzugte Imidazoline (In) sind solche, bei denen unabhängig voneinander
R¹, R² oder R³ unter Wasserstoff, Methyl oder Ethyl und
R⁴ bis R⁶ unter Wasserstoff oder Methyl
ausgewählt sind.

Besonders bevorzugte Thiazole (Io) oder Oxazole (Ip) sind solche, bei denen unabhängig voneinander
R¹ unter Wasserstoff, Methyl, Ethyl oder Phenyl und
R² oder R³ unter Wasserstoff oder Methyl
ausgewählt sind.

Besonders bevorzugte 1,2,4-Triazole (Iq) sind solche, bei denen unabhängig voneinander
R¹ oder R² unter Wasserstoff, Methyl, Ethyl oder Phenyl und
R³ unter Wasserstoff, Methyl oder Phenyl
ausgewählt sind.

Besonders bevorzugte 1,2,3-Triazole (Ir) sind solche, bei denen unabhängig voneinander
R¹ unter Wasserstoff, Methyl oder Ethyl und
R² oder R³ unter Wasserstoff oder Methyl ausgewählt sind oder
R² und R³ 1,4-Buta-1,3-dienylen und alle anderen Wasserstoff sind.

Unter diesen sind die Pyridine und die Imidazole bevorzugt.

Ganz besonders bevorzugt sind als Basen 3-Chlorpyridin, 4-Dimethylaminopyridin, 2-Ethyl-4-aminopyridin, 2-Methylpyridin (α-Picolin), 3-Methylpyridin (β-Picolin), 4-Methylpyridin (γ-Picolin), 2-Ethylpyridin, 2-Ethyl-6-methylpyridin, Chinolin, Isochinolin, 1-C₁-C₄-Alkylimidazol, 1-Methylimidazol, 1,2-Dimethylimidazol, 1-n-Butylimidazol, 1,4,5-Trimethylimidazol, 1,4-Dimethylimidazol, Imidazol, 2-Methylimidazol, 1-Butyl-2-methylimidazol, 4-Methylimidazol, 1-n-Pentylimidazol, 1-n-Hexylimidazol, 1-n-Octylimidazol, 1-(2'-Aminoethyl)-imidazol, 2-Ethyl-4-methylimidazol, 1-Vinylimidazol, 2-Ethylimidazol, 1-(2'-Cyanoethyl)-imidazol und Benzotriazol.

Insbesondere bevorzugt sind 1-n-Butylimidazol, 1-Methylimidazol, 2-Methylpyridin und 2-Ethylpyridin.

Weiterhin geeignet sind tertiäre Amine der Formel (XI),

NR^{a}R^{b}R^{c} (XI),

worin

R^{a}, R^{b} und R^{c} unabhängig voneinander jeweils C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₆ - C₁₂-Aryl oder C₅ - C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können, mit der Maßgabe, dass
- mindestens zwei der drei Reste R^{a}, R^{b} und R^{c} unterschiedlich sind und
- die Reste R^{a}, R^{b} und R^{c} zusammen mindestens 8, bevorzugt mindestens 10, besonders bevorzugt mindestens 12 und ganz besonders bevorzugt mindestens 13 Kohlenstoffatome aufweisen.

Bevorzugt sind R^{a}, R^{b} und R^{c} unabhängig voneinander jeweils C₁-C₁₈-Alkyl, C₆-C₁₂-Aryl oder C₅-C₁₂-Cycloalkyl und besonders bevorzugt C₁-C₁₈-Alkyl, wobei die genannten Reste jeweils durch funktionelle Gruppen, Aryl, Alkyl, Aryloxy, Alkyloxy, Halogen, Heteroatome und/oder Heterocyclen substituiert sein können.

Beispiele für die jeweiligen Gruppen sind bereits oben aufgeführt.

Bevorzugte Bedeutungen für die Reste R^{a}, R^{b} und R^{c} sind Methyl, Ethyl, n-Propyl, lsopropyl, n-Butyl, sec-Butyl, tert.-Butyl, n-Pentyl (n-Amyl), 2-Pentyl (sek-Amyl), 3-Pentyl, 2,2-Dimethyl-prop-1-yl (neo-Pentyl), n-Hexyl, n-Heptyl, n-Octyl, iso-Octyl, 2-Etylhexyl, 1,1-Dimethylpropyl, 1,1-Dimethylbutyl, Benzyl, 1-Phenylethyl, 2-Phenylethyl, α,α -Dimethylbenzyl, Phenyl, Tolyl, Xylyl, α-Naphthyl, β-Naphthyl, Cyclopentyl oder Cyclohexyl.

Bilden zwei der Reste R^{a}, R^{b} und R^{c} eine Kette, so kann dies beispielsweise 1,4-Butylen oder 1,5-Pentylen sein.

Beispiele für die tertiären Amine der Formel (XI) sind Diethyl-n-butylamin, Diethyl-tert-butylamin, Diethyl-n-pentylamin, Diethyl-hexylamin, Diethyl-octylamin, Diethyl-(2-ethylhexyl)-amin, Di-n-propyl-butylamin, Di-n-propyl-n-pentylamin, Di-n-propyl-hexylamin, Di-n-propyl-octylamin, Di-n-propyl-(2-ethylhexyl)-amin, Di-iso-propyl-ethylamin, Di-iso-propyl-n-propylamin, Di-iso-propyl-butylamin, Di-iso-propyl-pentylamin, Di-iso-propyl-hexylamin, Di-iso-propyl-octylamin, Di-iso-propyl-(2-ethylhexyl)-amin,Di-n-butyl-ethylamin, Di-n-butyl-n-propylamin, Di-n-butyl-n-pentylamin, Di-n-butyl-hexylamin, Di-n-butyl-octylamin, Di-n-butyl-(2-ethylhexyl)-amin, N-n-Butyl-pyrrolidin, N-sek-Butyl-pyrrolidin, N-tert-Butyl-pyrrolidin, N-n-Pentyl-pyrrolidin, N,N-Dimethylcyclohexylamin, N,N-Diethylcyclohexylamin, N,N-Di-n-butylcyclohexylamin, N-n-Propyl-piperidin, N-iso-Propyl-piperidin, N-n-Butyl-piperidin, N-sek-Butyl-piperidin, N-tert-Butyl-piperidin, N-n-Pentyl-piperidin, N-n-Butylmorpholin, N-sek-Butylmorpholin, N-tert-Butylmorpholin, N-n-Pentylmorpholin, N-Benzyl-N-ethyl-anilin, N-Benzyl-N-n-propyl-anilin, N-Benzyl-N-iso-propyl-anilin, N-Benzyl-N-n-butyl-anilin, N,N-Dimethyl-p-toluidin, N,N-Diethyl-p-toluidin, N,N-Di-n-butyl-p-toluidin, Diethylbenzylamin, Di-n-propylbenzylamin, Di-n-butylbenzylamin, Diethylphenylamin, Di-n-propylphenylamin und Di-n-butylphenylamin sowie 1,5-Diazabicyclo[4.3.0]-non-5-en (DBN).

Bevorzugte tertiäre Amine (XI) sind Di-iso-propyl-ethylamin, Diethyl-tert-butylamin, Di-iso-propyl-butylamin, Di-n-butyl-n-pentylamin, N,N-Di-n-butylcyclohexylamin sowie tertiäre Amine aus Pentylisomeren.

Besonders bevorzugte tertiäre Amine sind Di-n-butyl-n-pentylamin und tertiäre Amine aus Pentylisomeren.

Ein tertiäres Amin, das ebenfalls bevorzugt und erfindungsgemäß einsetzbar ist, jedoch im Gegensatz zu den oben angeführten drei identische Reste aufweist, ist Triallylamin.

Tertiäre Amine, bevorzugt der Formel (XI), sind gegenüber heterocyclischen Verbindungen, beispielsweise der Formeln (la) bis (Ir), in der Regel dann bevorzugt, wenn die Basizität letzterer Hilfsbasen für die Reaktion nicht ausreichend ist, beispielsweise für Eliminierungen.

Säuren, mit denen die Basen Salze bilden können sind beispielsweise lodwasserstoffsäure (HI), Fluorwasserstoff (HF), Chlorwasserstoff (HCl), Salpetersäure (HNO₃), salpetrige Säure (HNO₂), Bromwasserstoffsäure (HBr), Kohlensäure (H₂CO₃), Hydrogencarbonat (HC03'), Methylkohlensäure (HO(CO)OCH₃), Ethylkohlensäure (HO(CO)OC₂H₅), n-Butylkohlensäure, Schwefelsäure (H₂SO₄), Hydrogensulfat (HSO₄⁻), Methylschwefelsäure (HO(SO₂)OCH₃), Ethylschwefelsäure (HO(SO₂)OC₂H₅), Phosphorsäure (H₃PO₄), Dihydrogenphosphat (H₂PO₄), Ameisensäure (HCOOH), Essigsäure (CH₃COOH), Propionsäure, n- und iso-Buttersäure, Pivalinsäure, para-Toluolsulfonsäure, Benzolsulfonsäure, Benzoesäure, 2,4,6-Trimethylbenzoesäure, Mandelsäure, Methansulfonsäure, Ethansulfonsäure oder Trifluormethansulfonsäure, bevorzugt sind Chlorwasserstoff, Essigsäure, p-Toluolsulfonsäure, Methansulfonsäure, 2,4,6-Trimethylbenzoesäure und Trifluormethansulfonsäure und besonders bevorzugt ist Chlorwasserstoff.

In einer bevorzugten Ausführungsform zur Abtrennung von Brönsted-Säuren (Protonensäuren) werden diese ohne große Anteile von Lewis-Säuren abgetrennt, d.h. im abgetrennten Salz der Säure mit der Hilfsbase ist das molare Verhältnis von BrönstedSäuren zu Lewis-Säuren größer als 4:1, bevorzugt größer als 5:1, besonders bevorzugt größer als 7:1, ganz besonders bevorzugt größer als 9:1 und insbesondere größer als 20:1.

Bevorzugt sind solche Hilfsbasen, deren Salze aus Hilfsbasen und Säuren, eine Schmelztemperatur aufweisen, bei der im Zuge der Abtrennung des Salzes als flüssige Phase keine signifikante Zersetzung des Wertproduktes auftritt, d.h. weniger als 10 Mol% pro Stunde, bevorzugt weniger als 5 Mol %/h, besonders bevorzugt weniger als 2 Mol %/h und ganz besonders bevorzugt weniger als 1 Mol %/h.

Die Schmelzpunkte der Salze der besonders bevorzugten Hilfsbasen liegen in der Regel unterhalb von 160 C, besonders bevorzugt unterhalb von 100 C und ganz besonders bevorzugt unterhalb von 80°C.

Unter den Hilfsbasen sind solche ganz besonders bevorzugt, deren Salze einen E_{T}(30)-Wert von> 35, bevorzugt von >40, besonders bevorzugt von> 42 aufweisen. Der E_{T}(30)-Wert ist ein Maß für die Polarität und wird von C. Reichardt in Reichardt, Christian Solvent Effects in Organic Chemistry Weinheim : VCH, 1979. - XI, (Monographs in Modern Chemistry; 3), ISBN 3-527-25793-4 Seite 241 beschrieben.

Eine außergewöhnlich bevorzugte Base, welche die Aufgabenstellung z.B. erfüllt, ist 1-Methylimidazol. Die Verwendung von 1-Methylimidazol als Base wird z.B. in DE-A 35 02 106 erwähnt, jedoch wird dort nicht deren Verwendbarkeit als ionische Flüssigkeit erkannt.

1-Methylimidazol ist zudem noch als nucleophiler Katalysator wirksam [Julian Chojnowski, Marek Cypryk, Witold Fortuniak, Heteroatom. Chemistry, 1991, 2, 63-70]. Chojnowski et al. haben gefunden, daß 1-Methylimidazol im Vergleich zu Triethylamin die Phosphorylierung von *t*-Butanol um den Faktor 33 und die Silylierung von Pentamethyldisiloxanol um den Faktor 930 beschleunigt.

Es wurde weiterhin gefunden, daß das Hydrochlorid von 1-Methylimidazol einen Schmelzpunkt von etwa 75°C aufweist und mit unpolaren organischen Wertprodukten, wie z.B. Diethoxyphenylphosphin, Triethylphosphit, Ethoxydiphenylphosphin, Alkylketendimer, Alkoxysilane oder Ester, oder Lösemitteln im wesentlichen nicht mischbar ist. So bildet 1-Methylimidazol•HCl im Gegensatz zu dem polaren Lösemittel Wasser sogar mit Aceton zwei nicht mischbare Phasen aus. 1-Methylimidazol kann zugleich als Hilfsbase und nucleophiler Katalysator dienen und als flüssiges. Hydrochlorid über eine verfahrenstechnisch einfache Flüssig-Flüssig-Phasentrennung von organischen Medien abgetrennt werden.

Statt 1-Methylimidazol kann auch 1-Butylimidazol verwendet werden. Das Hydrochlorid des 1-Butylimidazols ist bereits bei Raumtemperatur flüssig, so daß 1-Butylimidazol als Hilfsbase und Katalysator für Reaktionen verwendet werden kann, bei denen Stoffe gehandhabt werden, die bei Temperaturen oberhalb der Raumtemperatur bereits zersetzlich sind. Ebenfalls bei Raumtemperatur flüssig ist das Acetat und Formiat von 1-Methylimidazol.

Ebenso können alle Derivate des Imidazols verwendet werden, deren Salze einen E_{T}(30)-Wert von > 35, bevorzugt von >40, besonders bevorzugt von > 42 aufweisen und eine Schmelztemperatur haben, bei der im Zuge der Abtrennung des Salzes als flüssige Phase keine signifikante Zersetzung des Wertproduktes auftritt. Die polaren Salze dieser Imidazole bilden wie oben angeführt mit weniger polaren organischen Medien zwei nicht mischbare Phasen aus.

Eine weitere außergewöhnlich bevorzugte Base, die die Aufgabenstellung erfüllt, ist 2-Ethylpyridin. Die Verwendung verschiedener Pyridine als Hilfsbase wird z.B. in DE 198 50 624 beschrieben, jedoch wird dort nicht deren Verwendbarkeit als ionische Flüssigkeit erkannt.

Pyridin selbst und Derivate des Pyridin sind dem Fachmann als nucleophile Katalysatoren bekannt [Jerry March, "Advanced Organic Chemistry, 3^{rd} Edition, John Wiley & Sons, New York 1985, S. 294, 334, 347].

Es wurde weiterhin gefunden, daß das Hydrochlorid von 2-Ethylpyridin einen Schmelzpunkt von etwa 55°C aufweist und mit unpolaren organischen Wertprodukten (s.o.) oder Lösemitteln nicht mischbar ist. 2-Ethylpyridin kann also zugleich als Hilfsbase und nucleophiler Katalysator dienen und als flüssiges Hydrochlorid über eine verfahrenstechnisch einfache Flüssig-Flüssig-Phasentrennung von organischen Medien abgetrennt werden.

Ebenso können alle Derivate des Pyridins verwendet werden, deren Salze einen E_{T}(30)-Wert von > 35, bevorzugt von >40, besonders bevorzugt von > 42 aufweisen und eine Schmelztemperatur haben, bei der im Zuge der Abtrennung des Salzes als flüssige Phase keine signifikante Zersetzung des Wertproduktes auftritt. Die polaren Salze dieser Pyridine bilden mit weniger polaren organischen Medien zwei nicht mischbare Phasen aus.

Die Durchführung der Reaktion ist nicht beschränkt und kann erfindungsgemäß unter Abfangen der freigesetzten oder zugesetzten Säuren, gegebenenfalls unter nucleophiler Katalyse, diskontinuierlich oder kontinuierlich und an Luft oder unter einer Schutzgasatmosphäre durchgeführt werden.

Bei temperaturempfindlichen Wertprodukten kann es ausreichend sein, das Salz aus Hilfsbase und Säure als festes Salz während der Reaktion ausfallen zu lassen und erst zur Aufarbeitung oder nach Abtrennung der Hauptmenge des Wertproduktes in einer fest-flüssig-Trennung aufzuschmelzen. Das Produkt wird dadurch thermisch weniger belastet.

Ein weiterer Gegenstand der Erfindung ist ein Verfahren zur Abtrennung der oben angeführten Hilfsbasen oder Hilfsbasen, die als nucleophile Katalysatoren verwendet werden, aus einem Reaktionsgemisch, indem man das Reaktionsgemisch pro mol Hilfsbase mit mindestens einem mol Säure versetzt. Dadurch wird die Abtrennung solcher Hilfsbasen als ionische Flüssigkeiten mit Hilfe einer flüssig-flüssig-Trennung möglich.

Aus dem vom Wertprodukt abgetrennten Salz der Hilfsbase kann nach dem Fachmann bekannter Art und Weise die freie Base wiedergewonnen und in den Prozeß zurückgeführt werden.

Dies kann beispielsweise erfolgen, indem man das Salz der Hilfsbase mit einer starken Base, z.B. NaOH, KOH, Ca(OH)₂, Kalkmilch, Na₂CO₃, NaHCO₃, K₂CO₃, oder KHCO₃, gegebenenfalls in einem Lösungsmittel, wie z.B. Wasser, Methanol, Ethanol, n- oder *iso*-Propanol, n-Butanol, n-Pentanol oder Butanol- oder Pentanol-Isomerengemische oder Aceton, freisetzt. Die so freigesetzte Hilfsbase kann , wenn sie eine eigene Phase ausbildet abgetrennt oder falls sie mit dem Salz der stärkeren Base bzw. der Lösung des Salzes der stärkeren Base mischbar ist, durch Destillation aus der Mischung abgetrennt werden. Falls erforderlich kann man die freigesetzte Hilfsbase auch vom Salz der stärkeren Base bzw. der Lösung des Salzes der stärkeren Base durch Extraktion mit einem Extraktionsmittel abtrennen. Extraktionsmittel sind z.B. Lösemittel, Alkohole oder Amine.

Falls erforderlich kann die Hilfsbase mit Wasser oder wäßriger NaCl oder Na₂SO₄-Lösung gewaschen und anschließend getrocknet werden, z.B. durch Abtrennung von gegebenenfalls enthaltenem Wasser mit Hilfe einer Azeotropdestillation mit Benzol, Toluol, Xylol Butanol oder Cyclohexan.

Falls erforderlich, kann die Base vor erneuter Verwendung destilliert werden.

Eine weitere Möglichkeit der Rückführung ist, das Salz der Hilfsbase zu destillieren, wobei das Salz thermisch in seine Ausgangsstoffe, d.h. die freie Base und die abgefangene Säure gespalten wird. Der leichter siedende Anteil des Salzes wird abdestilliert, während der höhersiedende im Sumpf verbleibt. Die freie Hilfsbase ist dabei entweder der Leicht- oder Hochsieder. Auf diese Weise kann z.B. 1-Butylimidazolformiat destillativ in Ameisensäure (Kopfprodukt) und 1-Butylimidazol (Sumpfprodukt) getrennt werden, wie in der EP-A 181 078 beschrieben.

Eine bevorzugte Ausführungsform besteht darin, aus einem Reaktionsgemisch das Wertprodukt in Anwesenheit der Hilfsbase in der protonierten Form abzudestillieren und anschließend, nach weitgehender Entfernung des Wertprodukts, die Hilfsbase mit einer starken Base freizusetzen und im Anschluß die freigesetzte Hilfsbase zu destillieren. Bei dem Reaktionsgemisch kann es sich dabei um das Produkt einer chemischen Umsetzung handeln oder um einen Strom aus einer Destillation oder Rektifikation, beispielsweise eines azeotropen Gemsichs, das mit einer ionischen Flüssigkeit als Entrainer versetzt worden ist.

Wesentlich dabei ist, das Wertprodukt unter Bedingungen zu rektifizieren, unter denen die ionische Flüssigkeit in ihrer protonierten Form nicht wesentlich flüchtig ist, beispielsweise durch thermische Spaltung der protonierten Hilfsbase, und die ionische Flüssigkeit erst dann freigesetzt und destilliert wird, wenn das Wertprodukt bereits abgetrennt ist. Ein solches Vorgehen ist auch möglich, wenn das Wertprodukt gegenüber der Hilfsbase in ihrer freien Form nicht stabil ist und zersetzt wird.

Liegt der Siedepunkt des Wertprodukts relativ hoch, so daß keine Bedingungen auffindbar sind, unter denen das Wertprodukt in Anwesenheit der protonierten Hilfsbase destilliert werden kann, so kann die Trennung auch in umgekehrter Reihenfolge durchgeführt werden, indem zuerst die Hilfsbase mit einer starken Base freigesetzt wird und anschließend die Hilfsbase in Gegenwart des Wertprodukts destilliert wird, und erst danach das Wertprodukt destilliert wird. Dies ist dann besonders von Vorteil, wenn das Wertprodukt nicht durch die verwendete starke Base zersetzt wird.

Das gleiche Prinzip ist auch anwendbar, wenn die protonierte Form der Hilfsbase als saurer Katalysator eingesetzt wird, d.h. statt einer Säure, wie z.B. Salzsäure, Schwefelsäure, Phosphorsäure, Methansulfonsäure, Trifluormethansulfonsäure, Toluolsulfonsäure, Essigsäure oder Ameisensäure, wird deren Salz mit einer Hilfsbase als ionische Flüssigkeit in einer Reaktion eingesetzt. Vorteil dabei ist, daß die protonierte Hilfsbase während der Reaktionsführung eine flüssige Phase ausbildet. Die katalytische Wirkung der protonierten Hilfsbase kann dann jederzeit durch Zugabe einer starken Base gestoppt werden.

In einer weiteren bevorzugten Ausführung wird ein saurer Katalysator in einer chemischen Umsetzung mit einer Hilfsbase neutralisiert, die mit dem verwendeten sauren Katalysator ein flüssiges Salz bildet, so daß der auf diesem Wege desaktivierte Katalysator in einer leichten Flüssig-flüssig-Trennung abgetrennt werden kann.

Selbstverständlich kann die Destillation einer ionischen Flüssigkeit auch in Abwesenheit des Wertprodukts erfolgen, beispielsweise indem man die ionische Flüssigkeit aus einer Phasentrennung oder einer flüssig-flüssig-Extraktion destilliert. Dabei kann die ionische Flüssigkeit, also die Hilfsbase in protonierter Form, auch noch einen Anteil an Wertprodukt oder gegebenenfalls Lösungsmittel enthalten, in der Regel weniger als jeweils 10 Gew%, bevorzugt weniger als je 5 Gew%, besonders bevorzugt weniger als je 3 Gew%. In diesem Fall können aus der ionischen Flüssigkeit zunächst Wertprodukt- und Lösungsmittelreste entfernt werden, beispielsweise durch Vakuumdestillation oder Strippen mit einem inerten Gas wie z.B. Stickstoff, und anschließend, nach Freisetzen der Hilfsbase mit einer starken Base, kann die Hilfsbase destillativ oder rektifikativ gereinigt werden.

Eine aufgereinigte Base kann dann jederzeit wieder in den Prozeß zurückgeführt werden.

Es kann auch vorteilhaft sein, die Hilfsbase in protonierter Form als Lösungsmittel für organische Reaktionen einzusetzen. Nach Abtrennung der Reaktionsprodukte kann die Hilfsbase wie oben beschrieben durch Freisetzung mit einer starken Base und Destillation wiedergewonnen und rückgeführt werden.

Bevorzugte mit dem erfindungsgemäßen Verfahren durchführbare Phosphorylierungen sind solche Reaktionen, bei denen Phosphorverbindungen, beispielsweise Phosphine, Phosphinsäureester, Phosphinigsäureester (Phosphinite), Phosphonsäureester, Phosphonsäurehalogenide, Phosphonsäureamide, Phosphonigsäureester (Phosphonite), Phosphonigsäureamide, Phosphonigsäurehalogenide, Phosphorsäureester, Phosphorsäurediesterhalogenide, Phosphorsäurediesteramide, Phosphorsäureesterdihalogenide, Phosphorsäureesterdiamide, Phosphorigsäureester (Phosphite), Phosphorigsäurediestefialogenide, Phosphorigsäurediesteramide, Phosphorigsäureesterdihalogenide oder Phosphorigsäureesterdiamide gebildet werden und im Reaktionsverlauf eine Säure abgespalten wird, die mit der Hilfsbase ein Salz bildet wie oben beschrieben.

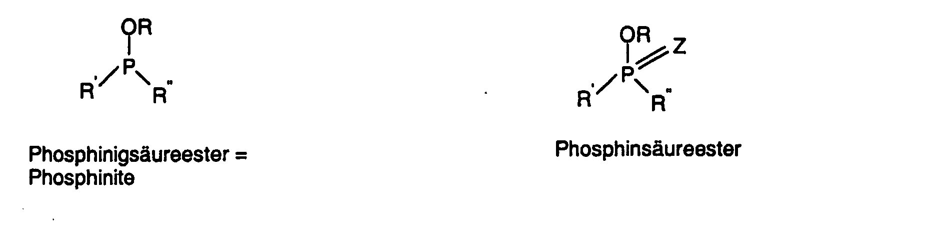

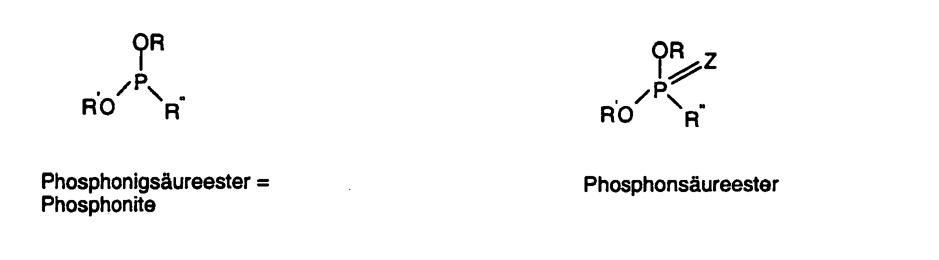

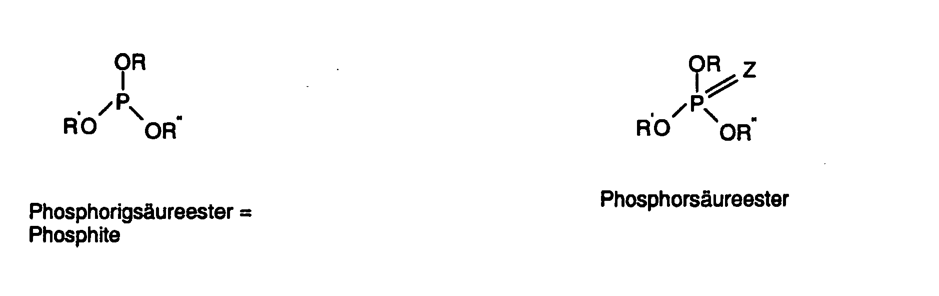

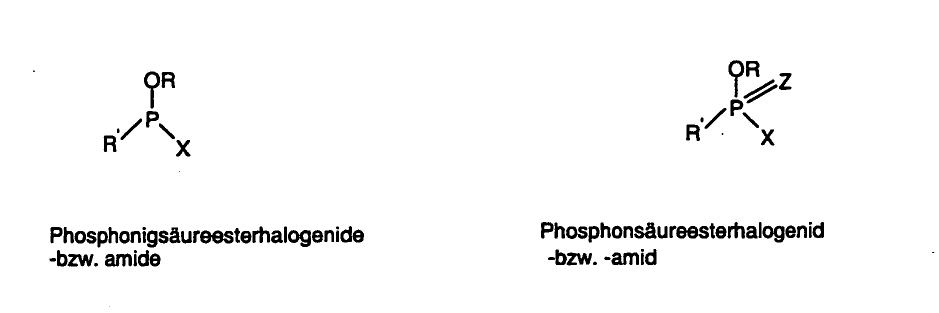

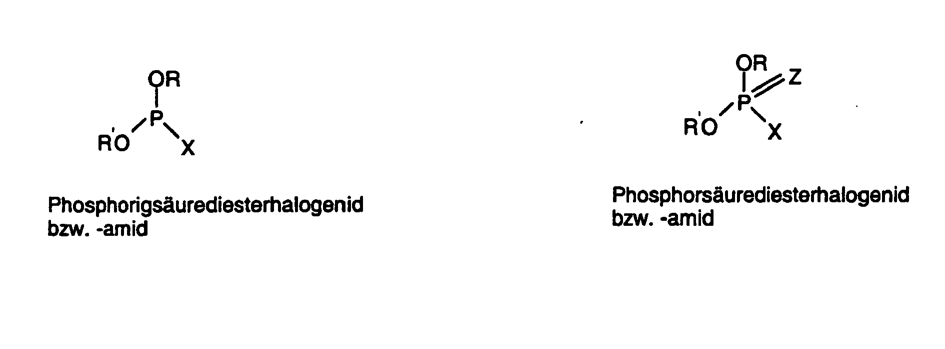

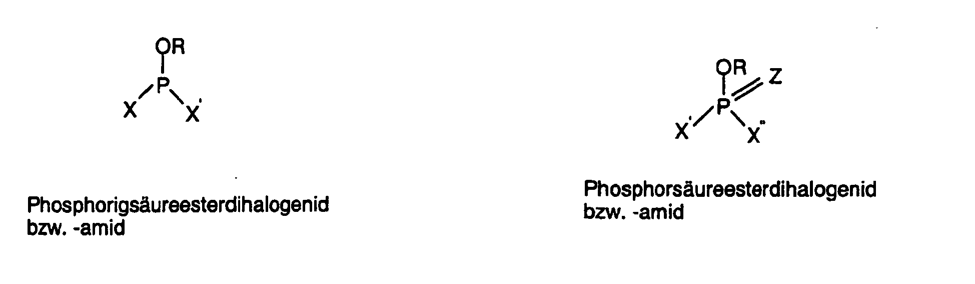

Darin stehen R, R' und R" für beliebige Reste, X und X' für Halogen oder Pseudohalogen, wie beispielsweise F, CI, Br, I, CN, OCN oder SCN oder un-, mono- oder disubstituierte Aminogruppen und Z für Sauerstoff, Schwefel oder ein un- oder monosubstituiertes Stickstoffatom.

Dabei kann es sich um Phosphorverbindungen handeln, die ein oder mehrere, beispielsweise zwei, drei oder vier, bevorzugt zwei oder drei, besonders bevorzugt zwei Phosphoratome aufweisen. In solchen Verbindungen sind die Phosphoratome typischerweise durch ein Brücke verbunden.

Beispielsweise können solche verbrückten Verbindungen mit zwei Phosphoratomen sein:

### Diphosphite

(RO)(R'O)P-O-Z-O-P(OR")(OR"') (Formel II),

### Diphosphonite

(RO)R'P-O-Z-O-PR"(OR"') , (Formel III)

### Diphosphinite

(R)(R')P-O-Z-O-P(R")(R"') (Formel IV),

### Phosphit-Phosphonite

(RO)(R'O)P-O-Z-O-P(OR")(R"') (Formel V),

### Phosphit-Phosphinite

(RO)(R'O)P-O-Z-O-P(R")(R"') (Formel VI),

### Phosphonit-Phosphinite

(R)(R'O)P-O-Z-O-P(R")(R"') (Formel VII),

Darin können R, R', R" und R'" beliebige organische Reste und Z eine beliebige bivalente Brücke sein.

Dabei kann es sich jeweils und unabhängig voneinander beispielsweise um ein bis 20 Kohlenstoffatome aufweisende, lineare oder verzweigte, substituierte oder unsubstituierte, aromatische oder aliphatische Reste, wie C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, handeln.

Die genannten Verbindungen können jeweils symmetrisch oder unsymmetrisch substituiert sein.

Phosphorverbindungen mit einem Phosphoratom sind beispielsweise solche der Formel (VIII)

P (X¹R⁷) (X²R⁸) (X³R⁹) (VIII)

mit
x¹, X², X³ unabhängig voneinander Sauerstoff, Schwefel, NR¹⁰ oder Einzelbindung
R⁷, R⁸, R⁹, R¹⁰ unabhängig voneinander gleiche oder unterschiedliche organische Reste.

Phosphorverbindungen mit zwei Phosphoratomen sind beispielsweise solche der Formel (IX) 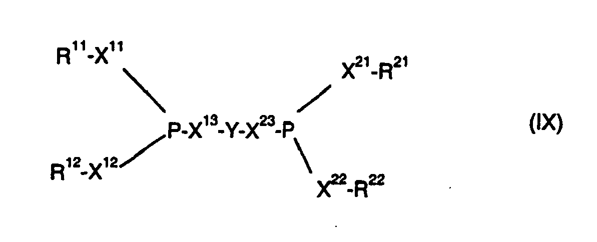 mit
X¹¹, X¹², X¹³, X²¹, X²², X²³ unabhängig voneinander Sauerstoff, Schwefel, NR¹⁰ oder Einzelbindung
R¹¹, R¹² unabhängig voneinander gleiche oder unterschiedliche, einzelne oder verbrückte organische Reste
R²¹, R²² unabhängig voneinander gleiche oder unterschiedliche, einzelne oder verbrückte organische Reste,
Y Brückengruppe.

Die beschriebenen Phosphorverbindungen sind beispielsweise als Liganden für Katalysatoren für die Hydrocyanierung von Butadien zu einem Gemisch isomerer Pentennitrile geeignet. Neben der Hydrocyanierung von 1,3-Butadien-haltigen Kohlenwasserstoffgemischen eignen sich die Katalysatoren im Allgemeinen für alle gängigen Hydrocyanierungsverfahren. Dabei sei insbesondere die Hydrocyanierung von nichtaktivierten Olefinen, z. B. von Styrol und 3-Pentennitril, genannt. Weiterhin ist der Einsatz zur Hydrierung, Hydroformylierung, Hydrocarboxylierung, Hydroamidierung, Hydroveresterung und Aldolkondensation denkbar.

Derartige Katalysatoren können einen oder mehrere der Phosphorverbindungen als Liganden aufweisen. Zusätzlich zu den Phosphorverbindungen als Liganden können sie noch wenigstens einen weiteren Liganden, der ausgewählt ist unter Cyanid, Halogeniden, Aminen, Carboxylaten, Acetylaceton, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃ sowie ein, zwei- und mehrzähnigen Phosphin-, Phosphinit-, Phosphonit- und Phosphitliganden aufweisen. Diese weiteren Liganden können ebenfalls ein, zwei- oder mehrzähnig sein und an das Metall koordinieren. Geeignete weitere phosphorhaltige Liganden sind z. B. die zuvor als Stand der Technik beschriebenen Phosphin-, Phosphinit- und Phosphitliganden.

Bevorzugt handelt es sich bei dem Metall um eines der VIII. Nebengruppe, besonders bevorzugt um Cobalt-, Rhodium-, Ruthenium-, Palladium- oder Nickelatome in beliebigen Oxidationsstufen. Werden die erfindungsgemäßen Katalysatoren zur Hydrocyanierung eingesetzt, so handelt es sich bei dem Metall der VIII. Nebengruppe insbesondere um Nickel.

Setzt man Nickel ein, so kann dieses in verschiedenen Wertigkeiten, wie 0, +1, +2, +3, vorliegen. Bevorzugt ist hierbei Nickel(0) und Nickel(+2), insbesondere Nickel(0).

Bei Katalysatoren für Hydroformylierungen werden im Allgemeinen unter Hydroformylierungsbedingungen aus den jeweils eingesetzten Katalysatoren oder Katalysatorvorstufen katalytisch aktive Spezies gebildet.

Hierfür wird als Metall vorzugsweise Cobalt, Ruthenium, Rhodium, Palladium, Platin, Osmium oder Iridium und insbesondere Cobalt, Rhodium und Ruthenium in beliebigen Oxidationsstufen verwendet.

Die Herstellung dieser Katalysatorsysteme ist technisch aufwendig und teuer. Dies gilt insbesondere, als daß die Katalysatorsysteme im Laufe ihrer Verwendung allmählich zersetzt werden und somit ausgeschleust und durch neuen Katalysator ersetzt werden müssen.

Verfahren zur Herstellung der Phosphorverbindungen und den entsprechenden Katalysatoren sind an sich bekannt, beispielsweise aus US 3,903,120, US 5,523,453, US 5,981,772, US 6,127,567, US 5,693,843, US 5,847,191, WO 01/14392,

WO 99/13983 und WO 99/64155.

Zur Herstellung der in den Katalysatoren eingesetzten Phosphorverbindungen als Liganden kann man beispielsweise zunächst eine Dihalogenphosphor(III)verbindung mit einem Monoalkohol zu einem Diester umsetzen. Gewünschtenfalls kann diese Verbindung vor der weiteren Umsetzung nach bekannten Verfahren isoliert und/oder gereinigt werden, z. B. durch Destillation. Dieser Diester wird beispielsweise dann mit einem Diol zu den zweizähnigen Phosphonitliganden umgesetzt. Für den Fall, dass symmetrische Liganden erhalten werden sollen, können zwei Äquivalente des Diesters in einer einstufigen Reaktion mit einem Äquivalent des Diols umgesetzt werden. Ansonsten wird zunächst ein Äquivalent des Diesters mit einem Äquivalent des Diols umgesetzt und nach Bildung des Monokondensationsproduktes wird ein zweites Diol zugegeben und weiter zu der Phosphorverbindung umgesetzt.

Die in der Reaktion freigesetzte Säure kann erfindungsgemäß mit einer der genannten Hilfsbasen unter Ausbildung eines flüssigen Salzes abgefangen werden, so daß diese Synthese erheblich vereinfacht werden kann.

Organodiphosphonite der Formel III, sowie Katalysatorsysteme, die solche Organodiphosphonite enthalten, sind bekannt, beispielsweise aus WO 99/64155. Zur Herstellung solcher Organodiphosphonite der Formel III beschreibt WO 99/64155 die Umsetzung von R'PCl₂ mit einem Mol ROH und die nachfolgende Umsetzung des erhaltenen (RO)R'PCI mit einem halben mol, bezogen auf ein mol (RO)R'PCI, einer Verbindung HO-Z-OH bei einer Temperatur im Bereich von 40 bis etwa 200°C. Dabei sollte die Abspaltung des Halogenwasserstoffs in ersten Schritt bevorzugt rein thermisch erfolgen. Zudem sollen beide Schritte in Gegenwart einer Base durchgeführt werden können.

Erfindungsgemäß werden die im Stand der Technik bekannten Verfahren, wie z.B. das aus der WO 99/64155 bekannte, zur Herstellung der genannten Phosphorverbindungen analog durchgeführt, mit dem Unterschied, daß erfindungsgemäß eine Hilfsbase wie oben beschrieben eingesetzt wird und die freigesetzte Säure aus dem Reaktionsgemisch mittels der Hilfsbase abgetrennt wird, wobei wie oben die Hilfsbase ein Salz mit der Säure bildet, das bei Temperaturen flüssig ist, bei denen die Phosphorverbindung während der Abtrennung des Flüssigsalzes nicht signifikant zersetzt wird und das Salz der Hilfsbase mit der Phosphorverbindung oder der Lösung der Phosphorverbindung in einem geeigneten Lösungsmittel zwei nicht mischbare flüssige Phasen ausbildet.

Allgemein können die genannten Phosphorverbindungen beispielsweise wie folgt hergestellt werden:

Die Edukte werden in der gewünschten Stöchiometrie, gegebenenfalls in einem Lösungsmittel gelöst oder dispergiert, d.h. suspendiert oder emulgiert, miteinander vermischt. Dabei kann es sinnvoll sein, die Edukte in eine oder mehrere Zusammensetzungen, d.h. voneinander getrennte Ströme, aufzuteilen, so daß die Reaktion nicht vor der Vermischung stattfindet. Die Hilfsbase, die mit der Säure erfindungsgemäß ein flüssiges Salz bildet, kann einem oder mehreren dieser Ströme beigemischt werden oder getrennt von den Strömen als gesonderter Strom der Reaktion zugeführt werden. Es ist auch möglich, wenn auch weniger bevorzugt, die Hilfsbase erst nach der Reaktion zur Abtrennung der Säure zuzugeben.

Die Edukte oder die genannten Zusammensetzungen werden einem Reaktor zugeführt und unter Reaktionsbedingungen miteinander umgesetzt, die zur Reaktion der Edukte zum Produkt führen. Solche Reaktionsbedingungen sind abhängig von den eingesetzten Edukten und den gewünschten Produkten und in dem in dieser Schrift genannten Stand der Technik angegeben.

Die Reaktion kann kontinuierlich, halbkontinuierlich oder diskontinuierlich erfolgen. Die Temperatur reicht in der Regel von 40°C bis 200°C, der Druck ist erfindungsgemäß nicht wesentlich und kann Unter-, Über oder Normaldruck, beispielsweise von 10 mbar bis 10 bar, bevorzugt 20 mbar bis 5 bar, besonders bevorzugt 50 mbar bis 2 bar und insbesondere 100 mbar bis 1,5 bar betragen. Die Verweilzeit des Reaktionsgemisches im Reaktor kann von wenigen Sekunden bis mehreren Stunden betragen und ist von der Reaktionstemperatur und, in der Regel in geringerem Ausmaß, von dem angelegten Druck abhängig.

Bevorzugt wird die Verweilzeit bei einer kontinuierlichen Reaktionsführung bei einer für die Reaktion ausreichend hohen Temperatur kurz gewählt, d.h. von wenigen Sekunden bis ca. 2 Stunden, bevorzugt von 1 Sekunde bis 2 Stunden, besonders bevorzugt von 1 Sekunde bis 1 Stunde, ganz besonders bevorzugt von 1 Sekunde bis 30 Minuten, insbesondere von 1 Sekunde bis 15 Minuten und außergewöhnlich bevorzugt von 1 Sekunde bis 5 Minuten.

In einer besonders bevorzugten Ausführungsform wird die Herstellung der Phosphorverbindungen, bevorzugt solcher mit mehreren Phosphoratomen, besonders bevorzugt solcher mit 2 oder 3 und ganz besonders bevorzugt solcher mit 2 Phosphoratomen, aus den jeweiligen Edukten kontinuierlich bei einer Temperatur von 60°C bis 150°C, bevorzugt bei einer Temperatur oberhalb des Schmelzpunktes des Salzes der verwendeten Hilfsbase mit der freigesetzten Säure bis 130°C, bei einer Verweilzeit unter 1 Stunde, bevorzugt unter 30 Minuten, besonders bevorzugt unter 15 Minuten, ganz besonders bevorzugt von 1 Sekunde bis 5 Minuten, insbesondere von 1 Sekunde bis 1 Minute und außergewöhnlich bevorzugt von 1 bis 30 Sekunden durchgeführt.

Durch eine derartige Ausführungsform wird der Austausch von Substituenten an den Phosphoratomen zurückgedrängt und es ist so möglich, unter überwiegend kinetischer Kontrolle Verbindungen mit mehreren Phosphoratomen, wie beispielsweise Verbindungen der Formel (IX), und Phosphorverbindungen mit gemischten Substituenten, beispielsweise Verbindungen der Formel (VIII) mit unterschiedlichen Resten R⁷, R⁸ und/oder R⁹, herzustellen, ohne daß die Substituenten infolge Equilibrierung am Phosphoratom/an den Phosphoratomen ausgetauscht werden.

Während der Reaktion ist für eine gute Durchmischung zu sorgen, beispielsweise durch Rühren oder Umpumpen mit statischen Mischern oder Düsen.

Als Reaktoren können dem Fachmann an sich bekannte Apparate eingesetzt werden, beispielsweise ein oder mehrere kaskadierte Rühr- oder Rohrreaktoren mit innen- und/oder außenliegenden Heizungen und bevorzugt Strahldüsenreaktoren oder Reaktionsmischpumpen.

Der Reaktionsaustrag wird in einen Apparat geführt, in dem sich während der Reaktion entstandene Phasen voneinander trennen können, beispielsweise Phasenscheider oder Mixer-Settler-Apparaturen. In diesem Apparat wird bei einer Temperatur, bei der das Salz der Hilfsbase mit der Säure flüssig ist, eine Phasentrennung der Phase, die überwiegend ionische Flüssigkeit enthält, von der Phase, die überwiegend das gewünschte Reaktionsprodukt enthält, durchgeführt. Falls erforderlich kann Lösungsmittel hinzugegeben werden, um eine Phasentrennung zu beschleunigen.

Aus der Phase, die überwiegend ionische Flüssigkeit enthält, kann die Hilfsbase, wie oben beschrieben, wiedergewonnen werden.

Aus der Phase, die das gewünschte Reaktionsprodukt enthält, kann das Reaktionsprodukt mit an sich bekannten Methoden isoliert und/oder gereinigt werden, beispielsweise durch Destillation, Rektifikation, Extraktion, fraktionierter oder einfacher Kristallisation, Membrantrennverfahren, Chromatographie oder Kombinationen davon.

Bei dem in der Reaktion verwendeten Lösungsmittel kann es sich um die oben angeführten Lösungsmittel handeln.

Die in der Reaktion verwendete Hilfsbase wird in der Regel in, bezogen auf zu erwartende Menge Säure, stöchiometrischer Menge oder leichtem Überschuß eingesetzt, beispielsweise 100 bis 200 Mol% bezogen auf die zu erwartende Menge Säure, bevorzugt 100 bis 150 und besonders bevorzugt 105 bis 125 Mol%.

Die Edukte zur Herstellung der gewünschten Phosphorverbindungen sind dem Fachmann an sich bekannt oder leicht erschließbar und sind beispielsweise in dem in dieser Schrift genannten Stand der Technik angegeben, ebenso die stöchiometrischen Verhältnisse, um die Edukte miteinander zur Reaktion zu bringen.

Die Edukte werden möglichst als Flüssigkeiten oder Schmelzen eingesetzt, gegebenenfalls werden sie dazu in einem Lösungsmittel gelöst oder dispergiert. Selbstverständlich ist es aber auch möglich, die Edukte zumindest teilweise als Feststoffe einzusetzen.

Werden sie mit einem Lösungsmittel versetzt, so wird das Lösungsmittel in der Regel in einer derartigen Menge eingesetzt, daß das Gemisch flüssig ist, beispielsweise als Lösung oder Dispersion. Typische Konzentrationen der Edukte bezogen auf die Gesamtmenge der Lösung oder Dispersion sind 5 bis 95 Gew.%, bevorzugt 10 bis 90 Gew.%, besonders bevorzugt 25 bis 90 Gew.% und ganz besonders bevorzugt 50 bis 90 Gew.-%.

Verbindungen (VIII) weisen die Formel

P (X¹R⁷) (X²R⁸) (X³R⁹) (VIII)

auf.

Unter Verbindung (VIII) wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff, Schwefel, NR¹⁰ oder Einzelbindung.

R¹⁰ steht darin für Wasserstoff oder einen organischen Rest mit 1 -10 Kohlenstoffatomen, bevorzugt für Wasserstoff, Phenyl oder C₁- C₄-Alkyl, worunter in dieser Schrift Methyl, Ethyl, *iso*-Propyl, n-Propyl, n-Butyl, *iso*-Butyl, *sek*-Butyl und *tert*-Butyl verstanden wird.

Falls alle der Gruppen X¹, X² und X³ für Einzelbindungen stehen, so stellt Verbindung (VIII) ein Phosphin der Formel P(R⁷ R⁸ R⁹) mit den für R⁷, R³ und R⁹ in dieser Beschreibung genannten Bedeutungen dar.

Falls zwei der Gruppen X¹, X² und X³ für Einzelbindungen stehen und eine für Sauerstoff, so stellt Verbindung (VIII) ein Phosphinit der Formel P(OR⁷)(R⁸)(R⁹) oder P(R⁷)(OR⁸)(R⁹) oder P(R⁷)(R⁸)(OR⁹) mit den für R⁷, R⁸ und R⁹ in dieser Beschreibung genannten Bedeutungen dar.

Falls eine der Gruppen X¹, X² und X³ für eine Einzelbindung steht und zwei für Sauerstoff, so stellt Verbindung (VIII) ein Phosphonit der Formel P(OR⁷)(OR^{B})(R⁹) oder P(R⁷)(OR⁸)(OR⁹) oder P(OR⁷)(R⁸)(OR⁹) mit den für R⁷, R⁸ und R⁹ in dieser Beschreibung genannten Bedeutungen dar.

In einer bevorzugten Ausführungsform sollten alle der Gruppen X¹, X² und X³ für Sauerstoff stehen, so daß Verbindung (VIII) vorteilhaft ein Phosphit der Formel P(OR⁷)(OR⁸)(OR⁹) mit den für R⁷, R⁸ und R⁹ in dieser Beschreibung genannten Bedeutungen darstellt.

Erfindungsgemäß stehen R⁷, R⁸, R⁹ unabhängig voneinander für gleiche oder unterschiedliche organische Reste.

Als R⁷, R⁸ und R⁹ kommen unabhängig voneinander Alkylreste, vorteilhaft mit 1 bis 10 C-Atomen, wie Methyl, Ethyl, n-Propyl, i-Propyl, n-Butyl, i-Butyl, s-Butyl, t-Butyl, ArylGruppen, wie Phenyl, o-Tolyl, m-Tolyl, p-Tolyl, p-Fluor-Phenyl, 1-Naphthyl, 2-Naphthyl, oder Hydrocarbyl, vorteilhaft mit 1 bis 20 C-Atomen, wie 1,1'-Biphenol, 1,1'-Binaphthol in Betracht.

Die Gruppen R⁷, R⁸ und R⁹ können miteinander direkt, also nicht allein über das zentrale Phosphor-Atom, verbunden sein. Vorzugsweise sind die Gruppen R⁷, R⁸ und R⁹ nicht miteinander direkt verbunden.

In einer bevorzugten Ausführungsform kommen als Gruppen R⁷, R⁸ und R⁹ Reste ausgewählt aus der Gruppe bestehend aus Phenyl, o-Tolyl, m-Tolyl und p-Tolyl in Betracht.

In einer besonders bevorzugten Ausführungsform sollten dabei maximal zwei der Gruppen R⁷, R⁸ und R⁹ Phenyl-Gruppen sein.

In einer anderen bevorzugten Ausführungsform sollten dabei maximal zwei der Gruppen R⁷, R³ und R⁹ o-Tolyl-Gruppen sein.

Als besonders bevorzugte Verbindungen (VIII) können solche der Formel

(o-Tolyl-O-)_{w} (m-Tolyl-O-)ₓ (p-Tolyl-O-)_{y} (Phenyl-O-)_{z} P

mit w, x, y, z eine natürliche Zahl
mit w + x + y + z = 3 und
w, z kleiner gleich 2
eingesetzt werden, wie (p-Tolyl-O-)(Phenyl)₂P, (m-Tolyl-O-)(Phenyl)₂P,
(o-Tolyl-O-)(Phenyl)₂P, (p-Tolyl-O-)₂(Phenyl)P, (m-Tolyl-O-)₂(Phenyl)P,
(o-Tolyl-O-)₂(Phenyl)
P, (m-Tolyl-O-)(p-Tolyl-O-)(Phenyl)P, (o-Tolyl-O-)(p-Tolyl-O-)(Phenyl)P, (o-Tolyl-O-)
(m-Tolyl-O-)(Phenyl)P, (p-Tolyl-O-)₃P, (m-Tolyl-O-)(p-Tolyl-O-)₂P, (o-Tolyl-O-)
(p-Tolyl-O-)₂P, (m-Tolyl-O-)₂(p-Tolyl-O-)P, (o-Tolyl-O-)₂(p-Tolyl-O-)P, (o-Tolyl-O-)
(m-Tolyl-O-)(p-Tolyl-O-)P, (m-Tolyl-O-)₃P, (o-Tolyl-O-)(m-Tolyl-O-)₂P (o-Tolyl-O-)₂(m-Tolyl-O-)
P oder Gemische solcher Verbindungen eingesetzt werden.

So können beispielsweise Gemische enthaltend (m-Tolyl-O-)₃P, (m-Tolyl-O-)₂(p-Tolyl-O-)P, (m-Tolyl-O-)(p-Tolyl-O-)₂P und (p-Tolyl-O-)₃P durch Umsetzung eines Gemisches enthaltend m-Kresol und p-Kresol, insbesondere im Molverhältnis 2:1, wie es bei der destillativen Aufarbeitung von Erdöl anfällt, mit einem Phosphortrihalogenid, wie Phosphortrichlorid, erhalten werden.

Solche Verbindungen (VIII) und deren Herstellung sind an sich bekannt.

Verbindungen (IX) weisen die Formel 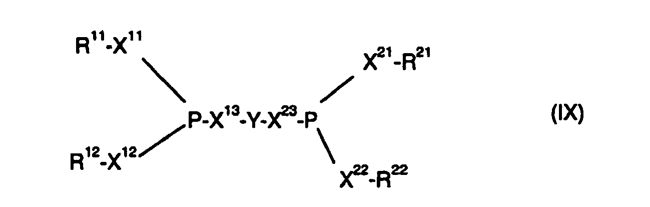 mit
X¹¹, X¹², X¹³ X²¹, X²², X²³ unabhängig voneinander Sauerstoff, Schwefel, NR¹⁰ oder Einzelbindung
R¹¹, R¹² unabhängig voneinander gleiche oder unterschiedliche, einzelne oder verbrückte organische Reste
R²¹, R²² unabhängig voneinander gleiche oder unterschiedliche, einzelne oder verbrückte organische Reste,
Y Brückengruppe
auf.

Unter Verbindung (IX) wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so daß das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so daß das mit X¹¹, X¹² und X¹³ umgebene Phosphoratom Zentralatom eines Phosphinits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so daß das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so daß das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so daß das mit X²¹, X²² und X²³ umgebene Phosphoratom Zentralatom eines Phosphits oder Phosphins, vorzugsweise eines Phosphins, sein kann.

Als Brückengruppe Y kommen vorteilhaft substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituierte, Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterschiedliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, inbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein.

Die Reste R²¹ und R²² können einzeln oder verbrückt sein.

Die Reste R¹¹, R¹², R²¹ und R²² können alle einzeln, zwei verbrückt und zwei einzeln oder alle vier verbrückt sein in der beschriebenen Art.

Auf die folgenden, besonders bevorzugten Ausführungsformen wird im Rahmen der vorliegenden Offenbarung im genannten Umfang ausdrücklich Bezug genommen:

In einer besonders bevorzugten Ausführungsform kommen die in US 3,773,809 genannten Verbindungen in Betracht, insbesondere die in Spalte 2, Zeile 23 bis Spalte 4, Zeile 14 und in den Beispielen beschriebenen.

In einer besonders bevorzugten Ausführungsform kommen die in US 6,127,567 genannten Verbindungen in Betracht, insbesondere die in Spalte 2, Zeile 23 bis Spalte 6, Zeile 35, in den Formeln I, II, III, IV, V, VI, VII, VIII und IX und in den Beispielen 1 bis 29 eingesetzten Verbindungen.

In einer besonders bevorzugten Ausführungsform kommen die in US 6,171,996 genannten Verbindungen in Betracht, insbesondere die in Spalte 2, Zeile 25 bis Spalte 6, Zeile 39, in den Formeln I, II, III, IV, V, VI, VII, VIII und IX und in den Beispielen 1 bis 29 eingesetzten Verbindungen.

In einer besonders bevorzugten Ausführungsform kommen die in US 6,380,421 genannten Verbindungen in Betracht, insbesondere die in Spalte 2, Zeile 58 bis Spalte 6, Zeile 63; in den Formeln I, II und III und in den Beispielen 1. bis 3 eingesetzten Verbindungen.

In einer besonders bevorzugten Ausführungsform kommen die in US 5,488,129 genannten Verbindungen in Betracht, insbesondere die in Spalte 3, Zeile 4 bis Spalte 4, Zeile 33, in der Formel I und in den Beispielen 1 bis 49 eingesetzten Verbindungen.

In einer besonders bevorzugten Ausführungsform kommen die in US 5,856,555 genannten Verbindungen in Betracht, insbesondere die in Spalte 2, Zeile 13 bis Spalte 5, Zeile 30, in den Formeln I und II und in den Beispielen 1 bis 4 eingesetzten Verbindungen.

In einer besonders bevorzugten Ausführungsform kommen die in WO 99/46044 genannten Verbindungen in Betracht, besonders die in Seite 3, Zeile 7 bis Seite 8, Zeile 27, und insbesondere die in den Formeln la bis Ig und in den Beispielen 1 bis 6 eingesetzten Verbindungen.

In einer besonders bevorzugten Ausführungsform kommen die in US 5,723,641 genannten Verbindungen der Formel I**,** II, III, IV und V in Betracht.

In einer besonders bevorzugten Ausführungsform kommen die in US 5,512,696 genannten Verbindungen der Formel I, II, III, IV, V, VI und VII, insbesondere die dort in den Beispielen 1 bis 31 eingesetzte Verbindungen, in Betracht.

In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzte Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzte Verbindungen, in Betracht.

In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzte Verbindungen, in Betracht.

In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzte Verbindungen, in Betracht.

In einer besonders bevorzugten Ausführungsform kommen die in US 5,959,135 genannten Verbindungen und dort in den Beispielen 1 bis 13 eingesetzte Verbindungen in Betracht.

In einer besonders bevorzugten Ausführungsform kommen die in US 5,847,191 genannten Verbindungen der Formel I, II und III in Betracht.

In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 12, 13, 14, 15, 16, 17, 18, 19, 20 und 21 dargestellten Verbindungen, in Betracht.

In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht.

In einer besonders bevorzugten Ausführungsform kommen die in WO 99/13983 genannten Verbindungen in Betracht, besonders die auf Seite 5, Zeile 1 bis Seite 11, Zeile 45 und insbesondere die in den Formeln la bis lh und den Beispielen 1 bis 24 genannten Verbindungen.

In einer besonders bevorzugten Ausführungsform kommen die in WO 99/64155 genannten Verbindungen in Betracht, besonders die auf Seite 4, Zeile 1 bis Seite 12, Zeile 7 und insbesondere die in den Formeln Ia bis Ic und den Beispielen 1 bis 4 genannten Verbindungen.

In einer besonders bevorzugten Ausführungsform kommen die in der deutschen Patentanmeldung DE 10038037 genannten Verbindungen in Betracht.

In einer besonders bevorzugten Ausführungsform kommen die in der deutschen Patentanmeldung DE 10046025 genannten Verbindungen in Betracht.

In einer besonders bevorzugten Ausführungsform kommen die in der deutschen Patentanmeldung DE 10156292 genannten Verbindungen in Betracht, insbesondere die auf Seite 1, Zeilen 6 bis 19 und von Seite 2, Zeile 21 bis Seite 2, Zeile 30 genannten Verbindungen.

In einer besonders bevorzugten Ausführungsform kommen die in der deutschen Patentanmeldung DE 10150281 genannten Verbindungen in Betracht, insbesondere die auf Seite 1, Zeile 36 bis Seite 5, Zeile 45 genannten Verbindungen.

In einer besonders bevorzugten Ausführungsform kommen die in der deutschen Patentanmeldung DE 10150285 genannten Verbindungen in Betracht, insbesondere die auf Seite 1, Zeile 35 bis Seite 5, Zeile 37 genannten Verbindungen.

In einer besonders bevorzugten Ausführungsform kommen die in der deutschen Patentanmeldung DE 10150286 genannten Verbindungen in Betracht, insbesondere die auf Seite 1, Zeile 37 bis Seite 6, Zeile 15 genannten Verbindungen.

In einer besonders bevorzugten Ausführungsform kommen die in der deutschen Patentanmeldung DE 10148712 genannten Verbindungen in Betracht, insbesondere die auf Seite 1, Zeilen 6 bis 29 und Seite 2, Zeile 15 bis Seite 4, Zeile 24 genannten Verbindungen.

Zur Abtrennung von Lewis-Säuren wird erfindungsgemäß ein Komplex aus Hilfsbase und Lewis-Säure gebildet, der wie oben beschrieben bei den betreffenden Temperaturen flüssig ist und eine mit dem Wertprodukt nicht mischbare Phase ausbildet.

Zur Abtrennung von z.B. Aluminiumtrichlorid ist es bekannt, äquimolare Mengen Phosphorylchlorid (POCI3) zum Produkt zu geben, wobei der resultierende Cl₃PO•AlCl₃-Komplex ausfällt und beispielsweise durch Filtration abgetrennt werden kann (W. T. Dye, J. Am. Chem. Soc., 1948, 70, 2595). Des weiteren ist es aus derselben Schrift bekannt, ein genau bestimmte Menge Wasser zum Produkt hinzuzugeben, um das Hydrat von Aluminiumtrichlorid zu bilden, das ebenfalls durch eine Filtration von Produkt abgetrennt werden kann.

Nach Gefter, Zh. Obshch. Khim., 1958, 28, 1338, kann AlCl3 auch durch Komplexbildung mit Pyridin ausgefällt und so abgetrennt werden.

Aus DE 32 48 483 ist ein Verfahren zur Abtrennung von AICI3 mit Hilfe von NaCI bekannt.

Nachteilig an diesen Verfahren ist, daß diese Komplexe hygroskopisch sind, als feste Komplexe eine fest-flüssig-Trennung erfordern und in dieser oftmals ungünstige Filtrationseigenschaften aufweisen, wie z.B. Klumpenbildung, was eine gegebenenfalls nachfolgende Wäsche erschwert.

EP 838 447 beschreibt die Bildung flüssiger Clathrate, die in dem jeweiligen Friedel-Crafts-Produkt unlöslich und z.B. über Phasentrennung abtrennbar sind.

K. R. Seddon, J. Chem. Tech. Biotechnol. 68 (1997) 351 beschreibt Prinzipien einer Abtrennung von Lewis-Säuren mit Hilfe von lonischen Flüssigkeiten wie 1-Butylpyridinium chlorid-Aluminium(III) chlorid, 1-Butyl-3-methylimidazolium chlorid-Aluminium(111) chlorid. Dabei handelt es sich jedoch um permanent kationische Systeme, die im Gegensatz zu beispielsweise den Hilfsbasen (la) bis (lr) nicht als freie, nichtionische Hilfsbasen eingesetzt werden können.

EP-A1 1 142 898 beschreibt Phosphorylierungen zur Herstellung von Biphenylphosphoniten, in denen Phasen von eutektischen Pyridin-Hydrochlorid-/Pyridin-Aluminiumchlorid-Gemischen von produkthaltigen Lösungsmittelphasen abgetrennt werden.

Nachteilig ist, daß die flüssige Abtrennung solcher Gemische vom Produkt ohne die Bildung eines Eutektikums nicht möglich ist.

Erfindungsgemäß wird das oben beschriebene Verfahren zur Abtrennung von Lewis-Säuren aus Reaktionsgemischen mittels einer Hilfsbase durchgeführt, in dem die Hilfsbase
b) ein Salz mit der Lewis-Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet.

Dazu wird in der Regel die Reaktion mit der Lewis-Säure zur Herstellung des Produkts wie üblich durchgeführt und nach Beendigung der Reaktion zur Abtrennung der Lewis-Säure die Hilfsbase zur Reaktionsmischung gegeben. Selbstverständlich kann auch das Reaktionsgemisch zur Hilfsbase gegeben werden. Wichtig ist eine Vermischung des Reaktionsgemisches mit der Hilfsbase, wobei Hilfsbase und Lewis-Säure in der Regel einen Komplex bilden. Pro mol abzutrennender Lewis-Säure im Reaktionsgemisch wird in der Regel mindestens ein mol Hilfsbase verwendete, bevorzugt 1,0 bis 1,5 mol/mol, besonders bevorzugt 1,0 bis 1,3 mol/mol, ganz besonders bevorzugt 1,0 bis 1,3 und insbesondere 1,0 bis 1,25 mol/mol.

Nach Vermischung von Lewis-Säure und Hilfsbase kann sofort weiter aufgearbeitet werden, es kann aber auch noch einige Minuten bis mehrere Stunden weitergerührt werden, bevorzugt 5 bis 120, besonders besonders 10 bis 60 und ganz besonders bevorzugt 15 bis 45 Minuten.

Dabei kann das Reaktionsgemisch vorteilhafterweise bei einer Temperatur gehalten werden, bei der der Komplex aus Hilfsbase und Lewis-Säure flüssig ist, jedoch noch keine wesentliche Zersetzung auftritt, es kann aber auch unterhalb der Schmelztemperatur des Komplexes gehalten werden.

Die Phasentrennung erfolgt unter Bedingungen, wie sie bereits oben beschrieben sind. Bei einem Komplex aus beispielsweise AICI₃ und N-Methylimidazol beträgt der Schmelzpunkt ca. 60 °C, so daß die Abtrennung, z.B. durch Phasentrennung, vom Wertprodukt bei relativ niedrigen Temperaturen verfolgen kann.

Die erfindungsgemäße Abtrennung kann überall dort eingesetzt werden, wo Lewis-Säuren von einem Wertprodukt abgetrennt werden müssen, bevorzugt bei Friedel-Crafts-Alkylierungen oder -Acylierungen, Phosphorylierungen oder Sulfurierungen von Aromaten und besonders bevorzugt bei Phosphorylierung von Aromaten.

Bevorzugte Beispiele von Phosphorylierungen von Aromaten sind die Lewis-Säurekatalysierte Umsetzung von Aromaten mit Phosphorylhalogeniden, beispielsweise PCl₃, PCl₅, POCl₃ oder PBr₃.

Als Aromaten können beispielsweise solche der Formel (X) eingesetzt werden, 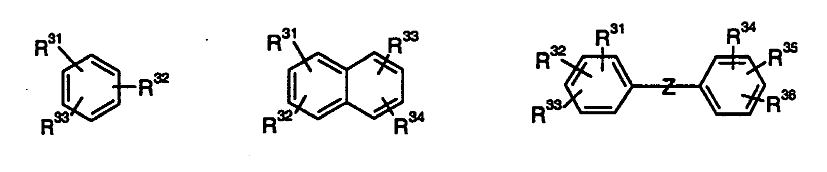 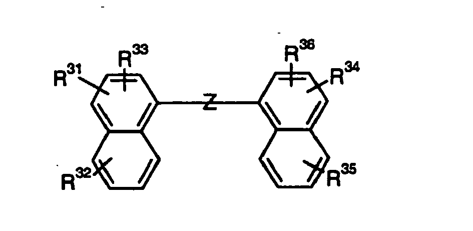 worin
Z eine Einfachbindung oder beliebige bivalente Brücke und
R³¹, R³², R³³, R³⁴, R³⁵ und R³⁶ unabhängig voneinander
Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₁-C₁₈-Alkyloxy, C₁-C₁₈-Alkyloxycarbonyl, C₆-C₁₂-Aryl, C₅-C₁₂-Cycloalkyl, einen fünf- bis sechsgliedrigen, Sauerstoff-, Stickstoff- und/oder Schwefelatome aufweisenden Heterocyclus oder funktionelle Gruppen bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte lminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein kann.

Funktionelle Gruppen bedeuten darin beispielsweise Nitro- (-NO₂), Nitroso- (-NO), Carboxyl- (-COOH), Halogen- (-F, -Cl, -Br, -l), Amino- (-NH₂, -NH(C₁-C₄-Alkyl), -N(C₁-C₄-Alkyl)₂), Carboxamid- (-CONH₂, -CONH(C₁-C₄-Alkyl), -CON(C₁-C₄-Alkyl)₂), Nitril- (-CN), Thiol- (-SH) oder Thioetherfunktionen (-S(C₁-C₄-Alkyl)).

Bevorzugt sind die Reste R³¹, R³², R³³, R³⁴, R³⁵ und R³⁶ unabhängig voneinander Wasserstoff, C₁-C₄-Alkyl, C₁-C₄-Alkyloxy, C₁-C₄-Alkyloxycarbonyl oder Halogen.

Besonders bevorzugt sind die Reste R³¹, R³², R³³, R³⁴, R³⁵ und R³⁶ unabhängig voneinander Wasserstoff, Methyl, *tert*-Butyl, Ethyl, Methoxy, Fluor oder Chlor.

Beispiele für Z sind eine Einfachbindung, Methylen, 1,2-Ethylen, 1,1-Ethylen, 1,1,-Propylen, 2,2-Propylen, 1,2-Phenylen, 1,4-Dimethyl-2,3-phenylen, Sauerstoff (-O-), unsubstituierter oder einfach substituierter Stickstoff (-NH- oder -N(C₁-C₄-Alkyl)-) oder Schwefel (-S-).

Bevorzugt ist Z eine Einfachbindung, Sauerstoff oder Methylen.

Besonders bevorzugte Aromaten sind Benzol, Toluol, o-, m- oder p-Xylol, 2,4,6-Trimethylbenzol, Ethylbenzol, 1-Ethyl-3-methylbenzol, 1-Ethyl-4-methylbenzol, iso-Propylbenzol, 1,3-Di-*iso*-propylbenzol, *tert*-Butylbenzol, 1,3-Di-*tert*-Butylbenzol, 1-*tert*-Butyl -3-methylbenzol, 1-*tert*-Butyl-3,5-dimethylbenzol, n-Propyibenzo), Styrol, lnden, Fluoren, Dimethylanilin, Fluorbenzol, Chlorbenzol, Brombenzol, 1,2-, 1,3- oder 1,4-Dichlorbenzol, 1,2-, 1,3- oder 1,4-Difluorbenzol, 1,1'-Binaphthyl, 2,2'-Di(C₁-C₄-alkyl)-1,1'-binaphthyl, besonders 2,2'-Dimethyl-1,1'-binaphthyl, 2,2'-Di(C₁-C₄-alkyloxy)-1,1'-binaphthyl, besonders 2,2'-Dimethoxy-1,1'-binaphthyl, 3,3'-Bis(C₁-C₄-Alkyloxycarbonyl)-1,1'-binaphthyl, Biphenyl, 3,3',5,5'-Tetra(C₁-C₄-alkyl)oxybiphenyl, besonders 3,3',5,5'-Tetramethoxybiphenyl, 3,3',5,5'-Tetra(C₁-C₄-alkyl)biphenyl, besonders 3,3',5,5'-Tetramethylbiphenyl, 3,3'-Dimethoxy-5,5'-dimethyl-biphenyl, Naphthalin, 2-(C₁-C₄-Alkyl)-naphthalin, besonders 2-Methyl-naphthalin, 2-(C₁-C₄-Alkyloxy)-naphthalin, besonders 2-Methoxy-naphthalin oder Diphenylmethan.

Ganz besonders bevorzugte Aromaten sind Benzol, Toluol, o-, m- oder p-Xylol, 2,4,6-Trimethylbenzol, *iso*-Propylbenzol, *tert*-Butylbenzol, Fluorbenzol, Chlorbenzol, Naphthalin und Binaphthyl.

Beispiele für Wertprodukte, die durch Phosphorylierungen oder Sulfurierungen von Aromaten, Friedel-Crafts-Alkylierungen oder -Acylierungen erhalten werden können sind Ethylbenzol, Acetophenon, 4-Methylacetophenon, 4-Methoxyacetophenon, Propiophenon, Benzophenon, Dichlorphenylphosphin, Diphenylchlorphosphin, Tosylchlorid, 1,2-, 1,3- und 1,4-Diethylbenzol, 1;2;3-, 1,2,4- und 1,3,5-Triethylbenzol, Cumol (*iso-*Propylbenzol), *tert*-Butylbenzol, 1,3- und 1,4-Methyl-*iso*-propylbenzol, 9,10-Dihydroanthracen, Indan, Kresol, 2,6-Xylenol, 2-*sec*-Butylphenol, 4-*tert*-Butylphenol, Octylphenol, Nonylphenol, Dodecylphenol, Thymol oder 2,6-di-*tert*-Butyl-4-methylphenol.

Erfindungsgemäß wird die Säure mit einer nichtionischen, d.h. ungeladenen Hilfsbase, abgetrennt. Dazu eignen sich besonders die oben angeführten Hilfsbasen der Formeln (Ia) bis (Ir).

In einer bevorzugten Ausführungsform zur Abtrennung von Lewis-Säuren werden diese ohne überwiegende Anteile von Brönsted-Säuren (Protonensäuren) abgetrennt, d.h. im abgetrennten Salz der Säure mit der Hilfsbase ist das molare Verhältnis von Brönsted-Säuren zu Lewis-Säuren nicht größer als 1:1, bevorzugt nicht größer als 0,75:1, besonders bevorzugt nicht größer als 0,5:1, ganz besonders bevorzugt nicht größer als 0,3:1 und insbesondere nicht größer als 0,2:1.

In einer weiteren bevorzugten Ausführungsform können als weitere Phosphorverbindungen mit dem erfindungsgemäßen Verfahren Aminodihalogenphosphine, Diaminohalogenphosphine, Triaminophosphine, Phosphorigsäuresterdiamide, Aminophosphine, Diaminophosphine, Phosphorigsäuresteramidhalogenide und Aminophosphinhalogenide hergestellt werden.

Aus WO 98/19985 ist bekannt, die Synthese von Aminochlorphosphinen durch Umsetzung einer N-H-aciden Verbindung mit Phosphortrichlorid in einem organischen Lösungsmittel in Gegenwart einer Hilfsbase unter Bildung eines unlöslichen Salzes durchzuführen. Nachteilig an dieser Methode ist, daß das Salz anschließend durch Filtration abgetrennt werden muß.

Van der Slot et al. beschreiben in Organometallics 2002, 21, 3873 die Synthese von Aminochlorphosphinen, Aminophosphinen und Phoshoramiditen unter Einsatz von Triethylamin als Hilfsbase.

Die bei der Umsetzung gebildeten, unlöslichen Salze müssen ebenfalls durch Filtration entfernt werden.

WO 02/83695 beschreibt die Synthese von Phosphoramiditen und deren Verwendung in der Hydroformylierung von terminalen und internen Olefinen.

Mit dem erfindungsgemäßen Verfahren können Phosphorhalogenide und chelatisierende Phosphoramidit-Liganden technisch einfacher gehandhabt werden (keine Feststoffabtrennung der Salze der Hilfsbase) und mit einer höheren Raum-Zeit-Ausbeute in der Umsetzung mit hoher Selektivität hergestellt werden.
Aminodihalogenphosphine
   [N]PXX'
Diaminohalogenphosphine
   [N][N']PX
Triaminophosphine
   [N][N'][N"]P
Phosphorigsäuresterdiamide
   (RO)P[N][N']
Aminophosphine
   R'R"P[N]
Diaminophosphine
   R'P[N][N']
Phosphorigsäuresteramidhalogenide
   (RO)[N]PX
Aminophosphinhalogenide
   [N]R'PX

Darin stehen R, R' und R" für beliebige organische Reste, die jeweils gleich oder verschieden sein können, X und X' für Halogen oder Pseudohalogen, wie beispielsweise F, Cl, Br, l, CN, OCN oder SCN, bevorzugt um CI, die jeweils gleich oder verschieden sein können, und [N], [N'] und [N"] für un-, mono- oder disubstituierte Aminogruppen, die jeweils gleich oder verschieden sein können.

Dabei kann es sich um Phosphorverbindungen handeln, die ein oder mehrere, beispielsweise zwei, drei oder vier, bevorzugt zwei oder drei, besonders bevorzugt zwei Phosphoratome aufweisen. In solchen Verbindungen sind die Phosphoratome typischerweise durch ein Brücke verbunden.

Beispielsweise können solche verbrückten Verbindungen mit zwei Phosphoratomen sein:

Am Phosphor jeweils Stickstoff- und Sauerstoff-substituierte Systeme:
Diphosphorigsäuredieesteramide
   [N](R'O)P-O-Z-O-P[N'](OR")

Am Phosphor jeweils Stickstoff-substituierte Systeme:
Diphosphorigsäureesterdiamide
   [N][N']P-O-Z-O-P[N"][N"']
Bistriaminophosphane
   [N][N']P-[N"]-Z-[N'"]-P[N""][N"'"]

Unsymmetrische substituierte Systeme:
[N](R'O)P-O-Z-O-P(OR")(OR"')
[N][N']P-O-Z-O-P(OR")(OR"')
[N][N']P-O-Z-O-P[N"](OR"')

Am Phosphor jeweils Stickstoff-und Kohlenstoff-substituierte Systeme:
[N](R')P-O-Z-O-P[N'](R"')
[N](R')P-[N"]-Z-[N'"]-P[N'](R'")

Unsymmetrische Systeme:
[N](R'O)P-O-Z-O-P[N'](R"')

Darin können R, R', R" und R'" beliebige organische Reste, die jeweils gleich oder verschieden sein können, [N], [N'], [N"], [N"'], [N""] und [N""'] für un-, mono- oder disubstituierte Aminogruppen, die jeweils gleich oder verschieden sein können und Z eine beliebige bivalente Brücke sein.

Selbstverständlich sind auch andere, hier nicht explizit angeführte Permutationen denkbar.

Bei R, R', R" und R'" kann es sich jeweils und unabhängig voneinander beispielsweise um ein bis 20 Kohlenstoffatome aufweisende, lineare oder verzweigte, substituierte oder unsubstituierte, aromatische oder aliphatische Reste, wie C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₂- C₁₈-Alkenyl, C₆- C₁₂-Aryl, C₅- C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, handeln.

Die bivalente Brücke Z kann beispielsweise unsubstituiertes oder mit Halogen, C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₈-C₁₂-Arylen, C₃-C₁₂-Cycloalkylen, C₁-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, - O(CO)(NH)-, -O(CO)- oder -(CO)O-Gruppen unterbrochenes C₂-C₂₀-Alkylen bedeuten.

Bevorzugt sind bivalente Brücken Z der Formel (XII), 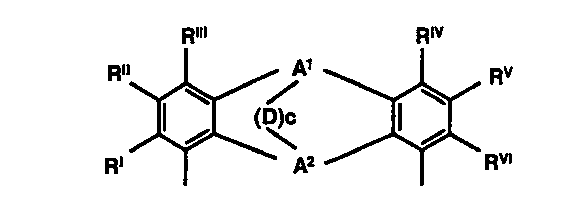 sowie solche der Formeln (Xlll.a) bis (Xlll.t) 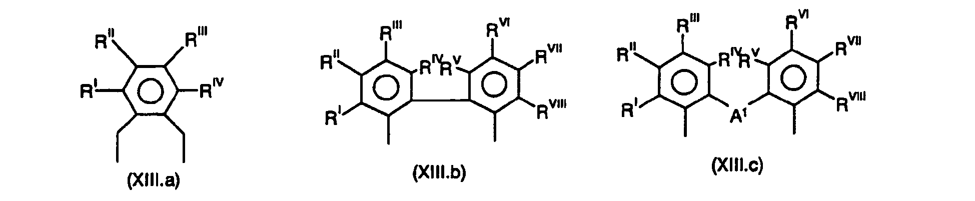 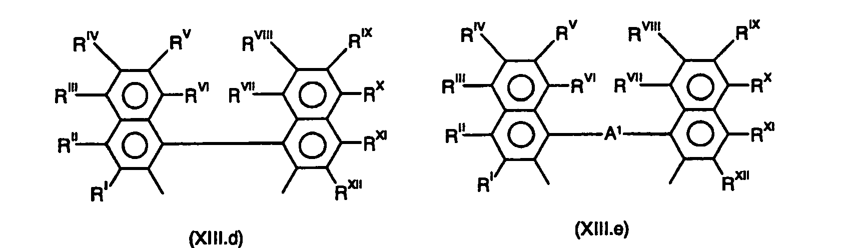 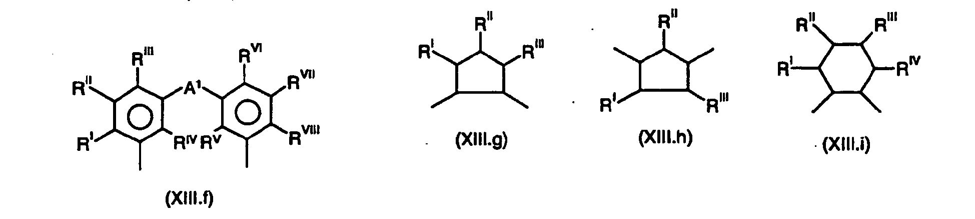 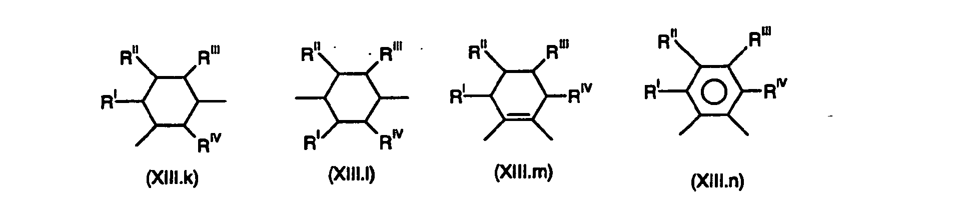 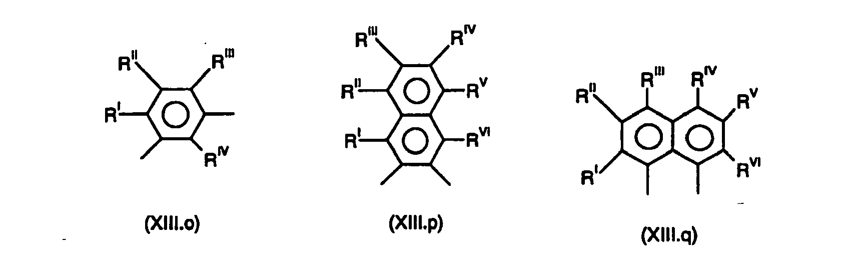 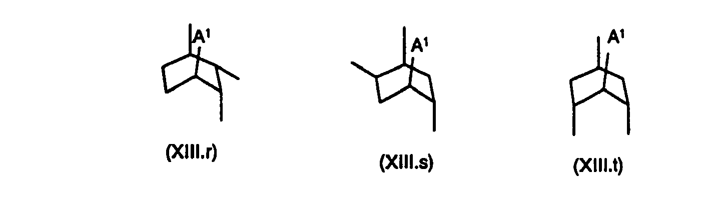 worin
A¹ und A² unabhängig voneinander für O, S, SiR⁵¹R⁵², NR⁵³ oder CR⁵⁴R⁵⁵ stehen, wobei
R⁵¹,R⁵² und R⁵³ unabhängig voneinander für Wasserstoff, Alkyl, Cycloalkyl, Heterocycloalkyl, Aryl oder Hetaryl stehen,
R⁵⁴ und R⁵⁵ unabhängig voneinander für Wasserstoff, Alkyl, Cycloalkyl, Heterocycloalkyl, Aryl oder Hetaryl stehen oder die Gruppe R⁵⁴ gemeinsam mit einer weiteren Gruppe R⁵⁴ oder die Gruppe R⁵⁵ gemeinsam mit einer weiteren Gruppe R⁵⁵ eine intramolekulare Brückengruppe D bilden,
wobei in den Formeln Xlll.a bis Xlll.t A¹ zusätzlich für eine C₂ oder C₃-Alkylenbrücke steht, die eine Doppelbindung und/oder einen Alkyl-, Cycloalkyl-, Hetero-cycloalkyl-, Aryl- oder Hetaryl-Substituenten aufweisen kann, oder die durch O, S, SiR⁵¹R⁵² oder NR⁵³ unterbrochen sein kann,
- D: eine zweibindige Brückengruppe, ausgewählt aus den Gruppen 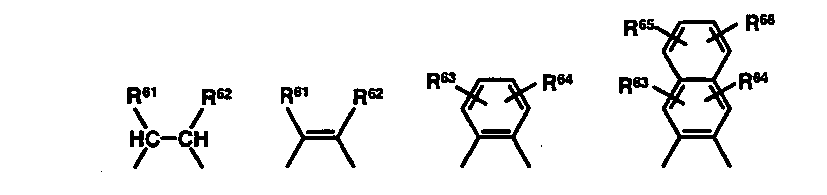 ist, in denen

R⁶¹ und R⁶² unabhängig voneinander für Wasserstoff, Alkyl, Cycloalkyl, Aryl, Halogen, Trifluormethyl, Carboxyl, Carboxylat oder Cyano stehen oder miteinander zu einer C₃-bis C₄-Alkylenbrücke verbunden sind,
R⁶³, R⁶⁴, R⁶⁵ und R⁶⁶ unabhängig voneinander für Wasserstoff, Alkyl, Cycloalkyl, Aryl, Halogen, Trifluormethyl, COOH, Carboxylat, Cyano, Alkoxy, SO₃H, Sulfonat, NE¹E², Alkylen-NE¹E²E³⁺X⁻, Acyl oder Nitro stehen,
   - c: 0 oder 1 ist,
wobei für den Fall, daß c=0 ist, die Gruppen A¹ und A² nicht durch eine Einfachbindung miteinander verbunden sind
R^{I}, R^{II}, R^{III}, R^{IV}, R^{V}, R^{VI}, R^{VII}, R^{VIII}, R^{IX}, R^{X}, R^{XI} und R^{XII} unabhängig voneinander für Wasserstoff, Alkyl, Alkoxy, Cycloalkyl, Heterocycloalkyl, Aryl, Hetaryl, Halogen, COOR⁵⁶, COO⁻M⁺, SO₃R⁵⁶, SO⁻₃M⁺, NE¹E², NE¹E²E³⁺X⁻, Alkylen-NE¹E², Alkylen-NE¹E²E³⁺X⁻, OR56, SR56, (CHR⁵⁷CH₂O)_{w}R⁵⁶, (CH₂N(E¹))_{w}R⁵⁶, (CH₂CH₂N(E¹))_{w}R⁵⁶, Halogen, Trifluormethyl, Nitro, Acyl oder Cyano stehen,
worin
R⁵⁶, E¹, E² und E³ jeweils gleiche oder verschiedene Reste, ausgewählt unter Wasserstoff, Alkyl, Cycloalkyl oder Aryl bedeuten,
   - R⁵⁷: für Wasserstoff, Methyl oder Ethyl steht,
   - M⁺: für ein Kation steht,
   - X⁻: für ein Anion steht, und
   - w: für eine ganze Zahl von 1 bis 120 steht,
oder
zwei benachbarte Reste, ausgewählt unter R^{I}, R^{II}, R^{III}, R^{IV}, R^{V}, R^{VI}, R^{VII} und R^{VIII} zusammen mit zwei benachbarten Kohlenstoffatomen des Benzolkerns, an den sie gebunden sind, für ein kondensiertes Ringsystem, mit 1, 2 oder 3 weiteren Ringen stehen.

Bevorzugte Brückengruppen Z der Formel (XII) sind solche, in denen der Index c für 0 steht und die Gruppen A¹ und A² ausgewählt sind aus den Gruppen 0, S und CR^{d}R^{e}, insbesondere unter 0, S, der Methylengruppe (R⁵⁴ = R⁵⁵ = H), der Dimethylmethylengruppe (R⁵⁴ = R⁵⁵ = CH₃), Diethylmethylengruppe (R⁵⁴ = R⁵⁵ = C₂H₅), der Di-n-propyl-methylengruppe (R⁵⁴ = R⁵⁵ = n-Propyl) oder der Di-n-butylmethylengruppe (R⁵⁴ = R⁵⁵ = n-Butyl). Insbesondere sind solche Brückengruppen Z bevorzugt, in denen A¹ von A² verschieden ist, wobei A¹ bevorzugt eine CR^{d}R^{e}-Gruppe und A² bevorzugt eine O- oder S-Gruppe, besonders bevorzugt eine Oxagruppe O ist.

Besonders bevorzugte Brückengruppen Z sind somit solche, die aus einem Triptycenartigen oder Xanthen-artigen (A¹: CR^{d}R^{e}, A²: O) Gerüst aufgebaut sind.

Die Substituenten R^{I}, R^{II}, R^{III}, R^{IV}, R^{V}, R^{VI}, R^{VII}, R^{VIII}, R^{IX}, R^{X}, R^{XI} and R^{XII} sind vorzugsweise ausgewählt unter Wasserstoff, Alkyl, Alkoxy, Cycloalkyl, Heterocycloalkyl, Aryl und Hetaryl. Nach einer ersten bevorzugten Ausführungsform stehen R^{I}, R^{II}, R^{III}, R^{IV}, R^{V}, R^{VI}, R^{VII}, R^{VIII}, R^{IX}, R^{X}, R^{XI} and R^{XII} für Wasserstoff. Nach einer weiteren bevorzugten Ausführungsform stehen R^{I} und R^{VI} unabhängig voneinander in XIII.p und XIII.q. für C₁-C₄-Alkyl oder C₁-C₄-Alkoxy. Vorzugsweise sind R¹ und R^{VI} ausgewählt unter Methyl, Ethyl, Isopropyl, tert-Butyl und Methoxy. Bevorzugt stehen in diesen Verbindungen R^{II}, R^{III} R^{IV} und R^{V} für Wasserstoff.

Nach einer weiteren bevorzugten Ausführungsform stehen in Xlll.b, Xlll.c und XIII.f R^{I}, R^{III}, R^{VI} und R^{VIII} unabhängig voneinander für C₁-C₄-Alkyl oder C₁-C₄-Alkoxy. Vorzugsweise sind R^{I}, R^{III}, R^{VI} und R^{VIII} ausgewählt unter Methyl, Ethyl, Isopropyl, tert-Butyl und Methoxy. Bevorzugt stehen in diesen Verbindungen R^{II}, R^{IV}, R^{V} und R^{VII} für Wasserstoff.

Nach einer weiteren bevorzugten Ausführungsform stehen in XIII.b, Xlll.c und Xlll.f R^{I}, R^{III}, R^{IV}, R^{V}, R^{VI} und R^{VIII} unabhängig voneinander für C₁-C₄-Alkyl oder C₁-C₄-Alkoxy. Vorzugsweise sind R¹, R^{III}, R^{IV}, R^{V}, R^{VI} und R^{VIII} ausgewählt unter Methyl, Ethyl, lsopropyl, tert-Butyl und Methoxy. Bevorzugt stehen in diesen Verbindungen R^{II} und R^{VII} für Wasserstoff.

Nach einer weiteren bevorzugten Ausführungsform stehen in XIII.d und XIII.e R^{I} und R^{XII} unabhängig voneinander für C₁-C₄-Alkyl, C₁-C₄-Alkoxy, C₁-C₄-Carboalkoxy oder C₁-C₄-Trialkylsilyl. Vorzugsweise sind R^{I} und R^{XII} ausgewählt unter Methyl, Ethyl, I-sopropyl, tert-Butyl, Methoxy, Carbomethoxy und Trimethylsilyl. Bevorzugt stehen in diesen Verbindungen R^{II}, R^{III}, R^{IV}, R^{V}, R^{VI}, R^{VII}, R^{VIII}, R^{IX}, R^{X} und R^{XI} für Wasserstoff.

Wenn zwei benachbarte Reste, ausgewählt unter R^{I}, R^{II}, R^{III}, R^{IV}, R^{V}, R^{VI}, R^{VII}, R^{VIII}, R^{IX}, R^{X}, R^{XI} and R^{XII} für ein ankondensiertes, also anelliertes, Ringsystem stehen, so handelt es sich bevorzugt um Benzol- oder Naphthalinringe. Anellierte Benzolringe sind vorzugsweise unsubstituiert oder weisen 1, 2 oder 3, insbesondere 1 oder 2 Substituenten auf, die ausgewählt sind unter Alkyl, Alkoxy, Halogen, SO₃H, Sulfonat, NE¹E², Alkylen-NE¹E², Trifluormethyl, Nitro, COOR', Alkoxycarbonyl, Acyl und Cyano. Anellierte Naphthalinringe sind vorzugsweise unsubstituiert oder weisen im nicht anellierten Ring und/oder im anellierten Ring insgesamt 1, 2 oder 3, insbesondere 1 oder 2 der zuvor bei den anellierten Benzolringen genannten Substituenten auf.

Unter den Gruppen Xlll.a bis Xlll.t sind die Gruppen Xlll.a bis Xlll.e bevorzugt, besonders bevorzugt sind die Gruppen XIII.b und Xlll.d.

Bei den un-, mono- oder disubstituierte Aminogruppen [N], [N'], [N"], [N"'], [N""] und [N""'] kann es sich unabhängig voneinander jeweils um Gruppen -NR⁴¹R⁴² handeln, worin R⁴¹ und R⁴² unabhängig voneinander C₁- C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₂ - C₁₈-Alkenyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, wobei R⁴¹ und R⁴² auch gemeinsam einen Ring bilden können.

Bevorzugte Gruppen -NR⁴¹R⁴², in denen R⁴¹ und R⁴² einen Ring bilden, sind solche Gruppen der Formeln XIV.a bis XIV.k 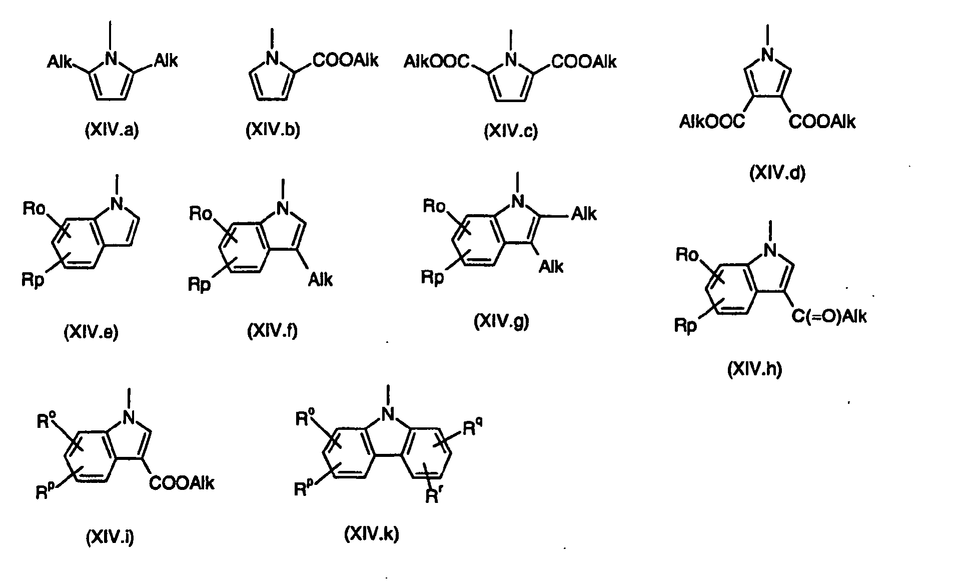 worin
Alk eine C₁-C₄-Alkylgruppe ist und
R^{o}, R^{p}, R^{q} und R^{r} unabhängig voneinander für Wasserstoff, C₁-C₄-Alkyl, C₁-C₄-Alkoxy, Acyl, Halogen, Trifluormethyl, C₁-C₄-Alkoxycarbonyl oder Carboxyl stehen.

Zur Veranschaulichung werden im Folgenden einige vorteilhafte Pyrrolgruppen aufgelistet:

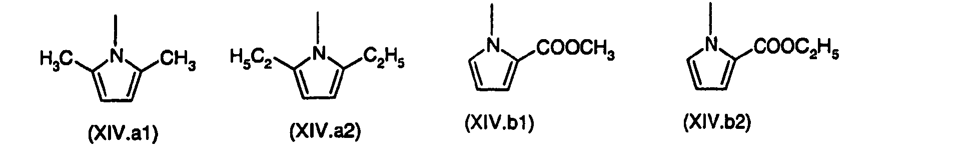

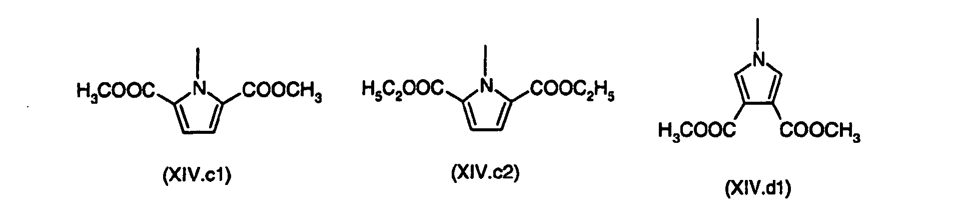

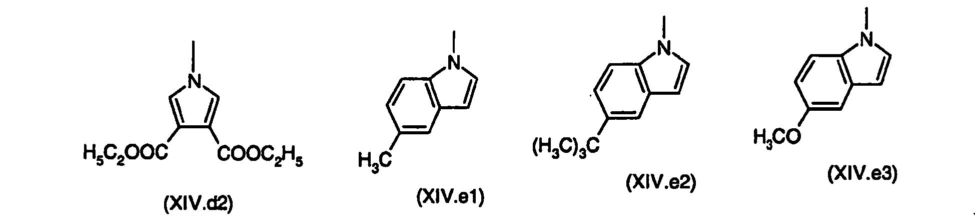

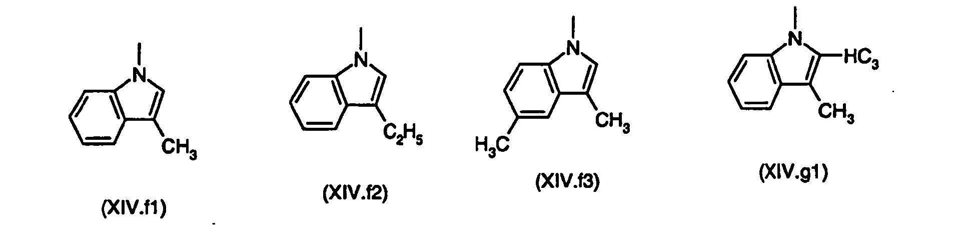

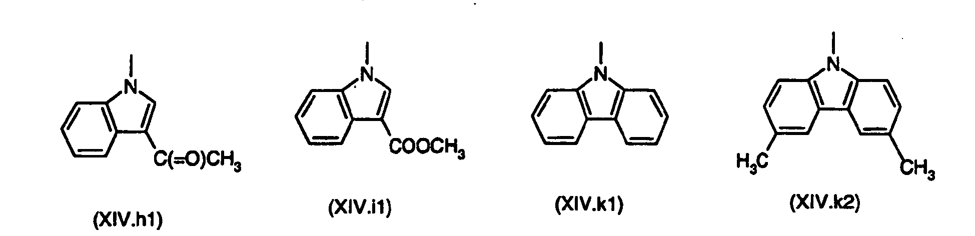

Besonders vorteilhaft ist die 3-Methylindolylgruppe (Skatolylgruppe) der Formel XIV.f1.

Weiterhin kann es von Vorteil sein, wenn zwei an ein Phosphoratom gebundene Gruppen [N] und [N'] beziehungsweise [N"] und [N"'], beispielsweise Pyrrole oder Indole, in den Positionen 2 oder 3 über Brücken A³ miteinander verbunden sind, 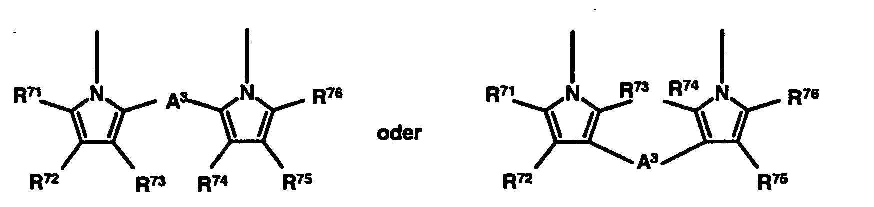 worin
A³ eine Einfachbindung, O, S, SiR⁵¹R⁵², NR⁵³, CR⁵⁴R⁵⁵ oder eine C₂ oder C₁₀-Alkylenbrücke steht, die eine Doppelbindung und/oder einen Alkyl-, Cycloalkyl-, Hetero-cycloalkyl-, Aryl- oder Hetaryl-Substituenten aufweisen kann, oder die durch O, S, SiR⁵¹R⁵² oder NR⁵³ unterbrochen sein kann, wobei R⁵¹, R⁵², R⁵³, R⁵⁴ und R⁵⁵ die oben genannte Bedeutung haben, und
R⁷¹, R⁷², R⁷³, R⁷⁴, R⁷⁵ und R⁷⁶ unabhängig voneinander für Wasserstoff, Alkyl, Alkoxy, Cycloalkyl, Heterocycloalkyl, Aryl, Hetaryl, Halogen, COOR⁵⁶, COO⁻M⁺, SO₃R⁵⁶, SO⁻₃M⁺, NE¹E², NE¹E²E³⁺X⁻, Alkylen-NE¹E², Alkylen-NE¹E²E³⁺X⁻, OR⁵⁶, SR⁵⁶, (CHR⁵⁷CH₂O)_{w}R⁵⁶, (CH₂N(E¹))_{w}R⁵⁶, (CH₂CH₂N(E¹))_{w}R⁵⁶, Halogen, Trifluormethyl, Nitro, Acyl oder Cyano stehen, worin R⁵⁶, E¹, E², E³ und X⁻ wie oben definiert sind.

Dabei können die Gruppen R⁷¹ und R⁷² und/oder R⁷⁵ und R⁷⁶ auch gemeinsam einen fünf-, sechs- oder siebengliedrigen Ring bilden, indem sie zusammen eine gegebenenfalls mit Alkyl, Alkoxy, Cycloalkyl, Heterocycloalkyl, Aryl, Hetaryl oder Halogen substituierte Kette bilden, die drei, vier oder fünf Kohlenstoffatome in der Kette enthält, beispielsweise 1,3-Propylen, 1,4-Butylen, 1,5-Pentylen und bevorzugt 1,4-Buta-1,3-dienylen.

Die genannten Verbindungen können jeweils symmetrisch oder unsymmetrisch substituiert sein.

Die beschriebenen Phosphorverbindungen sind beispielsweise als Liganden für Katalysatoren für die Hydroformylierung von terminalen und internen Olefinen geeignet. Weiterhin ist der Einsatz zur Hydrocyanierung, Hydrierung, Hydrocarboxylierung, Hydroamidierung, Hydroveresterung und Aldolkondensation denkbar.

Derartige Katalysatoren können einen oder mehrere der Phosphorverbindungen als Liganden aufweisen. Zusätzlich zu den Phosphorverbindungen als Liganden können sie noch wenigstens einen weiteren Liganden, der ausgewählt ist unter Hydrid, Alkyl, Cyanid, Halogeniden, Aminen, Carboxylaten, Acetylaceton, Aryl- oder Alkylsulfonaten, Hydrid, CO, Olefinen, Dienen, Cycloolefinen, Nitrilen, N-haltigen Heterocyclen, Aromaten und Heteroaromaten, Ethem, PF₃ sowie ein, zwei- und mehrzähnigen Phosphin-, Phosphinit-, Phosphonit- und Phosphitliganden aufweisen. Diese weiteren Liganden können ebenfalls ein, zwei- oder mehrzähnig sein und an das Metall koordinieren. Geeignete weitere phosphorhaltige Liganden sind z. B. die zuvor als Stand der Technik beschriebenen Phosphin-, Phosphinit- und Phosphitliganden.

Bevorzugt handelt es sich bei dem Metall um eines der VIII. Nebengruppe, besonders bevorzugt um Cobalt-, Rhodium-, Ruthenium-, Palladium- oder Nickelatome in beliebigen Oxidationsstufen. Werden die erfindungsgemäßen Katalysatoren zur Hydroformylierung eingesetzt, so handelt es sich bei dem Metall der VIII. Nebengruppe insbesondere um Rhodium.

Bei Katalysatoren für Hydroformylierungen werden im Allgemeinen unter Hydroformylierungsbedingungen aus den jeweils eingesetzten Katalysatoren oder Katalysatorvorstufen katalytisch aktive Spezies gebildet.

Hierfür wird als Metall vorzugsweise Cobalt, Ruthenium, Rhodium, Palladium, Platin, Osmium oder Iridium und insbesondere Cobalt, Rhodium und Ruthenium in beliebigen Oxidationsstufen verwendet.

Verfahren zur Herstellung der Phosphorverbindungen und den entsprechenden Katalysatoren sind an sich bekannt, beispielsweise aus US 3,903,120, US 5,523,453, US 5,981,772, US 6,127,567, US 5,693,843, US 5,847,191, WO 01/14392, WO 99/13983 und WO 99/64155.

Zur Herstellung der in den Katalysatoren eingesetzten Phosphorverbindungen als Liganden kann beispielsweise Phosphortrichlorid mit zwei Äquivalenten einer pyrrolischen Verbindung umgesetzt werden, wobei ein Diaminochlorphosphin entsteht. Zur Synthese von Diphosphoramiditen kann das erfindungsgemäß (oder auch konventionell) hergestellte Diaminochlorphosphin mit einem Diol zu einem zweizähnigen Liganden umgesetzt werden. Für den Fall, daß unsymmetrische Liganden hergestellt werden sollen, wird zunächst ein Äquivalent des z. B. Diaminochlorphosphins mit dem Diol umgesetzt und anschließend die weitere Kupplungskomponente (z. B. ein Aryldichlorphosphin) zugesetzt.

Die Edukte werden in der gewünschten Stöchiometrie, gegebenenfalls in einem Lösungsmittel gelöst oder dispergiert, d.h. suspendiert oder emulgiert, miteinander vermischt. Dabei kann es sinnvoll sein, die Edukte in eine oder mehrere Zusammensetzungen, d.h. voneinander getrennte Ströme, aufzuteilen, so daß die Reaktion nicht vor der Vermischung stattfindet. Die Hilfsbase, die mit der Säure erfindungsgemäß ein flüssiges Salz bildet, kann einem oder mehreren dieser Ströme beigemischt werden oder getrennt von den Strömen als gesonderter Strom der Reaktion zugeführt werden. Es ist auch möglich, wenn auch weniger bevorzugt, die Hilfsbase erst nach der Reaktion zur Abtrennung der Säure zuzugeben.

Die Edukte oder die genannten Zusammensetzungen werden einem Reaktor zugeführt und unter Reaktionsbedingungen miteinander umgesetzt, die zur Reaktion der Edukte zum Produkt führen. Solche Reaktionsbedingungen sind abhängig von den eingesetzten Edukten und den gewünschten Produkten und in dem in dieser Schrift genannten Stand der Technik angegeben.

Die Reaktion kann kontinuierlich, halbkontinuierlich oder diskontinuierlich erfolgen. Die Temperatur reicht in der Regel von 30°C bis 190°C, bevorzugt 70 bis 120 °C, der Druck ist erfindungsgemäß nicht wesentlich und kann Unter-, Über- oder Normaldruck, beispielsweise von 10 mbar bis 10 bar, bevorzugt 20 mbar bis 5 bar, besonders bevorzugt 50 mbar bis 2 bar und insbesondere 100 mbar bis 1,5 bar betragen. Die Verweilzeit des Reaktionsgemisches im Reaktor kann von wenigen Sekunden bis mehreren Stunden betragen und ist von der Reaktionstemperatur und, in der Regel in geringerem Ausmaß, von dem angelegten Druck abhängig.

Bevorzugt wird die Verweilzeit bei einer kontinuierlichen Reaktionsführung bei einer für die Reaktion ausreichend hohen Temperatur, beispielsweise 30°C bis 190°C, bevorzugt 70 bis 120 °C, kurz gewählt, d.h. von wenigen Sekunden bis ca. 2 Stunden, bevorzugt von 1 Sekunde bis 2 Stunden, besonders bevorzugt von 1 Sekunde bis 1 Stunde, ganz besonders bevorzugt von 1 Sekunde bis 30 Minuten, insbesondere von 1 Sekunde bis 15 Minuten und außergewöhnlich bevorzugt von 1 Sekunde bis 5 Minuten.

In einer besonders bevorzugten Ausführungsform wird die Herstellung der Phosphorverbindungen, bevorzugt solcher mit mehreren Phosphoratomen, besonders bevorzugt solcher mit 2 oder 3 und ganz besonders bevorzugt solcher mit 2 Phosphoratomen, aus den jeweiligen Edukten kontinuierlich bei einer Temperatur von 60°C bis 150°C, bevorzugt bei einer Temperatur oberhalb des Schmelzpunktes des Salzes der verwendeten Hilfsbase mit der freigesetzten Säure bis 130°C, bei einer Verweilzeit unter 1 Stunde, bevorzugt unter 30 Minuten, besonders bevorzugt unter 15 Minuten, ganz besonders bevorzugt von 1 Sekunde bis 5 Minuten, insbesondere von 1 Sekunde bis 1 Minute und außergewöhnlich bevorzugt von 1 bis 30 Sekunden durchgeführt.

Durch eine derartige Ausführungsform wird der Austausch von Substituenten an den Phosphoratomen zurückgedrängt und es ist so möglich, unter überwiegend kinetischer Kontrolle Verbindungen mit mehreren Phosphoratomen, und Phosphorverbindungen mit gemischten Substituenten herzustellen, ohne daß die Substituenten infolge Equilibrierung am Phosphoratom/an den Phosphoratomen ausgetauscht werden.

Während der Reaktion ist für eine gute Durchmischung zu sorgen, beispielsweise durch Rühren oder Umpumpen mit statischen Mischern oder Düsen.

Als Reaktoren können dem Fachmann an sich bekannte Apparate eingesetzt werden, beispielsweise ein oder mehrere kaskadierte Rühr- oder Rohrreaktoren mit innen- und/oder außenliegenden Heizungen und bevorzugt Strahldüsenreaktoren oder Reaktionsmischpumpen.

Der Reaktionsaustrag wird in einen Apparat geführt, in dem sich während der Reaktion entstandene Phasen voneinander trennen können, beispielsweise Phasenscheider oder Mixer-Settler-Apparaturen. In diesem Apparat wird bei einer Temperatur, bei der das Salz der Hilfsbase mit der Säure flüssig ist, eine Phasentrennung der Phase, die überwiegend ionische Flüssigkeit enthält, von der Phase, die überwiegend das gewünschte Reaktionsprodukt enthält, durchgeführt. Falls erforderlich kann Lösungsmittel hinzugegeben werden, um eine Phasentrennung zu beschleunigen.

Aus der Phase, die überwiegend ionische Flüssigkeit enthält, kann die Hilfsbase, wie oben beschrieben, wiedergewonnen werden.

Aus der Phase, die das gewünschte Reaktionsprodukt enthält, kann das Reaktionsprodukt mit an sich bekannten Methoden isoliert und/oder gereinigt werden, beispielsweise durch Destillation, Rektifikation, Extraktion, fraktionierter oder einfacher Kristallisation, Membrantrennverfahren, Chromatographie oder Kombinationen davon.

Bei dem in der Reaktion verwendeten Lösungsmittel kann es sich um die oben angeführten Lösungsmittel handeln.

Die in der Reaktion verwendete Hilfsbase wird in der Regel in, bezogen auf zu erwartende Menge Säure, stöchiometrischer Menge oder leichtem Überschuß eingesetzt, beispielsweise 100 bis 200 Mol% bezogen auf die zu erwartende Menge Säure, bevorzugt 100 bis 150 und besonders bevorzugt 105 bis 125 Mol%. Sofern die zugesetzte Hilfsbase als Lösungsvermittler dient, können auch größere Mengen an Hilfsbase zugesetzt werden, beispielsweise bis zu 1000 mol% oder mehr.

Die Edukte zur Herstellung der gewünschten Phosphorverbindungen sind dem Fachmann an sich bekannt oder leicht erschließbar und sind beispielsweise in dem in dieser Schrift genannten Stand der Technik angegeben, ebenso die stöchiometrischen Verhältnisse, um die Edukte miteinander zur Reaktion zu bringen.

Die Edukte werden möglichst als Flüssigkeiten oder Schmelzen eingesetzt, gegebenenfalls werden sie dazu in einem Lösungsmittel gelöst oder dispergiert. Selbstverständlich ist es aber auch möglich, die Edukte zumindest teilweise als Feststoffe einzusetzen.

Werden sie mit einem Lösungsmittel versetzt, so wird das Lösungsmittel in der Regel in einer derartigen Menge eingesetzt, daß das Gemisch flüssig ist, beispielsweise als Lösung oder Dispersion. Typische Konzentrationen der Edukte bezogen auf die Gesamtmenge der Lösung oder Dispersion sind 5 bis 95 Gew.-%, bevorzugt 10 bis 90 Gew.-%.

Die in der Reaktion freigesetzte Säure kann erfindungsgemäß mit einer der genannten Hilfsbasen unter Ausbildung eines flüssigen Salzes abgefangen werden, so daß die Synthese erheblich vereinfacht werden kann.

Bevorzugt ist die erfindungsgemäße Herstellung von Phosphorigsäuresterdiamiden der Formel (RO)P[N][N'], worin R, [N] und [N'] wie oben definiert ist.

Besonders bevorzugt ist die erfindungsgemäße Herstellung von Diphosphorigsäureesterdiamiden der Formel [N][N']P-O-Z-O-P[N"][N'"], worin Z, [N], [N'], [N"] und [N'"] wie oben definiert sind.

Insbesondere bevorzugt ist die erfindungsgemäße Herstellung der folgenden Verbindungen:

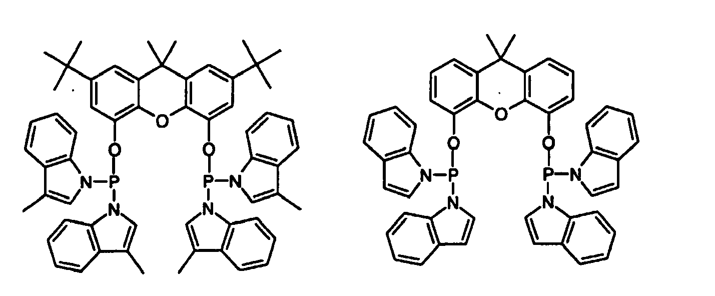

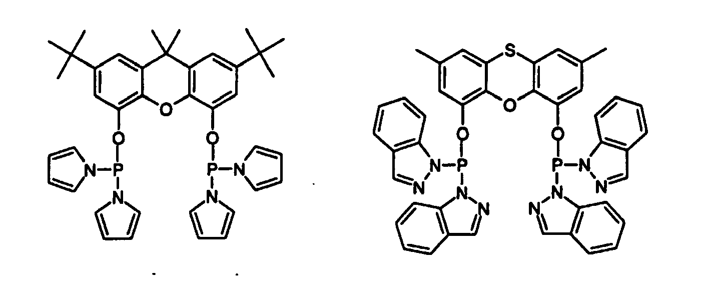

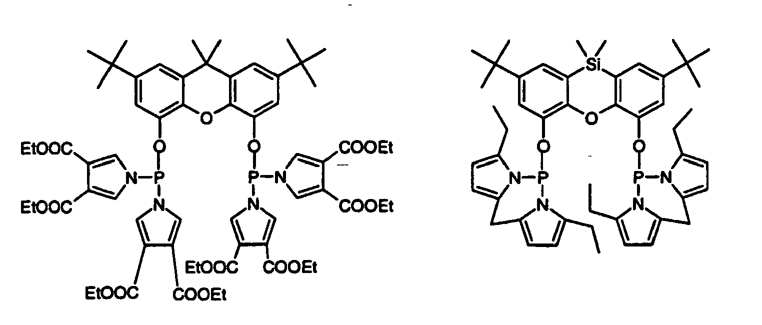

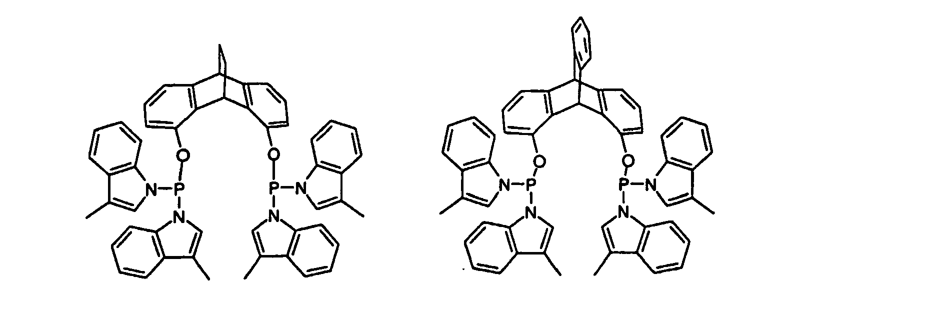

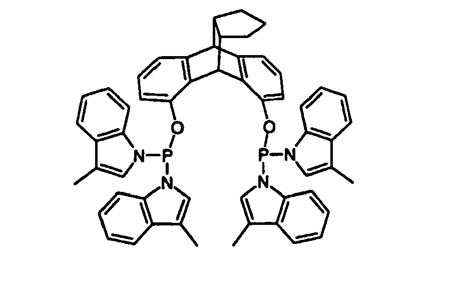

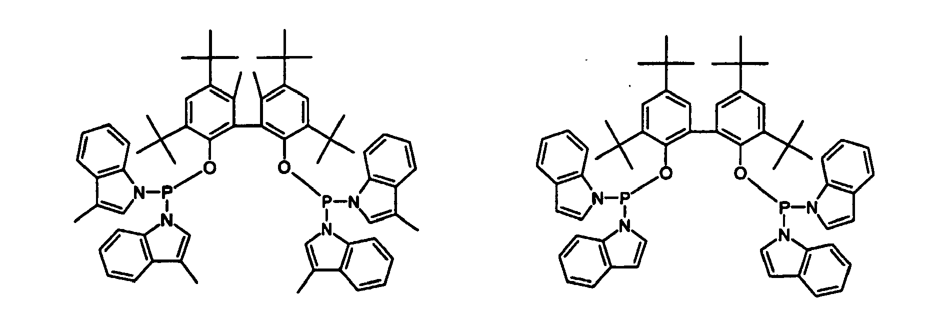

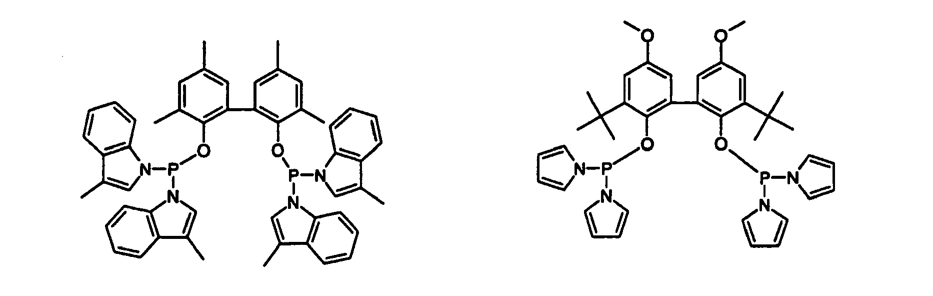

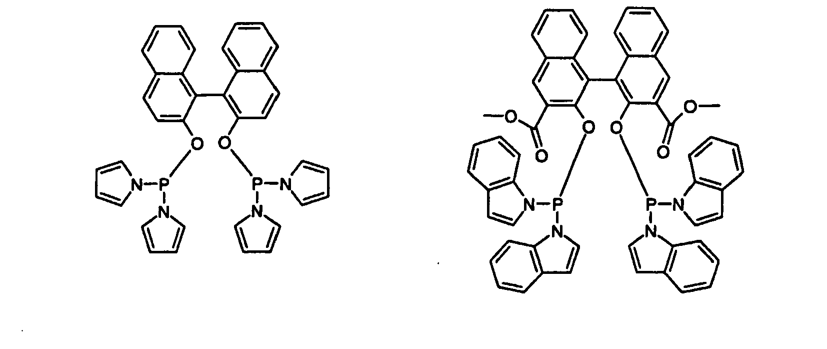

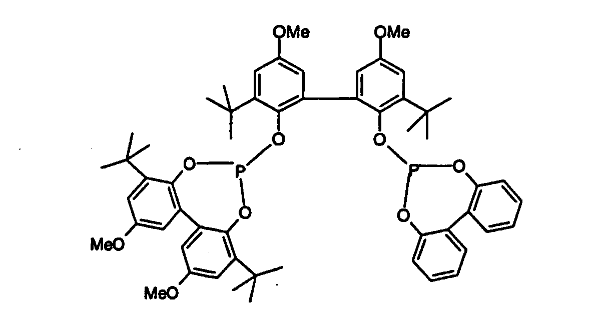

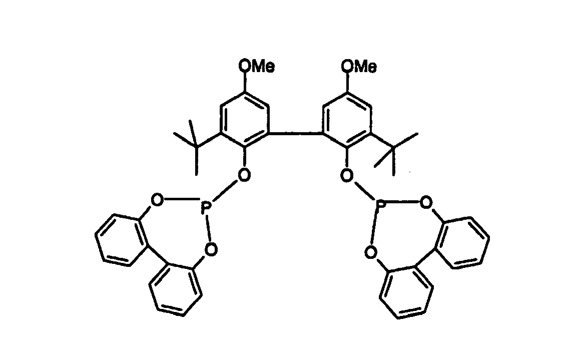

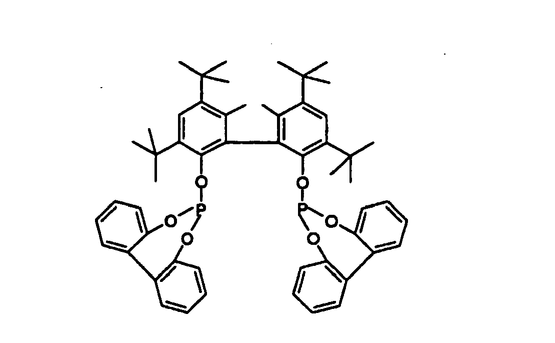

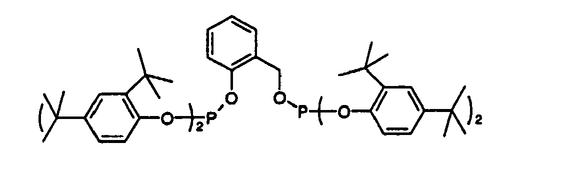

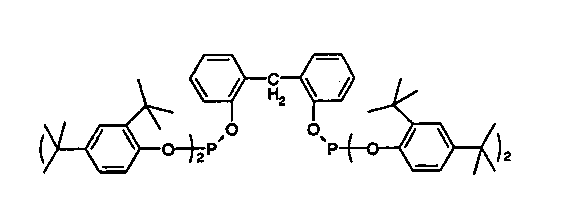

Auf die folgenden, besonders bevorzugten Ausführungsformen wird im Rahmen der vorliegenden Offenbarung im genannten Umfang ausdrücklich Bezug genommen:

In einer besonders bevorzugten Ausführungsform kommen die in US 4,668,651 genannten Verbindungen in Betracht, insbesondere die in Spalte 9, Zeile 25 bis Spalte 16, Zeile 53 und in den Beispielen 1 bis 11 beschriebenen Verbindungen, sowie Ligand A bis Q.

In einer besonders bevorzugten Ausführungsform kommen die in US 4,748,261 genannten Verbindungen in Betracht, insbesondere die in Spalte 14, Zeile 26 bis Spalte 62, Zeile 48 und in den Beispielen 1 bis 14 beschriebenen Verbindungen, sowie U-gand 1 bis 8.

In einer besonders bevorzugten Ausführungsform kommen die in US 4,769,498 genannten Verbindungen in Betracht, insbesondere die in Spalte 9, Zeile 27 bis Spalte 18, Zeile 14 und in den Beispielen 1 bis 14 beschriebenen Verbindungen, sowie Ligand A bis Q.

In einer besonders bevorzugten Ausführungsform kommen die in US 4,885,401 genannten Verbindungen in Betracht, insbesondere die in Spalte 12, Zeile 43 bis Spalte 30 einschließlich und in den Beispielen 1 bis 14 beschriebenen Verbindungen, sowie Ligand 1 bis 8.

In einer besonders bevorzugten Ausführungsform kommen die in US 5,235,113 genannten Verbindungen in Betracht, insbesondere die in Spalte 7 bis Spalte 40, Zeile 11 und in den Beispielen 1 bis 22 beschriebenen Verbindungen.

In einer besonders bevorzugten Ausführungsform kommen die in US 5,391,801 genannten Verbindungen in Betracht, insbesondere die in Spalte 7 bis Spalte 40, Zeile 38 und in den Beispielen 1 bis 22 beschriebenen Verbindungen.

In einer besonders bevorzugten Ausführungsform kommen die in US 5,663,403 genannten Verbindungen in Betracht, insbesondere die in Spalte 5, Zeile 23 bis Spalte 26, Zeile 33 und in den Beispielen 1 bis 13 beschriebenen Verbindungen.

In einer besonders bevorzugten Ausführungsform kommen die in US 5,728,861 genannten Verbindungen in Betracht, insbesondere die in Spalte 5, Zeile 23 bis Spalte 26, Zeile 23 und in den Beispielen 1 bis 13 beschriebenen Verbindungen, sowie Ligand 1 bis 11.

In einer besonders bevorzugten Ausführungsform kommen die in US 6,172,267 genannten Verbindungen in Betracht, insbesondere die in Spalte 11 bis Spalte 40, Zeile 48 und in den Beispielen 1 und 2 beschriebenen Verbindungen, sowie Ligand 1 bis 11.

In einer besonders bevorzugten Ausführungsform kommen die in JP2002-47294 genannten Verbindungen in Betracht.

In dieser Schrift verwendete ppm- und Prozentangaben beziehen sich, falls nicht anders angegeben, auf Gewichtsprozente und -ppm.

### Beispiele

Die Beispiele 1-9 und 11-43 sind nicht Erfindungsgegenstand, dienen aber zur Veranschaulichung der erfindungsgemäßen Verfahren.

### Vergleichsbeispiel 1 Herstellung von Diethoxyphenylphosphin (DEOPP)

In einem mit N2 inertisierten 1000ml-Reaktor mit Impellerrührer wurden 101,4 g Ethanol, 543 g Xylol und 232,7 g Triethylamin vorgelegt und auf 50°C aufgeheizt. In diese Mischung tropfte man innerhalb von 40 Minuten 181,5 g 98,6 %iges Dichlorphenylphosphin, worauf sich eine farblose, gut rührbare Suspension bildete. Durch Kühlen wurde die Reaktionstemperatur auf 50°C gehalten. Nach vollständiger Zugabe des Dichlorphenylphosphins wurde der Ansatz noch 60 Minuten bei 75-80°C nachgerührt und anschließend das ausgefallene Hydrochlorid abgesaugt und mit kaltem Xylol gewaschen. Filtrat und Waschxylol wurden vereinigt (insges. 859,9 g) und mittels GC mit internem Standard untersucht. Die xylolische Lösung enthielt 11,8 % Diethoxyphenylphosphin, was einer Ausbeute von 51 % entspricht.

### Vergleichsbeispiel 2 Herstellung von Diethoxyphenylphosphin (DEOPP)

In einem mit N₂ inertisierten 1000ml-Reaktor mit Impellerrührer wurden 90,9 g Ethanol und 382,2 g Tributylamin vorgelegt und auf 70°C aufgeheizt. In diese Mischung tropfte man innerhalb von 40 Minuten 162,7 g 98,6 %iges Dichlorphenylphosphin, worauf sich eine farblose Lösung bildete, die in der Wärme flüssig war und nach Abkühlen auf Ramutemperatur zu einem farblosen, kristallinem Feststoff erstarrte. Durch Kühlen wurde die Reaktionstemperatur auf 50°C gehalten. Nach vollständiger Zugabe des Dichlorphenylphosphins wurde der Ansatz noch 60 Minuten bei 75-80°C nachgerührt. Die 625,8 g Reaktionsaustrag enthielten laut GC mit internem Standard 23,7 % Diethoxyphenylphosphin, was einer Ausbeute von 82,7 % entspricht.

### Beispiel 1: Herstellung von Diethoxyphenylphosphin (DEOPP)

In einem mit N₂ inertisierten 1000ml-Reaktor mit Schrägblattrührer wurden 188,9 g (2,3 mol) 1-Methylimidazol und 101,4 g (2,2 mol) Ethanol vorgelegt. Innerhalb 90 min wurden nun 181,5 g (1,0 mol) 98,6 %iges Dichlorphenylphosphin dosiert. Dabei wurde zunächst eine Erwärmung auf 60°C zugelassen (Dauer: 6 min) und anschließend durch Kühlen die Temperatur bei der weiteren Zugabe auf 60°C gehalten. Nach Zulaufende war der Ansatz noch flüssig, kristallisierte aber in der Nachrührzeit von 45 min aus. Nach Aufheizen auf 80°C war das Reaktionsgemisch wieder restlos flüssig. Nach weiterem einstündigen Rühren wurde der Rührer ausgeschaltet. Es bildeten sich rasch zwei gut getrennte Phasen aus. Nach Phasentrennung bei 80 °C wurden 199,4 g einer klaren, farblosen Oberphase (DEOPP-Gehalt nach GC: 96,1 %; Gehalt an 1-Methyl-imidazol 1,7 %) und 266,4 g Unterphase ("ionische Flüssigkeit") erhalten.

Die Oberphase wurde im Vakuum über eine 40 cm Kolonne mit 5 mm Raschigringen destilliert. Dabei erhielt man 15,8 g eines klaren, farblosen Vorlaufs (GC: 76,9 % DEOPP-Gehalt) und 177,5 g eines farblosen Hauptlaufs (GC: 99,4 % DEOPP). Im Kolben blieben nur 4,3 g Sumpf zurück, der nach GC noch 11,1 % DEOPP enthielt. Die DEOPP-Ausbeute nach Destillation betrug 95,9 %.

### Beispiel 2

### Herstellung von Triethylphosphit (TEP)

In einem mit N₂ inertisierten 1000ml-Reaktor mit Schrägblattrührer wurden 425 g 1-Methylimidazol und 228,1 g Ethanol vorgelegt. Innerhalb 190 min wurden nun unter Eiskühlung bei 23-33°C Innentemperatur 206 g Phosphortrichlorid zugetropft. Die Reaktion verlief exotherm, so daß gekühlt wurden mußte, um diese Temperatur zu halten. Nach etwa der halben Zugabe wurde der Reaktionsansatz trübe, wobei zwei flüssige Phasen erhalten wurden. Die obere bestand laut GC aus 90,0 % Triethylphosphit, die untere aus dem Hydrochlorid des 1-Methylimidazol. Vor der Phasentrennung wurde auf 78°C aufgeheizt. Es wurden 231,4 g einer farblosen Oberphase und 611,9 g einer klaren Unterphase erhalten. Die Oberphase wurde im Vakuum über eine 30cm Glaskolonne mit einer Sulzer DX Packung destilliert. Es wurden 177 g Triethylphosphit mit einer Reinheit von 99 % erhalten. Im Vor- und Nachlauf waren weitere 28,3 g Triethylphosphit enthalten. Die Gesamtausbeute betrug 82,4 %.

### Beispiel 3

### Herstellung von Diethoxyphenylphosphin (DEOPP)

In einem 250 ml Glaskolben mit Teflonblattrührer wurden 85,7 g 2-Methylpyridin und 40,5 g Ethanol vorgelegt. Unter Kühlung wurden innerhalb 25 min 71,6 g Dichlorphenylphosphin (98,6 %ig) zugetropft, so daß die Innentemperatur bei 20-29°C blieb. Während der Zugabe fiel das Hydrochlorid des 2-Methylpyridins aus. Nach vollständiger Zugabe wurde der Ansatz aufgeheizt, wobei das Hydrochlorid ab etwa 70°C zu schmelzen begann. Es bildeten sich zwei klare, scharf getrennte flüssige Phasen aus, wobei 75,5 g Oberphase und 115,8 g Unterphase erhalten wurden. Die Oberphase enthielt 81,6 % DEOPP, so daß die Ausbeute 77,7 % betrug.

Wurde die Unterphase mit wäßriger Natronlauge neutralisiert, so bildete sich erneut ein Zweiphasensystem aus, wobei die untere aus einer wäßrigen Kochsalzlösung und die obere aus dem freigesetzten 2-Methylpyridin bestand, das auf diese Weise durch eine einfache Flüssig-flüssig-Phasentrennung zurückgeführt werden konnte.

### Beispiel 4

### Herstellung von Ethoxydiphenylphosphin (EODPP)

In einem mit N₂ inertisierten 1000ml-Reaktor mit Schrägblattrührer wurden 141,7 g 1-Methylimidazol und 76,0 g Ethanol vorgelegt, in das 315,8 g Chlordiphenylphosphin innerhalb 30 min getropft wurden, wobei sich zwei flüssige Phasen ausbildeten. Die Innentemperatur wurde unterhalb von 65°C gehalten. Nach vollständiger Zugabe heizte man auf 75°C auf, rührte 45 min und trennte die Phasen, wobei 194,3 g Unter- und 332,8 g Oberphase erhalten wurden. Die Oberphase enthielt It. GC zu 96,6 % das Produkt EODPP. Zur weiteren Aufreinigung wurde die Oberphase im Vakuum über eine Glaskolonne mit Raschigringen destilliert, wobei 292,5 g 99,4 %iges EODPP erhalten wurden. Zusammen mit dem EODPP im Vorlauf betrug die Gesamtausbeute 92,2 %.

Die Unterphase, die aus dem flüssigen Hydrochlorid des 1-Methylimidazols bestand, wurde mit 244,1 g 25 %iger Natronlauge versetzt. Um das ausgefallene Kochsalz vollständig zu lösen, wurden weitere 94,3 g Wasser zugegeben, bis eine klare Lösung erhalten wurde. Nach Zugabe von 450 g n-Propanol fiel erneut Kochsalz aus, das nach weiterer Zugabe von 69,8 g Wasser wieder in Lösung gebracht wurde. Man erhielt zwei flüssige Phasen, wobei die 739,3 g Oberphase 19,99 % Wasser und 16.7 % 1-Methylimidazol enthielt. Das sind 94,9 % der bei der Synthese eingesetzten Menge an 1-Methylimidazol. Die 304,2 g Unterphase enthielt neben dem Kochsalz 70,6 % Wasser und 2,2 % 1-Methylimidazol. Durch erneute Extraktion mit n-Propanol konnte der Gehalt an 1-Methylimidazol in der wässrigen Phase auf 0,4 % gesenkt werden. 1-Methylimidazol konnte nun wiedergewonnen werden, indem das Gemisch aus Propanol und Wasser aus der Oberphase der ersten Extraktion abdestilliert wurde.

### Beispiel 5

### Kontinuierliche Herstellung von Ethoxydiphenylphosphin (EODPP)

In einen mit Stickstoff inertisierten Reaktor mit dreistufigem Schrägblattrührer wurden kontinuierlich bei 80°C folgende Einsatzstoffe zugefördert: 1 ) Mischung aus 110,7 g Ethanol und 205,8 g 1-Methylimidazol 2) Chlordiphenylphosphin (99,4 %ig). Strom 1) wurde mit 330 ml/h und Strom 2) mit 380 ml/h zugegeben. Beide Zuläufe erfolgten getaucht. Der Reaktor war mit einem Überlauf ausgestattet, aus dem kontinuierlich Reaktionsgemisch ablaufen konnte. Das Reaktorvolumen bis zum Überlauf betrug 710 ml. Die Reaktionstemperatur wurde auf 80°C gehalten. Um das System ins Gleichgewicht zu bringen, wurde der Austrag der ersten 4 h verworfen. Anschließend wurde der Austrag über eine Zeitdauer von 1 h gesammelt und bilanziert. Der Austrag bestand aus zwei flüssigen Phasen. Innerhalb von einer Stunde wurden 497,2 g Ober- und 280,8 g Unterphase gesammelt. Die Oberphase bestand zu 96,8 % aus EODPP. Die Oberphase wurde anschließend im Vakuum über eine mit Raschigringen gefüllte Kolonne destilliert, wobei 438,2 g 99,74 %iges EODPP erhalten wurden. Zusammen mit dem EODPP im Vorlauf betrug die Gesamtausbeute 96,7 %.

### Beispiel 6

### Kontinuierliche Herstellung von Ethoxydiphenylphosphin (EODPP)

In einer Reaktionsmischpumpe wurden kontinuierlich folgende Zulaufströme gemischt: 1) Mischung aus 159,2 g 1-Methylimidazol und 85,4 g Ethanol 2) 372,8 g Chlordiphenylphosphin (99,1 %ig). Vom Strom 1) wurden 1257 g/h zugegeben, vom Strom 2) 1928 g/h. Das Volumen der Mischkammer betrug 3,3 ml. Der Kopf der Reaktionsmischpumpe wurde auf 120°C thermostatisiert. Das System wurde 5 min ins Gleichgewicht gebracht. Anschließend wurde der Austrag über 11 min gesammelt, um zu bilanzieren. Während der Bilanzfahrt wurde die Menge an Einsatzstoffen durch Wiegen der Vorlagen bestimmt. Es wurden 372,8 g Chlordiphenylphosphin zugegeben. Der Austrag bestand aus zwei flüssigen Phasen. In den 11 min wurden 392,2 g Ober- und 218,3 g Unterphase gesammelt. Die Oberphase bestand zu 96,5 % aus EODPP, so daß die gaschromatographisch bestimmte Ausbeute 98,2 % betrug. Die Verweilzeit der Reaktanden in der Mischkammer betrug 4 s. Dadurch ergab sich eine Raum-Zeit-Ausbeute von 0,69•10⁶kgm⁻³h⁻¹.

### Beispiel 7

### Kontinuierliche Herstellung von Ethoxydiphenylphosphin (EODPP)

In einer Reaktionsmischpumpe wurden kontinuierlich folgende Zulaufströme gemischt: 1) Mischung aus 156,7 g 1-Methylimidazol und 84,1 g Ethanol 2) 370,0 g Chlordiphenylphosphin (99,1 %ig). Vom Strom 1) wurden 167,5 g/h zugegeben, vom Strom 2) 257,4 g/h. Das Volumen der Mischkammer betrug 3,3 ml. Der Kopf der Reaktionsmischpumpe wurde auf 80°C thermostatisiert. Das System wurde 60 min ins Gleichgewicht gebracht. Anschließend wurde der Austrag über 87 min gesammelt, um zu bilanzieren. Während der Bilanzfahrt wurde die Menge an Einsatzstoffen durch Wiegen der Vorlagen bestimmt. Es wurden 370,0 g Chlordiphenylphosphin zugegeben. Der Austrag bestand aus zwei flüssigen Phasen. In den 87 min wurden 389,3 g Ober- und 219,2 g Unterphase gesammelt. Die Oberphase bestand zu 96,8 % aus EODPP, so daß die gaschromatographisch bestimmte Ausbeute 98,5 % betrug. Die Verweilzeit der Reaktanden in der Mischkammer betrug 30 s.

### Beispiel 8

### Kontinuierliche Herstellung von Diethoxyphenylphosphin (DEOPP)

In einer Reaktionsmischpumpe wurden kontinuierlich folgende Zulaufströme gemischt: 1) Mischung aus 237,1 g 1-Methylimidazol und 127,2 g Ethanol 2) 225,8 g Dichlorphenylphosphin. Vom Strom 1) wurden 385,6 g/h zugegeben, vom Strom 2) 239,0 g/h. Das Volumen der Mischkammer betrug 3,3 ml. Der Kopf der Reaktionsmischpumpe wurde auf 80°C thermostatisiert. Das System wurde 30 min ins Gleichgewicht gebracht. Anschließend wurde der Austrag über 58 min gesammelt, um zu bilanzieren. Während der Bilanzfahrt wurde die Menge an Einsatzstoffen durch Wiegen der Vorlagen bestimmt. Es wurden 225,8 g Dichlorphenylphosphin zugegeben. Der Austrag bestand aus zwei flüssigen Phasen. In den 58 min wurden 249,0 g Ober- und 335,6 g Unterphase gesammelt. Die Oberphase bestand zu 95,4 % aus DEOPP, so daß die gaschromatographisch bestimmte Ausbeute 95,5 % betrug. Die Verweilzeit der Reaktanden in der Mischkammer betrug 20 s.

### Beispiel 9

### Kontinuierliche Herstellung von Diethoxyphenylphosphin (DEOPP)

In einer Reaktionsmischpumpe wurden kontinuierlich folgende Zulaufströme gemischt: 1) Mischung aus 212,0 g 1-Methylimidazol und 113,7 g Ethanol 2) 201,7 g Dichlorphenylphosphin 3) rückgeführte Oberphase des Reaktionsaustrages. Vom Strom 1) wurden 1543,5 g/h zugegeben, vom Strom 2) 955,9 g/h, vom Strom 3) 2377 ml/h. Das Volumen der Mischkammer betrug 3,3 ml. Der Kopf der Reaktionsmischpumpe wurde auf 80°C thermostatisiert. Das System wurde 5 min ins Gleichgewicht gebracht. Anschließend wurde der Austrag über 12 min gesammelt, um zu bilanzieren. Während der Bilanzfahrt wurde die Menge an Einsatzstoffen durch Wiegen der Vorlagen bestimmt. Es wurden 201,7 g Dichlorphenylphosphin zugegeben. Der Austrag bestand aus zwei flüssigen Phasen, die in einem kontinuierlich betriebenen Phasenscheider getrennt wurden. Ein Teil der Oberphase wurde in den Prozeß zurückgeführt. In 12 min Bilanzfahrt wurden 227,0 g Ober- und 300,6 g Unterphase gesammelt. Die Oberphase bestand zu 95,2 % aus DEOPP, so daß die Ausbeute 97,2 % betrug. Die Verweilzeit der Reaktanden in der Mischkammer betrug 2,5 s. Dadurch ergibt sich eine Raum-Zeit-Ausbeute von 0,36•10⁶kgm⁻³h⁻¹.

### Beispiel 10

### Regenerierung von 1-Methylimidazol Hydrochlorid

Analog zu Beispiel 1 wurden aus 181,5 g Dichlorphenylphosphin, 101,4 g Ethanol und 189 g 1-Methylimidazol DEOPP hergestellt, wobei 202,2 g Oberphase mit einem DEOPP-Gehalt von 93,9 % und 265,5 g Unterphase anfallen. Die Oberphase enthält zudem noch 3,7 g 1-Methylimidazol. Die Unterphase wurde mit 169,6 g Paraffinöl gemischt. In diese Mischung wurde nun 168 g 50 %ige Natronlauge getropft, wobei eine gut rührbare Suspension erhalten wurde. Nach der Zugabe von 12,9 g Xylol und 78,4 g rückgeführten Xylols aus einem vorherigen Versuch, das noch 3,8 g 1-Methylimidazol enthielt, wurde mit Hilfe von Xylol bei Wasser ausgekreist. Insgesamt wurden 132,7 g Wasser ausgekreist. Wenn kein Wasser mehr abgeschieden wurde, destillierte man bei 30-85 mbar und 57 - 90°C Kopftemperatur das Xylol aus dem Reaktionsgemisch über eine 30 cm Füllkörperkolonne, wobei 88,4 g Destillat erhalten wurden, das 21,8 g 1-Methylimidazol enthielt. Das Destillat wurde im nächsten Vesuch als rückgeführtes Xylol wieder eingesetzt, so daß darin enthaltenes 1-Methylimidazol immer wieder in den Prozeß zurückgeführt wurde. Nach der Xyloldestillation wurde bei 30 mbar und 90°C Kopftemperatur das 1-Methylimidazol abdestilliert. Es wurden 164,0 g 1-Methylimidazol wiedergewonnen, das einen Gehalt von 99,7 % aufweist. Der Wassergehalt des destillierten 1-Methylimidazols betrug 0,06 %.

Der Destillationssumpf wurde nun mit 350 g Wasser versetzt, um das im Weißöl suspendierte Kochsalz zu lösen. Es bildeten sich zwei Phasen aus. Die 475,7 g Unterphase enthielten das Kochsalz und 0,3 % (1,4 g) 1-Methylimidazol. Die 161,1 g Oberphase bestanden aus dem Weißöl, das als inertes Suspendierhilfsmittel ebenfalls wieder in den Prozeß zurückgeführt wurde. Von den insgesamt eingesetzten 192,8 g 1-Methylimidazol (189,0 g frisch und 3,8 g im rückgeführten Xylol) wurden 164,0 g als Reinstoff wiedergewonnen. Weitere 21,8 g befanden sich im abdestillierten Xylol, das in den Prozeß zurückgeführt wurde und damit erhalten bleibt. Insgesamt konnten somit 185,8 g (96 %) des 1-Methylimidazols zurückgeführt wurden.

### Beispiel 11

51 g Essigsäure wurden in 120,8 g Cyclohexan gelöst. Um die Säure wieder zu entfernen, wurden nun 69,80 g 1-Methylimidazol in die Lösung gegeben, worauf sich ein Zweiphasengemisch, bestehend aus 119,4 g Oberphase (Cyclohexan) und 122,5 g Unterphase (lonische Flüssigkeit = 1-Methylimidazoliumacetat) bildete. Während der Zugabe von 1-Methylimidazol stieg die Temperatur aufgrund der Salzbildung bis auf 40°C an. Durch Kühlen mit einem Eisbad wurde die Temperatur bei der weiteren Zugabe auf 40°C gehalten. Nach Abkühlen konnte die Essigsäure nahezu vollständig in Form der gebildeten lonischen Flüssigkeit, die mit Cyclohexan nicht mischbar ist, von dem Lösemittel über eine Flüssig-Flüssig-Phasentrennung abgetrennt werden.

### Beispiel 12

### Kontinuierliche Herstellung des folgenden Chelatphosphonits:

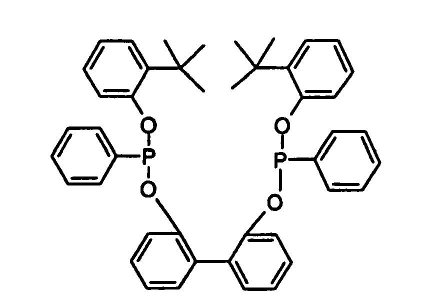

In einer Reaktionsmischpumpe wurden kontinuierlich folgende Zulaufströme gemischt:
1) Zusammensetzung: Mischung aus 11,9 g 1-Methylimidazol, 11,8 g o-Biphenol und 35,1 g Toluol und
2) Zusammensetzung: Mischung aus 38,4 g (2-tert-butylphenoxy)-chlorphenylphosphin und 153,5 g Toluol.

Vom Strom 1 ) wurden 681 ml/h, vom Strom 2) 2373 ml/h dosiert. Das Volumen der Mischkammer betrug 3,3 ml. Der Kopf der Reaktionsmischpumpe wurde auf 120°C thermostatisiert. Das System wurde 3 min ins Gleichgewicht gebracht. Anschließend wurde der Austrag über 7 min gesammelt, um zu bilanzieren. Die Temperatur des Reaktionsmediums am Ausgang der Reaktionsmischpumpe betrug 100°C. Der Austrag bestand aus zwei flüssigen Phasen, die in einem Gefäß gesammelt und anschließend getrennt wurden. In 7 min Bilanzfahrt wurden 233,9 g Ober- und 14,0 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Selektivität des erwünschten Chelatphosphonits gegenüber den unerwünschten monodentaten Phosphoniten wurde mit Hilfe von 31 P-NMR-Spektren ermittelt. Sie betrug 93,8 % zugunsten des Chelatphosphonits. Der Umsatz war vollständig.

### Beispiel 13

Die Synthese des Chelatphosphonits aus Beispiel 12 wurde durchgeführt wie unter Beispiel 12 beschrieben. Es wurden verschiedene Parameter variiert. Der Kopf der Reaktionsmischpumpe wurde so thermostatisiert, daß die in der Tabelle angegebenen Endtemperaturen des Reaktionsgemisches am Ausgang der Pumpe erhalten werden konnten. Die Ergebnisse sind in folgender Tabelle zusammengefaßt.

| Zusammensetzung Strom 1 | Zusammensetzung Strom 2 | Zulauf Strom 1 | Zulauf Strom 2 | Temperatur Reaktorausgang | Selektivität Chelatphosphonit in Bezug auf monodentate Phosphonite |
|---|---|---|---|---|---|
| 33,3 g MIA | 106,0 g TBCP | 1603 ml/h | 1367 ml/h | 105,5°C | 96,6 % |
| 32,8 g BP | | | | | |
| 98,0 g Tol | 45,4 g Tol | | | | |
| 37,3 g MIA | 118,7 g TBCP | 1603 ml/h | 1367 ml/h | 90,5°C | 97,3 % |
| 36,7 g BP | | | | | |
| 109,7 g Tol | 50,9 g Tol | | | | |
| 41,3 g MIA | 130,9 g TBCP | 1603 ml/h | 1367 ml/h | 76,8°C | 98,6 % |
| 40,7 g BP 121,6 g Tol | 56,1 g Tol | | | | |
| 41,3 g MIA | 130,9 g TBCP | 1603 ml/h | 1367 ml/h | 76,8 °C | 98,6 % |
| 40,7 g BP | 56,1 g Tol | | | | |
| 121,6 g Tol | | | | | |
| 21,2 g MIA | 71,2 g TBCP | 1270 ml/h | 1156 ml/h | 76,3 °C | 99,3 % |
| 20,9 g BP | 30,5 g Tol | | | | |
| 62,5 g Tol | | | | | |

| | | | | | |
|---|---|---|---|---|---|
| MIA =1-Methylimidazol BP = o-Biphenol Tol = Toluol TBCP = (2-tert-butylphenoxy)-chlorphenylphosphin | | | | | |

Der Umsatz war bei allen Varianten vollständig.

### Beispiel 14

### Kontinuierliche Herstellung des folgenden Chelatphosphonits:

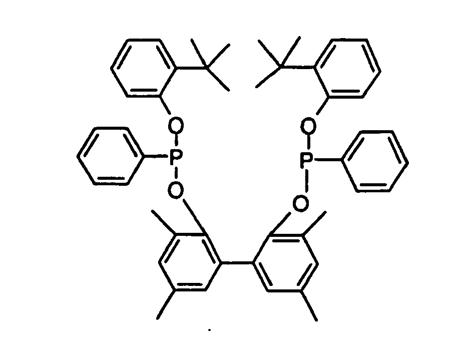

In einer Reaktionsmischpumpe wurden kontinuierlich folgende Zulaufströme gemischt:
1) Zusammensetzung: Mischung aus 28,0 g 1-Methylimidazol, 36,1 g 2,2',4,4'-Tetramethyl-o-Biphenol und 116,4 g Toluol und
2) Zusammensetzung: Mischung aus 88,4 g (2-tert-butylphenoxy)-chlorphenylphosphin und 37,9 g Toluol.

Vom Strom 1) wurden 1817 ml/h, vom Strom 2) 1153 ml/h dosiert. Das Volumen der Mischkammer betrug 3,3 ml. Das System wurde 2 min ins Gleichgewicht gebracht. Anschließend wurde der Austrag über 5 min gesammelt, um zu bilanzieren. Die Temperatur des Reaktionsmediums am Ausgang der Reaktionsmischpumpe betrug 76,3°C. Der Austrag bestand aus zwei flüssigen Phasen, die in einem Gefäß gesammelt und anschließend getrennt wurden. In 5 min Bilanzfahrt wurden 264,3 g Ober- und 40,1 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Selektivität des erwünschten Chelatphosphonits gegenüber den unerwünschten monodentaten Phosphoniten wurde mit Hilfe von 31 P-NMR-Spektren ermittelt. Sie betrug 95,6 % zugunsten des Chelatphosphonits. Der Umsatz war vollständig. Die Unterphase (ionische Flüssigkeit) enthielt nur ca. 300 ppm an phosphorhaltigen Nebenkomponenten.

### Beispiel 15

### Kontinuierliche Herstellung des folgenden Chelatphosphonits:

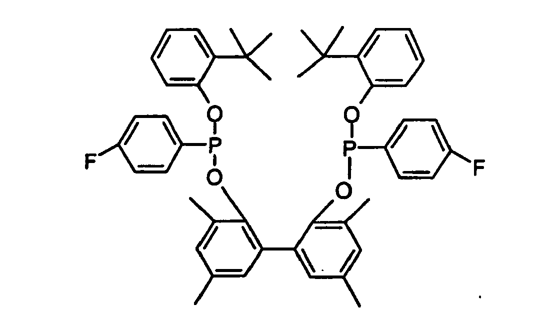

In einer Reaktionsmischpumpe wurden kontinuierlich folgende Zulaufströme gemischt:
1) Zusammensetzung: Mischung aus 188,9 g 1-Methylimidazol, 249,1 g 2,2',4,4'-Tetramethyl-o-Biphenol und 807,4 g Toluol und
2) Zusammensetzung: Mischung aus 664,7 g (2-tert-butylphenoxy)-p-fluorphenyl-chlorphosphin und 284,9 g Toluol.

Vom Strom 1) wurden 1781 ml/h, vom Strom 2) 1189 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69,8°C. Der Austrag bestand aus zwei flüssigen Phasen, die in einem Gefäß gesammelt und anschließend getrennt wurden. In 275 min Bilanzfahrt wurden 799,6 g Ober- und 98,9 g Unterphase gesammelt. Die Oberphase war eine toluolische Lösung der Reaktionsprodukte, die Unterphase war das Hydrochlorid von 1-Methytimidazol, das oberhalb von 75°C als ionische Flüssigkeit anfiel. Die Ausbeute an isoliertem Wertprodukt betrug 302,9 g (93,4 % d.Th).

### Beispiel 16

### Kontinuierliche Herstellung des folgenden Chelatphosphonits:

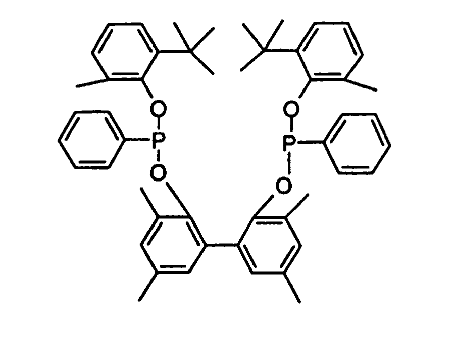

In einer Reaktionsmischpumpe wurden kontinuierlich folgende Zulaufströme gemischt:
1) Mischung aus 188,9 g 1-Methylimidazol, 249,1 g 2,2',4,4`-Tetramethyl-o-Biphenol und 807,4 g Toluol und
2) Zusammensetzung: Mischung aus 696,1 g (2-tert-Butyl-6-methylphenoxy)-chlorphenylphosphin und 298,3 g Toluol.

Vom Strom 1) wurden 1730 ml/h, vom Strom 2) 1238 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69,5°C. Der Austrag bestand aus zwei flüssigen Phasen, die in einem Gefäß gesammelt und anschließend getrennt wurden. In 275 min Bilanzfahrt wurden 798,1 g Ober- und 93,3 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 298,3 g (95,2 % d.Th).

### Beispiel 17

### Kontinuierliche Herstellung des folgenden Chelatphosphits:

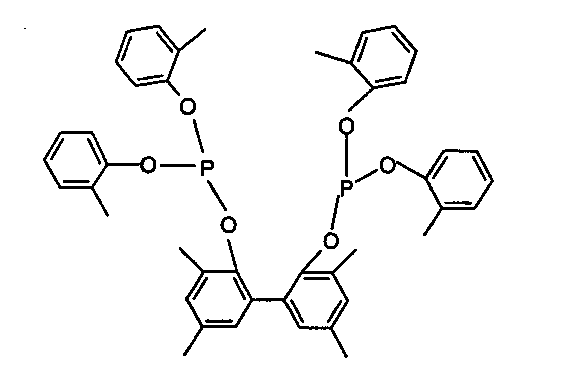

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 660,5 g (Di-o-kresyl)-chlorphosphin und 283,1 g Toluol.

Vom Strom 1) wurden 1793 ml/h, vom Strom 2) 1176 ml/h dosiert. Das Volumen der Mischkammer betrug 3,3 ml. Das System wurde 2 min ins Gleichgewicht gebracht. Anschließend wurde der Austrag über 160 min gesammelt, um zu bilanzieren. Die Temperatur des Reaktionsmediums am Ausgang der Reaktionsmischpumpe betrug 70,1 °C. Der Austrag bestand aus zwei flüssigen Phasen, die in einem Gefäß gesammelt und anschließend getrennt wurden. In 160 min Bilanzfahrt wurden 470,8 g Ober- und 60,8 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 166,6 g (93,0 % d.Th).

### Beispiel 18

### Kontinuierliche Herstellung des folgenden Chelatphosphinits:

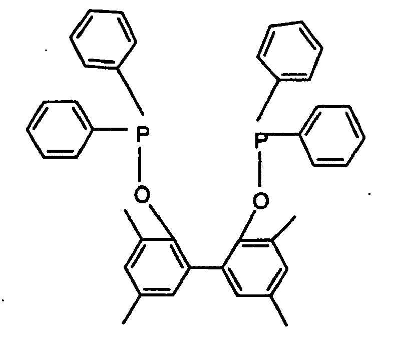

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 445,8 g Diphenylchlorphosphin und 191,1 g Toluol.

Vom Strom 1) wurden 1991 ml/h, vom Strom 2) 906 ml/h dosiert. Das Volumen der Mischkammer betrug 3,3 ml. Das System wurde 2 min ins Gleichgewicht gebracht. Anschließend wurde der Austrag über 218 min gesammelt, um zu bilanzieren. Die Temperatur des Reaktionsmediums am Ausgang der Reaktionsmischpumpe betrug 70,1°C. Der Austrag bestand aus zwei flüssigen Phasen, die in einem Gefäß gesammelt und anschließend getrennt wurden. In 218 min Bilanzfahrt wurden 641,8 g Ober- und 93 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 152,3 g (67,4 % d.Th).

### Beispiel 19

### Kontinuierliche Herstellung des folgenden Chelatphosphonits:

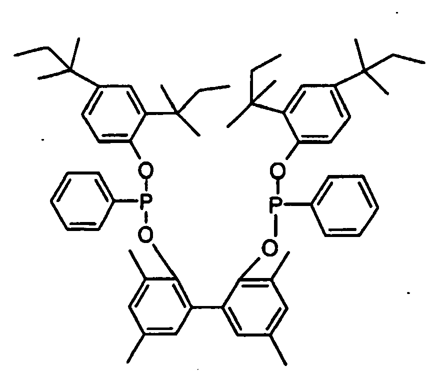

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 828,1 g (2,4-Di-isoamylphenoxy)- chlorphenylphosphin und 354,9 g Toluol.

Vom Strom 1) wurden 1532 ml/h, vom Strom 2) 1395 ml/h dosiert. Das Volumen der Mischkammer betrug 3,3 ml. Das System wurde 2 min ins Gleichgewicht gebracht. Anschließend wurde der Austrag über 275 min gesammelt, um zu bilanzieren. Die Temperatur des Reaktionsmediums am Ausgang der Reaktionsmischpumpe betrug 69°C. Der Austrag bestand aus zwei flüssigen Phasen, die in einem Gefäß gesammelt und anschließend getrennt wurden. In 275 min Bilanzfahrt wurden 787,9 g Ober- und 85,3 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 304 g (89,6 % d.Th).

### Beispiel 20

### Kontinuierliche Herstellung des folgenden Chelatphosphonits:

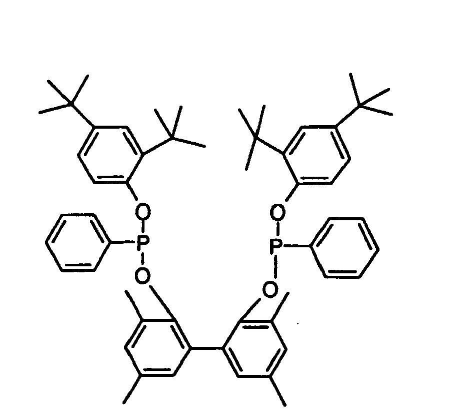

In einer Reaktionsmischpumpe wurden kontinuierlich folgende Zulaufströme gemischt:
1) Mischung aus 188,9 g 1-Methylimidazol, 249,1 g 2,2',4,4'-Tetramethyl-o-Biphenol und 807,4 g Toluol und
2) Zusammensetzung: Mischung aus 738,3 g (2,4-Di-tert-butylphenoxy)- chlorphenylphosphin und 316,4 g Toluol.

Vom Strom 1) wurden 1664 ml/h, vom Strom 2) 1308 ml/h dosiert. Das Volumen der Mischkammer betrug 3,3 ml. Das System wurde 2 min ins Gleichgewicht gebracht. Anschließend wurde der Austrag über 233 min gesammelt, um zu bilanzieren. Die Temperatur des Reaktionsmediums am Ausgang der Reaktionsmischpumpe betrug 75,8°C. Der Austrag bestand aus zwei flüssigen Phasen, die in einem Gefäß gesammelt und anschließend getrennt wurden. In 233 min Bilanzfahrt wurden 663,9 g Ober- und 79,8 g Unterphase gesammelt. Die Oberphase war eine toluolische Lösung der Reaktionsprodukte, die Unterphase war das Hydrochlorid von 1-Methylimidazol, das oberhalb von 75°C als ionische Flüssigkeit anfiel. Die Ausbeute an isoliertem Wertprodukt betrug 267 g (94,7 % d.Th).

### Beispiel 21

In einem 1l-Kolben mit thermostatiertem Doppelmantel, mechanischer Rührung, Thermometer und Rückflußkühler wurden unter Argonatmosphäre ein Gemisch aus 1,7 mol PCl₃ und 0,6 mol AlCl₃ (98% Reinhheit) bei 73 °C vorgelegt. Anschließend wurden innerhalb von 30 min 0,4 mol Fluorbenzol hinzugegeben, wobei ein leichter Argonstrom durch den Reaktionskolben geleitet wurde. Das Reaktionsgemisch wurde 3 Std gerührt, auf 60 °C abgekühlt und 0,62 mol N-Methylimidazol innerhalb von 45 min langsam zugegeben. Die Reaktion war exotherm und es entstanden Nebel. Anschließend wurde noch 30 min bei 60 °C weitergerührt. Bei Abstellen der Rührung trennten sich 2 Phasen. Die untere Phase wurde abgetrennt und die obere Phase bei 60 °C zweimal mit je 80 ml PCl₃ extrahiert.

Die Unterphase und die vereinigten PCl₃-Extrakte wurden destilliert, wobei man 55 g p-Fluorphenyl-dichlorphosphin in einer Ausbeute von 70 % d. Th. und einer Reinheit von 96 % (bestimmt per ³¹P-NMR) erhielt.

### Beispiele 22 - 27

Es wurde verfahren wie in Beispiel 21 beschrieben, jedoch wurden die in der Tabelle angegebenen Verhältnisse an Fluorbenzol, AlCl₃, PCl₃ und N-Methylimidazol verwendete.

| Bsp. | Molverhältnis AlCl₃: Fluorbenzol | Molverhältnis N-Methylimidazol: AlCl₃ | Reaktionsdauer [h] | Ausbeute [%] | Reinheit [%] |
|---|---|---|---|---|---|
| 21 | 1,5 | 1 | 3 | 70 | 96 |
| 22 | 1,5 | 1 | 6 | 65 | 96 |
| 23 | 1,5 | 1 | 3 | 80 | 91 |
| 24 | 1 | 1 | 3 | 54 | 96 |
| 25 | 1 | 0,5 | 3 | 16 | n.b. |
| 26 | 1,5 | 0,5 | 3 | 19 | n.b. |
| 27 | 2 | 1 | 3 | 79 | 73 |

| | | | | | |
|---|---|---|---|---|---|
| n.b.: nicht bestimmt | | | | | |

In Beispiel 23 wurde die Reaktionsführung analog Beispiel 21 gewählt, jedoch AlCl₃ einer höheren Reinheit (> 99 %) eingesetzt.

### Vergleichsbeispiel 3

In einem 1I-Kolben mit thermostatiertem Doppelmantel, mechanischer Rührung, Thermometer und Rückflußkühler wurden unter Argonatmosphäre ein Gemisch aus 3,4 mol PCl₃ und 1,2 mol AlCl₃ (98% Reinheit) bei 73 °C vorgelegt. Anschließend wurden innerhalb von 30 min 0,8 mol Fluorbenzol hinzugegeben, wobei ein leichter Argonstrom durch den Reaktionskolben geleitet wurde. Das Reaktionsgemisch wurde 3 Std gerührt, auf 60 °C abgekühlt und 1,25 mol Pyridin innerhalb von 45 min langsam zugegeben. Die Reaktion war exotherm und es entstanden Nebel. Anschließend wurde noch 30 min bei 60 °C weitergerührt. Es fiel ein ungleichmäßiger, großklumpiger Feststoff aus, der nicht über eine Nutsche, sondern lediglich durch Filtration abtrennbar war. Der abfiltrierte Rückstand wurde mit Petrolether gewaschen. Das Filtrat und die Waschflüssigkeit wurde vereinigt und destilliert, wobei man 73,3 g p-Fluorphenyldichlorphosphin in einer Ausbeute von 47 % d. Th. erhielt.

### Beispiel 28 - Acetylierung von Pyrrolidin

Zu einer Lösung von 5,33 g (75,0 mmol) Pyrrolidin in 20 mL MTBE (tert.-Butylmethylether) wurde eine Lösung von 5,88 g (75,0 mmol) Acetylchlorid in 10 ml MTBE bei 10 bis 15 °C zugetropft, wobei die Temperatur gehalten wurde. Die entstandene Suspension wurde unter Eiskühlung mit 6,76 g (82,5 mmol) 1-Methylimidazol versetzt und auf 20 °C erwärmt, wobei sich die Suspension in ein flüssiges Zweiphasengemisch verwandelte. Es wurde 1 h nachgerührt und die Phasen getrennt. Die obere Phase wurde am Rotationsverdampfer vom Lösungsmittel befreit und ergab 6,28 g (74,1 %) N-Acetylpyrrolidin. Die untere Phase enthielt neben 1-Methylimidazol-Hydrochlorid weiteres Zielprodukt. Durch zweifache Extraktion der unteren Phase mit Dichlormethan wurden nach Zugabe von Waser nochmals 1,70 g (20,1%) N-Acetylpyrrolidin erhalten.

### Beispiel 29 - Acetylierung von 1-Butanol

Zu einer Lösung von 5,55 g (75,0 mmol) 1-Butanol und 6,67 g (82,5 mmol) 1-Methylimidazol wurden 6,47 g (82,5 mmol) Acetylchlorid unter Rühren und Eiskühlung so zugetropft, dass die Temperatur 10°C nicht überstieg. Danach wurde die Reaktionsmischung auf 75 °C erhitzt, wobei ein flüssiges Zweiphasengemisch entstand. Die abgetrennte obere Phase bestand aus 6,73 g (77,5%) Essigsäure-1-butylester, die It. GC-Analyse ca. 1 % 1-Methylimidazol enthielt. Die untere Phase erstarrte beim Abkühlen auf 20°C.

### Beispiel 30 - Acetylierung von 2-Butanol

Zu einer Lösung von 5,55 g (75,0 mmol) 2-Butanol und 12,3 g (150 mmol) 1-Methylimidazol wurden 6,47 g (82,5 mmol) Acetylchlorid unter Rühren und Eiskühlung so zugetropft, dass die Temperatur 10°C nicht überstieg. Danach wurde 30 min bei 0 °C und 30 min bei 20 °C weitergerührt. Dabei verwandelte sich die zwischenzeitlich entstandene Suspension in ein flüssiges Zweiphasengemisch. Durch Abtrennung der oberen Phase erhielt man 7,90 g (Theorie: 8,68 g) Essigsäure-2-butylester als farbloses Öl mit einer Reinheit von 85% (GC).

### Beispiel 31- Acetylierung von Isobutanol (2-Methylpropan-1-ol)

Zu einer Lösung von 5,55 g (75,0 mmol) Isobutanol und 6,76 g (82,5 mmol) 1-Methylimidazol wurden 6,47 g (82,5 mmol) Acetylchlorid unter Rühren bei 20 °C zugetropft. Das Reaktionsgemisch wurde 30 min weitergerührt und anschließend auf 75 °C erhitzt. Dabei verwandelte sich die zwischenzeitlich entstandene Suspension in ein flüssiges Zweiphasengemisch. Durch Abtrennung der oberen Phase erhielt man 7,01 g (Theorie: 8,68 g) Essigsäureisobutylester als farbloses Öl mit einer Reinheit von 99% (GC).

### Beispiel 32 - Acetylierung von Neopentylalkohol (2,2-Dimethyl-1-propanol)

Zu einer Lösung von 6,61 g (75,0 mmol) Neopentylalkohol (2,2-Dimethyl-1-propanol) und 6,76 g (82,5 mmol) 1-Methylimidazol wurden 6,47 g (82,5 mmol) Acetylchlorid unter Rühren bei 20 °C zugetropft. Das Reaktionsgemisch wurde 30 min weitergerührt und anschließend auf 75 °C erhitzt. Dabei verwandelte sich die zwischenzeitlich entstandene Suspension in ein flüssiges Zweiphasengemisch. Durch Abtrennung der oberen Phase erhielt man 8,40 g (Theorie: 9,76 g) Essigsäureneopentylester als farbloses Öl mit einer Reinheit von 98% (GC).

### Beispiel 33 - Benzoylierung von n-Butanol

Zu einer Lösung von 5,55 g (75,0 mmol) 1-Butanol und 6,76 g (82,5 mmol) 1-Methylimidazol wurden 11,9 g (82,5 mmol) Benzoylchlorid unter Rühren bei 10 °C zugetropft. Das Reaktionsgemisch wurde 30 min weitergerührt und anschließend auf 75 °C erhitzt. Dabei verwandelte sich die zwischenzeitlich entstandene Suspension in ein flüssiges Zweiphasengemisch. Durch Abtrennung der oberen Phase erhielt man 9,90 g (Theorie: 13,3 g) Benzoesäure-1-butylester als farbloses Öl mit einer Reinheit von 99% (GC).

### Beispiel 34 - Palmitoylierung von Prenol

Zu einer Lösung von 6,46 g (75,0 mmol) Prenol (3-Methylbut-2-en-1-ol) und 6,76 g (82,5 mmol) 1-Methylimidazol in 40 mL Toluol wurde eine Lösung von 20,6 g (75,0 mmol) Palmitinsäurechlorid (C16) in 10 mL Toluol unter Rühren bei 20 bis 36 °C zugetropft. Das Reaktionsgemisch wurde 30 min weitergerührt und anschließend auf 80 °C erhitzt. Dabei verwandelte sich die zwischenzeitlich entstandene Suspension in ein flüssiges Zweiphasengemisch. Durch Abtrennung der oberen Phase und Einengen am Rotationsverdampfer erhielt man 23,6 g (Theorie: 24,3 g) Palmitinsäureprenylester als festflüssige Masse mit einer Reinheit von 95% (GC).

### Beispiel 35 - Palmitoylierung von all-trans-Retinol (Vitamin-A-Alkohol, VAA)

Zu einer 29%igen Lösung von all-trans-Retinol in Heptan (608,5 g, 0,616 mol) und 1-Methylimidazol (50,8 g, 0,62 mol) wurde unter Lichtausschluss und unter Kühlung Palmitinsäurechlorid (170,0 g, 0,618 mol) (C16) unter Rühren innerhalb von 25 min zugetropft. Die Reaktionstemperatur stieg bis auf 15°C an. Der Ansatz wurde 30 min bei 2-5°C, dann 30 min bei Raumtemperatur gerührt. Die Mischung wurde auf 90°C erwärmt, wobei sich zwei flüssige Phasen ausbildeten. Die Phasen wurden getrennt. Die obere Phase enthält außer dem Lösungsmittels 0,27% Retinol und 95,2 % Vitamin-A-Palmitat (HPLC).

### Beispiel 36 - Acylierung mit Ethylhexansäurechlorid

Zu einer Lösung von 4-(Hydroxymethyl)-1,3-dioxolan-2-on (20,0 g, 0,169 mol) und 1-Methylimidazol (MIA, 30,6 g, 0,373 mol) in Methylenchlorid (400 ml) wird unter Eisbadkühlung und Stickstoff-Atmosphäre bei einer Temperatur von 10-15°C 2-Ethylhexansäurechlorid (30,0 g, 0,186 mol) langsam zugegeben. Die Reaktionsmischung wird über Nacht gerührt und das Lösungsmittel wird im Vakuum entfernt. Der Rückstand wird zweimal in Methyl-tert-Butylether (MTBE) aufgenommen und die Phasen werden jeweils getrennt. Die organische Oberphase wird im Vakuum eingeengt. Man erhält den Ester als ein farbloses Öl mit Resten MIA. Das Gemisch wird zweimal in Toluol aufgenommen und das Lösungsmittel jeweils im Vakuum entfernt. Man erhält 45,83 g eines gelblichen Öls mit einem Anteil von 17% MIA (NMR).

### Beispiel 37 - Silylierung von n-Butanol

Zu einer Lösung von 3,00 g (40,5 mmol) 1-Butanol und 11,1 g (135 mmol) 1-Methylimidazol wurden 4,40 g (40,5 mmol) Chlortrimethylsilan unter Rühren bei 0 °C zugetropft. Das Reaktionsgemisch wurde 15 min bei 0 bis 5 °C und 15 min bei 20 °C weitergerührt, wobei sich ein flüssiges Zweiphasengemisch bildete. Durch Abtrennung der oberen Phase erhielt man 5,30 g (Theorie: 5,93 g) 1-Trimethylsilyloxybutan als farbloses Öl mit einer Reinheit von 90% (GC).

### Beispiel 38 - Silylierung von 2-Butanol

Zu einer Lösung von 5,00 g (67,5 mmol) 2-Butanol und 6,10 g (74,2 mmol) 1-Methylimidazol wurden 8,06 g (74,2 mmol) Chlortrimethylsilan unter Rühren bei 0 °C zugetropft. Das Reaktionsgemisch wurde 30 min bei 0 °C und 5 min bei 80 °C weitergerührt, wobei sich ein flüssiges Zweiphasengemisch bildete. Durch Abtrennung der oberen Phase erhielt man 8,50 g (Theorie: 9,88 g) 2-Trimethylsilyloxybutan als farbloses, leicht trübes Öl mit einer Reinheit von 96% (GC).

### Beispiel 39 - Silylierung von Neopentylalkohol (2,2-Dimethyl-1-propanol)

Zu einer Lösung von 5,00 g (56,7 mmol) Neopentylalkohol (2,2-Dimethyl-1-propanol) und 11,6 g (142 mmol) 1-Methylimidazol wurden 6,50 g (56,7 mmol) Chlortrimethylsilan unter Rühren bei 0°C zugetropft. Das Reaktionsgemisch wurde 2 h bei 0 °C und 2,5 h bei 20 °C weitergerührt. Durch Abtrennung der oberen Phase erhielt man 7,80 g (Theorie: 9,09 g) 2,2-Dimethyl-1-Trimethylsilyloxypropan als farbloses Öl mit einer Reinheit von 96% (GC).

### Beispiel 40 - Silylierung von Benzylalkohol

Zu einer Lösung von 5,00 g (46,0 mmol) Benzylalkohol und 4,20 g (51,0 mmol) 1 - Methylimidazol wurden 5,50 g (51,0 mmol) Chlortrimethylsilan unter Rühren bei 0 °C zugetropft. Das Reaktionsgemisch wurde 30 min bei 0 °C und 5 min bei 80 °C weitergerührt, wobei sich ein flüssiges Zweiphasengemisch bildete. Durch Abtrennung der oberen Phase erhielt man 7,30 g (Theorie: 8,29 g) Benzyltrimethylsilylether als farbloses Öl mit einer Reinheit von 99% (GC).

### Beispiel 41 - Umsetzung von Ethanol mit Siliciumtetrachlorid

Zu einer Lösung von Ethanol (54,3 g, 1,17 mol) und 1-Methylimidazol (MIA, 98,9 g, 1,21 mol) in Heptan (400 ml) wird unter Eisbadkühlung und N2-Atmosphäre bei einer Temperatur von 10-15°C SiCl₄ (50,0 g, 0,294 mol) langsam zugegeben. Die Reaktionsmischung wird über Nacht gerührt und die Phasen werden getrennt. Man erhält 142,9 g MIA-Hydrochlorid als farblosen Feststoff (Theorie: 141,9 g MIA+MIA•HCl). Die organische Phase wird vorsichtig eingeengt, um Verluste an flüchtigem Produkt gering zu halten. Man erhält 48,1 g Tetraethoxysilan (Theorie: 61,3 g) als leicht trübes, farbloses Öl mit einer Reinheit von 91,1% (GC).

### Beispiel 42 - Silylierung von Acetylaceton

Zu einer Lösung von 5,00 g (49,9 mmol) Acetylaceton und 4,50 g (55,0 mmol) 1-Methylimidazol wurden 5,97 g (55,0 mmol) Chlortrimethylsilan unter Rühren bei 0 °C zugetropft. Das Reaktionsgemisch wurde 1 h bei 0 °C und 5 min bei 80 °C weitergerührt, wobei sich ein flüssiges Zweiphasengemisch bildete. Durch Abtrennung der oberen Phase erhielt man 7,00 g (Theorie: 8,60 g) 4-Trimethylsilyloxypent-3-en-2-on als hellgelbes, trübes Öl mit einer Reinheit von 84% (GC).

### Beispiel 43 - Eliminierung von Bromwasserstoff aus 3-Bromcyclohexen

Eine Lösung aus 10,0 g (62,1 mmol) 3-Bromcyclohexan und 12,4 g (62,2 mmol) N,N-Dibutylpentylamin wurde 1 h bei 120 °C gerührt, auf 25 °C abgekühlt und mit 30 ml n-Pentan versetzt. Die Mischung wurde auf 30 °C erwärmt, wobei sich ein flüssiges Zweiphasengemisch bildete. Die Phasen wurden getrennt und die untere Phase mit 30 ml n-Pentan extrahiert. Die Pentanphasen wurden vereinigt und das Pentan am Rotationsverdampfer abdestilliert (20 °C, 400 - 500 mbar). Es verblieben 3,50 g (Theorie 4,97 g) eines farblosen Rückstandes, der laut GC-Chromatographie überwiegend aus 1,3-Cyclohexadien bestand.

### Beispiel 44 (Vergleich)

### Synthese von Bis(N-3-methylindolyl)chlorphosphin (=Bisskatylchlorphosphin)

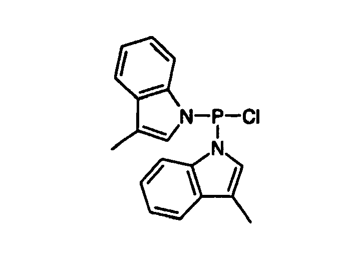

28.5 g (218 mmol) 3-Methylindol (Skatol) wurden in ca. 50 ml getrocknetem Toluol vorgelegt und das Lösungsmittel im Vakuum abdestilliert, um Wasserspuren azeotrop zu entfernen. Dieser Vorgang wurde noch einmal wiederholt. Der Rückstand wurde anschließend in 700 ml getrocknetem Toluol unter Argon aufgenommen und auf -65°C abgekühlt. Dann wurden 14.9 g (109 mmol) PCl₃ und danach 40 g (396 mmol) Triethylamin bei -65°C langsam zugegeben. Das Reaktionsgemisch wurde innerhalb von 16 h auf Raumtemperatur gebracht und danach 16 h unter Rückfluß erhitzt. ³¹P-NMR (Reaktionsmischung, 298K): δ = 102. Reinheit It. ³¹P-NMR ca. 90-95%.

### Beispiel 45 (Vergleich): Synthese von Ligand A

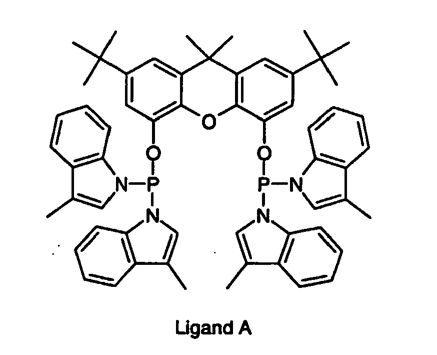

28.5 g (218 mmol) 3-Methylindol (Skatol) wurden in ca. 50 ml getrocknetem Toluol vorgelegt und das Lösungsmittel im Vakuum abdestilliert, um Wasserspuren azeotrop zu entfernen. Dieser Vorgang wurde noch einmal wiederholt. Der Rückstand wurde anschließend in 700 ml getrocknetem Toluol unter Argon aufgenommen und auf -65°C abgekühlt. Dann wurden 14.9 g (109 mmol) PCl₃ und danach 40 g (396 mmol) Triethylamin bei -65°C langsam zugegeben. Das Reaktionsgemisch wurde innerhalb von 16 h auf Raumtemperatur gebracht und danach 16 h unter Rückfluß erhitzt. Zum Reaktionsgemisch wurden 19.3 g (58 mmol) 4,5-Dihydroxy-2,7-di-tert-butyl-9,9-dimethylxanthen in 300 ml getrocknetem Toluol gegeben, danach 16 Stunden unter Rückfluß erhitzt und nach Abkühlung auf Raumtemperatur der ausgefallene farblose Feststoff (Triethylamin-Hydrochlorid) abgesaugt, das Lösungsmittel abdestilliert und der Rückstand zweimal aus heißem Ethanol umkristallisiert. Nach Trocknung im Vakuum wurden 36.3 g (71 % der Theorie) eines farblosen Feststoffes erhalten. ³¹P-NMR (298K): δ =105.

### Beispiel 46: Kontinuierliche Synthese von Bis(3-methylindolyl)chlorphosphin

15.9 g (0.12 mol) 3-Methylindol (Skatol) wurden in 22 g (0.27 mol) 1-Methylimidazol und 69 g getrocknetem Toluol gelöst (Lösung I). Ferner wurden 8.2 g (0.06 mol) Phosphortrichlorid mit 67 g getrocknetem Toluol vermischt (Lösung II). Die beiden Lösungen (I und II) wurden bei 90 °C kontinuierlich in einer Reaktionsmischpumpe gemischt. Strom I wurde mit 1735 ml/h zugefahren, Strom II mit 1235 ml/h. Das Volumen der Mischkammer betrug 3,3 ml. Das System wurde 3 min ins Gleichgewicht gebracht, anschließend der Austrag gesammelt. Der Austrag besteht aus zwei flüssigen Phasen, die durch Dekantieren getrennt wurden. ³¹P-NMR (Rohlösung, 298K): ö= 97. Reinheit It. ³¹P-NMR ca. 95%.

### Beispiel 47: Kontinuierliche Synthese von Ligand A Ansatz:

25.3 g (0.071 mol) 4,5-Dihydroxy-2,7-di-tert-butyl-9,9-dimethylxanthen wurden unter Zusatz von 84.2 g (1.03 mol) 1-Methylimidazol in 84 g Toluol gelöst (Lösung 1). Entsprechend Vorschrift 5.1. wurden 48.7 g Bis(N-3-methylindolyl)chlorphosphin in 84.3 Toluol hergestellt, wobei das bei der Synthese entstandene Ammoniumsalz mittels Schutzgasfritte abgetrennt wurde (Lösung II). Die beiden Lösungen (1 und II) wurden bei 90 °C kontinuierlich in einer Reaktionsmischpumpe gemischt. Strom I wurde mit 1767 ml/h zugefahren, Strom II mit 1203 ml/h. Das Volumen der Mischkammer betrug 3,3 ml, die Verweilzeit dementsprechend ca. 4 s. Das System wurde 3 min ins Gleichgewicht gebracht, anschließend der Austrag gesammelt. Der Austrag besteht aus zwei flüssigen Phasen (N-Methylimidazoliumhydrochlorid und Lösungsmittel/Produkt). Die obere Phase, die das Produkt enthält, wurde abdekantiert und im Vakuum eingeengt. Der Rückstand wurde in Ethanol unter Rückfluß erhitzt und die klare, gelbe Lösung dann auf Raumtemperatur abgekühlt, wobei ein Feststoff ausfällt, der abgesaugt, dann mit Ethanol gewaschen und anschließend im Vakuum getrocknet wurde. Es wurden 27.3 g (41 % d. Th.) eines farblosen Feststoffes erhalten. ³¹P-NMR (CDCl₃, 298K): δ =106.

### Feinreinigung:

Sofern Spuren an N-Methylimidazol Einfluß auf die Katalyse nehmen, so lassen sich diese durch Wäsche einer Lösung des Liganden in einem organischen Lösungsmittel mit Wasser entfernen.

56.8 g des farblosen Feststoffes (Ligand A) wurden in 500 ml Diethylether gelöst und sechsmal mit je 20 ml gesättigter wäßriger Natriumhydrogencarbonat-Lösung gewaschen. Anschließend wurde noch zweimal mit je 15 ml Wasser gewaschen, die organische Phase abgetrennt, flüchtige Bestandteile im Vakuum entfernt und der Rückstand mit 300 ml Ethanol gewaschen. Nach Trocknung im Vakuum wurden 48.1 g eines farblosen Feststoffes erhalten. ³¹P-NMR (CDCl₃, 298K): ö =106.

### Beispiel 48: Kontinuierliche Synthese von Ligand A

### Ansatz:

25.3 g (0.07 mol) 4,5-Dihydroxy-2,7-di-tert-butyl-9,9-dimethylxanthen wurden unter Zusatz von 84 g (1.03 mol) 1-Methylimidazol in 84 g Toluol gelöst (Lösung 1). Entsprechend Vorschrift 5.1. wurden 48.5 g (0.14 mol) Bis(N-3-methylindolyl)chlorphosphin in 84 g Toluol hergestellt, wobei das bei der Synthese entstandene Ammoniumsalz mittels Schutzgasfritte abgetrennt wurde. (Lösung 11). Die beiden Lösungen (I und II) wurden bei 90 °C kontinuierlich in einer Reaktionsmischpumpe gemischt. Strom I wurde mit 589 ml/h zugefahren, Strom II mit 401 ml/h. Das Volumen der Mischkammer betrug 3,3 ml, die Verweilzeit dementsprechend ca. 12 s. Das System wurde 3 min ins Gleichgewicht gebracht, anschließend der Austrag gesammelt. Der Austrag besteht aus zwei flüssigen Phasen (N-Methylimidazoliumhydrochlorid und Lösungsmittel/Produkt). Die obere Phase, die das Produkt enthält, wurde abdekantiert und im Vakuum eingeengt. Der Rückstand wurde in Ethanol unter Rückfluß erhitzt und die klare, gelbe Lösung dann auf Raumtemperatur abgekühlt, wobei ein Feststoff ausfällt, der abgesaugt, dann mit Ethanol gewaschen und anschließend im Vakuum getrocknet wurde. Es wurden 30.5 g (46 % d. Th.) eines farblosen Feststoffes erhalten. ³¹P-NMR (CDCl₃, 298K): δ =106.

### Beispiel 49 (Vergleich): Hydroformylierung von 1-Buten aus konventioneller Synthese (Beispiel 45)

5.5 mg Rh(CO)₂acac (acac=Actetylacetonat) und 200 mg Ligand A wurden separat eingewogen, in je 5 g Toluol gelöst, vermischt und bei 90°C mit 10 bar Synthesegas (CO:H₂ =1:1) begast (Präformierung). Nach 1 h wurde entspannt. Dann wurden 9.9 g 1-Buten mittels Druckschleuse zugegeben, mit Synthesegas (CO:H₂ = 1:1) ein Gesamtdruck von 17 bar eingestellt und 2 h bei 90°C hydroformyliert (109 ppm Rh; Ligand A:Rh =10:1). Nach der angegeben Reaktionszeit wurde der Autoklav abgekühlt, vorsichtig über eine Kühlfalle entspannt und beide Reaktionsausträge (Reaktor und Kühlfalle) mittels Gaschromatographie analysiert. Der Umsatz betrug 99%, die Ausbeute an Valeraldehyd 92% und die Linearität (n-Anteil) 98.5%. Die Linearität (n-Anteil) ist definiert als der Quotient aus n-Valeraldehyd zu der Summe aus n- Valeraldehyd und i-Valeraldehyd multipliziert mit 100.

### Beispiel 50 (Vergleich): Hydroformylierung von 2-Buten CO:H₂= 1:2 aus konventioneller Synthese (Beispiel 45)

5.0 mg Rh(CO)₂acac (acac=Actetylacetonat) und 176 mg Ligand A wurden separat eingewogen, in je 5 g Toluol gelöst, vermischt und bei 90°C mit 10 bar Synthesegas (CO:H₂ =1:2) begast (Präformierung). Nach 1 h wurde entspannt. Dann wurden 11.2 g 2-Buten mittels Druckschleuse zugegeben, mit Synthesegas (CO:H₂ =1:2) ein Gesamtdruck von 17 bar eingestellt. Die Gaszufuhr wurde dann auf Synthesegas (CO:H₂ =1:1) umgestellt. Anschließend wurde 4 h bei 90°C hydroformyliert (93 ppm Rh; Ligand A:Rh = 10:1). Der Umsatz betrug 34%, die Ausbeute an Valeraldehyd 32% und die Linearität (n-Anteil) 93%.

### Beispiel 51: Hydroformylierung von 1-Buten mit Ligand A aus Reaktionsmischpumpe (Beispiel 47)

5 mg Rh(CO)₂acac (acac=Actetylacetonat) und 200 mg Ligand A wurden separat eingewogen, in je 5 g Toluol gelöst, vermischt und bei 90°C mit 10 bar Synthesegas (CO:H₂ =1:1) begast (Präformierung). Nach 1 h wurde entspannt. Dann wurden 12.5 g 1-Buten mittels Druckschleuse zugegeben, mit Synthesegas (CO:H₂ = 1:1) ein Gesamtdruck von 17 bar eingestellt und 2 h bei 90°C hydroformyliert (88 ppm Rh; Ligand A:Rh =11:1). Nach der angegeben Reaktionszeit wurde der Autoklav abgekühlt, vorsichtig über eine Kühlfalle entspannt und beide Reaktionsausträge (Reaktor und Kühlfalle) mittels Gaschromatographie analysiert. Der Umsatz betrug 99%, die Ausbeute an Valeraldehyd 98% und die Linearität (n-Anteil) 96.3%.

### Beispiel 52: Hydroformylierung von 2-Buten mit Ligand A aus Reaktionsmischpumpe (Beispiel 47)

5.0 mg Rh(CO)₂acac (acac=Actetylacetonat) und 118 mg Ligand A wurden separat eingewogen, in je 5 g Toluol gelöst, vermischt und bei 90°C mit 10 bar Synthesegas (CO:H₂ = 1:2) begast (Präformierung). Nach 1 h wurde entspannt. Dann wurden 11.8 g 2-Buten mittels Druckschleuse zugegeben, mit Synthesegas (CO:H₂ =1:2) ein Gesamtdruck von 17 bar eingestellt. Die Gaszufuhr wurde dann auf Synthesegas (CO:H₂ = 1:1) umgestellt. Anschließend wurde 4 h bei 90°C hydroformyliert (91 ppm Rh; Ligand A:Rh = 7:1). Der Umsatz betrug 29%, die Ausbeute an Valeraldehyd 26% und die Linearität (n-Anteil) 93.8%.

### Beispiel 53: Kontinuierliche Synthese von Phenoxyphenylchlorphosphinen

100 g (0,66 mol) 2-tert.-Butylphenol werden unter Zusatz von 54,1 g (0,66 mol) 1-Methyl-imidazol in 102 g Mesitylen gelöst (Lösung 1). Lösung 1 wurde mit 4432,1 ml/h kontinuierlich mit Lösung 11, bestehend aus 121,6 g (0,66 mol) Dichlorphenylphosphin, in einer Reaktionsmischpumpe vermischt. Lösung II wurde mit 1507,9 ml/h zugefahren. Der Kopf der Reaktionsmischpumpe wurde in einem Ölbad auf 100 °C aufgeheizt. Das Volumen der Mischkammer betrug 3,3 ml, die Verweilzeit demnach ca. 2 s. Das System wurde 3 min ins Gleichgewicht gebracht und anschließend der Austrag gesammelt. Der Austrag besteht aus zwei flüssigen Phasen (Produkt/Lösungsmittel und 1-Methylimidazolium Hydrochlorid). Die obere, produkthaltige Phase wurde abdekantiert. GC: 2-tert-Butylphenoxyphenyl-chlorphosphin: 60 FI%.

## Patentansprüche

1. Verfahren zur Herstellung von Aminodihalogenphosphinen, Diaminohalogenphosphinen, Triaminophosphinen, Phosphorigsäuresterdiamiden, Aminophosphinen, Diaminophosphinen, Phosphorigsäuresteramidhalogeniden und Aminophosphinhalogeniden unter Abspaltung einer Säure und in Gegenwart einer Hilfsbase, **dadurch gekennzeichnet, daß** die Hilfsbase
b) ein Salz mit der Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet.

2. Verfahren nach Anspruch 1, **dadurch gekennzeichnet, daß** das Salz der Hilfsbase einen Schmelzpunkt unterhalb von 160 °C aufweist.

3. Verfahren nach einem der vorstehenden Ansprüche, **dadurch gekennzeichnet, daß** das Salz der Hilfsbase einen E_{T}(30)-Wert von mehr als 35 aufweist.

4. Verfahren nach einem der vorstehenden Ansprüche, **dadurch gekennzeichnet, daß** die Base mindestens ein Stickstoffatom enthält.

5. Verfahren nach einem der vorstehenden Ansprüche, **dadurch gekennzeichnet, daß** eine Base eingesetzt wird ausgewählt unter der Formel (la) bis (lr), 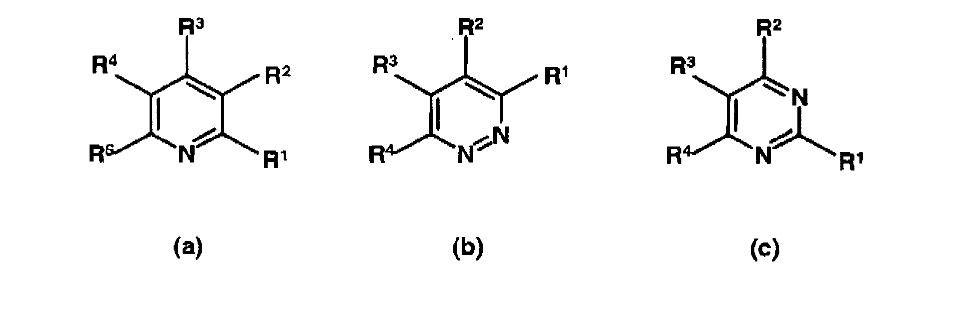 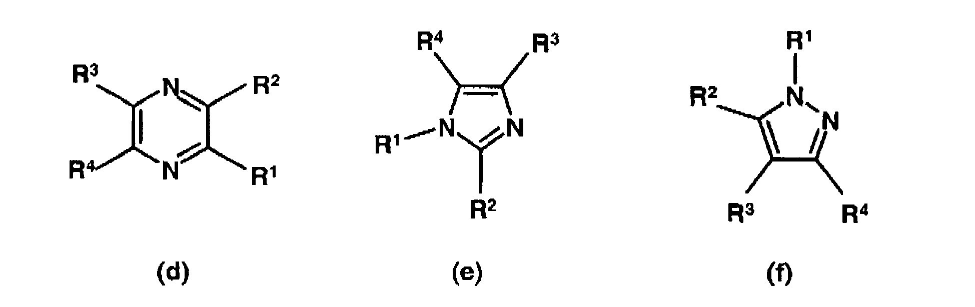 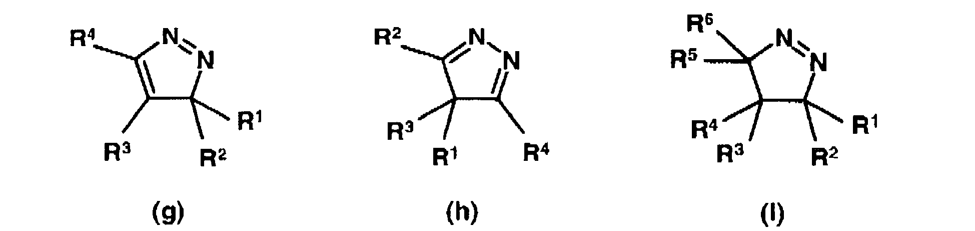 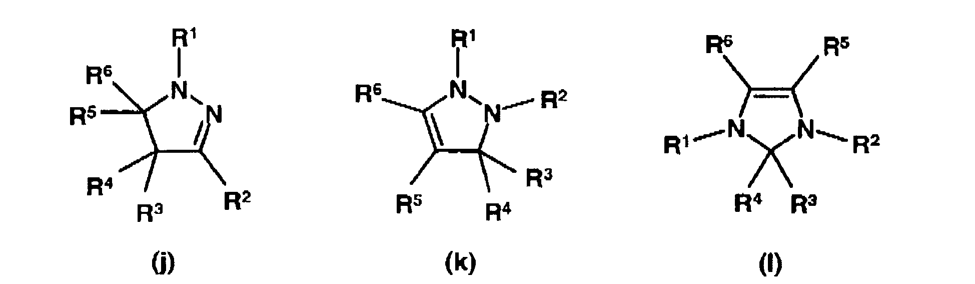 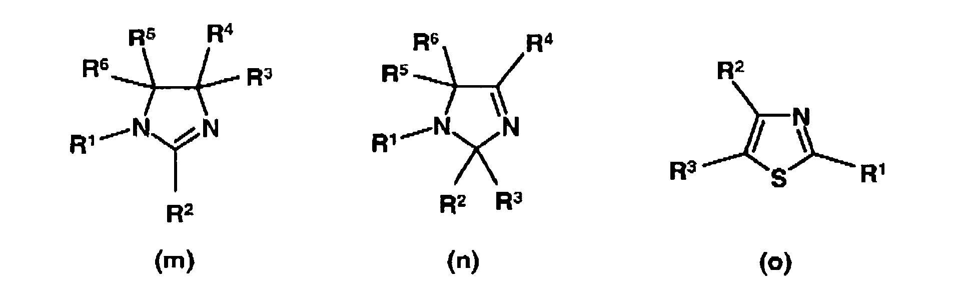 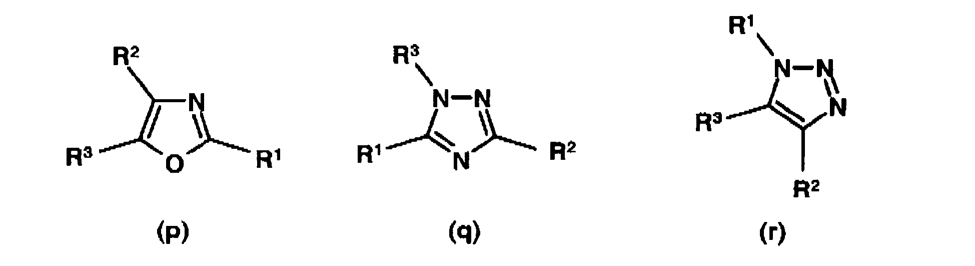 worin
R¹, R², R³, R⁴, R⁵ und R⁶ unabhängig voneinander Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₆ - C₁₂-Aryl, C₅ - C₁₂-Cycloalkyl oder einen fünfbis sechsgliedrigen, Sauerstoff-, Stickstoff- und/oder Schwefelatome aufweisenden Heterocyclus bedeuten, wobei die genannten Reste jeweils durch funktionelle Gruppen, Aryl, Alkyl, Aryloxy, Alkyloxy, Halogen, Heteroatome und/oder Heterocyclen substituiert sein können.

6. Verfahren nach einem der vorstehenden Ansprüche, **dadurch gekennzeichnet, daß** die Hilfsbase 1-n-Butylimidazol, 1-Methylimidazol, 2-Methylpyridin oder 2-Ethylpyridin ist.

7. Verfahren nach einem der Ansprüche 1 bis 4, **dadurch gekennzeichnet, daß** die Hilfsbase Di-n-butyl-n-pentylamin oder 1,5-Diazabicyclo[4,3,0]-non-5-en (DBN) ist.

8. Verfahren nach einem der vorstehenden Ansprüche, **dadurch gekennzeichnet, daß** das Salz der Hilfsbase in dem Wertprodukt oder in der Lösung des Wertproduktes in einem geeigneten Lösungsmittel weniger als 20 Gew% löslich ist.

9. Verfahren nach einem der vorstehenden Ansprüche, **dadurch gekennzeichnet, daß**
Diphosphorigsäuredieesteramide ([N](R'O)P-O.Z-O-P[N'](OR")) Diphosphorigsäureesterdiamide ([N][N']P-O-Z-O-P[N"][N"'])
Bistriaminophosphane ([N][N']P-[N"]-Z-[N"']-P[N""][N""'])
oder Systeme der Formeln
[N](R'O)P-O-Z-O-P(OR")(OR"'),
[N][N']P-O-Z-O-P(OR")(OR"') oder
[N][N']P-O-Z-O-P[N"](OR''')
oder am Phosphor jeweils Stickstoff-und Kohlenstoff-substituierte Systeme der Formeln
[N](R')P-O-Z-O-P[N'](R"') oder
[N](R')P-[N"]-Z-[N'"]-P[N'](R'")
oder Systeme der Formel
[N](R'O)P-O-Z-O-P[N'](R"')
hergestellt werden,
worin R, R', R" und R'" beliebige organische Reste sein können, die jeweils gleich oder verschieden sein können, [N], [N'], [N"], [N"'], [N""] und [N""'] für un-, mono- oder disubstituierte Aminogruppen stehen, die jeweils gleich oder verschieden sein können, und Z eine beliebige bivalente Brücke sein kann

10. Verfahren zur Herstellung von Phosphorverbindungen aus den jeweiligen Edukten gemäß einem der vorstehenden Ansprüche, **dadurch gekennzeichnet, daß** man die Herstellung kontinuierlich bei einer Temperatur von 30 °C bis 190 °C und einer Verweilzeit von 1 Sekunde bis 1 Stunde durchführt.

11. Verfahren zur Abtrennung von Säuren aus Reaktionsgemischen mittels einer Hilfsbase, **dadurch gekennzeichnet, daß** die Hilfsbase
b) ein Salz mit der Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet, wobei aus dem Reaktionsgemisch zunächst das Wertprodukt in Anwesenheit der Hilfsbase in der protonierten Form abdestilliert wird und anschließend die Hilfsbase mit einer starken Base freigesetzt wird und im Anschluß die freigesetzte Hilfsbase destilliert wird.

12. Verfahren zur Abtrennung von Säuren aus Reaktionsgemischen mittels einer Hilfsbase, **dadurch gekennzeichnet, daß** die Hilfsbase
b) ein Salz mit der Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet, wobei zunächst die Hilfsbase mit einer starken Base freigesetzt wird und im Anschluß die freigesetzte Hilfsbase in Gegenwart des Wertprodukts destilliert wird und anschließend das Wertprodukt destilliert wird.

13. Verfahren zum Stoppen sauer katalysierter Reaktionen, wobei der saure Katalysator in einer chemischen Umsetzung mit einer Hilfsbase neutralisiert wird, **dadurch gekennzeichnet, daß** die Hilfsbase
b) ein Salz mit der Säure bildet, das bei Temperaturen flüssig ist, bei denen das Wertprodukt während der Abtrennung des Flüssigsalzes nicht signifikant zersetzt wird und
c) das Salz der Hilfsbase mit dem Wertprodukt oder der Lösung des Wertproduktes in einem geeigneten Lösungsmittel zwei nicht mischbare flüssige Phasen ausbildet.

## Claims

1. A process for preparing aminodihalophosphines, diaminohalophosphines, triaminophosphines, phosphorous ester diamides, aminophosphines, diaminophosphines, phosphorous ester amide halides and aminophosphine halides with elimination of an acid in the presence of an auxiliary base, wherein
b) the auxiliary base and the acid form a salt which is liquid at temperatures at which the desired product is not significantly decomposed during the process of separating off the liquid salt and
c) the salt of the auxiliary base forms two immiscible liquid phases with the desired product or the solution of the desired product in a suitable solvent.

2. The process according to claim 1, wherein the salt of the auxiliary base has a melting point below 160°C.

3. The process according to any of the preceding claims, wherein the salt of the auxiliary base has an E_{T}(30) of more than 35.

4. The process according to any of the preceding claims, wherein the base comprises at least one nitrogen atom.

5. The process according to any of the preceding claims, wherein the base used is selected from among compounds of the formulae (Ia) to (Ir), 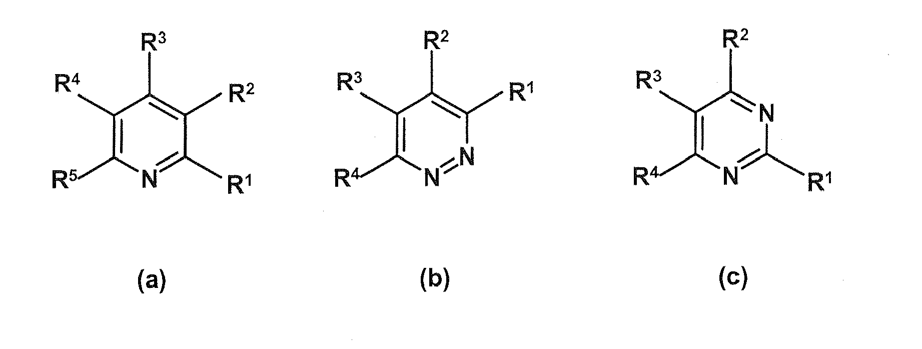 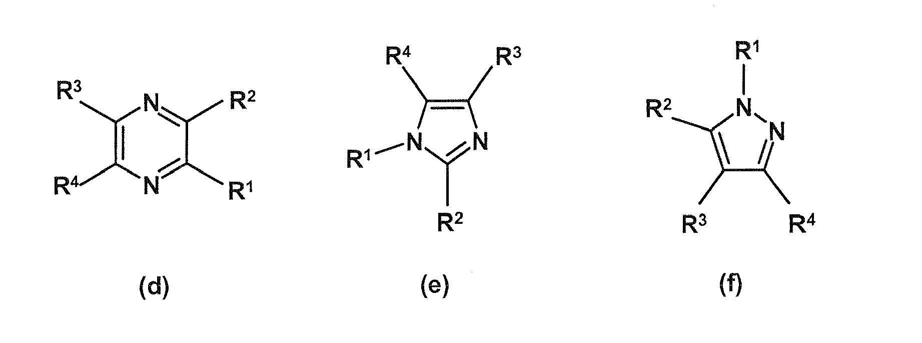 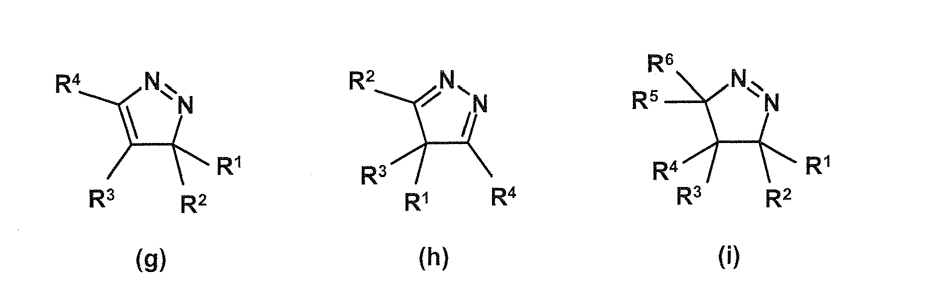 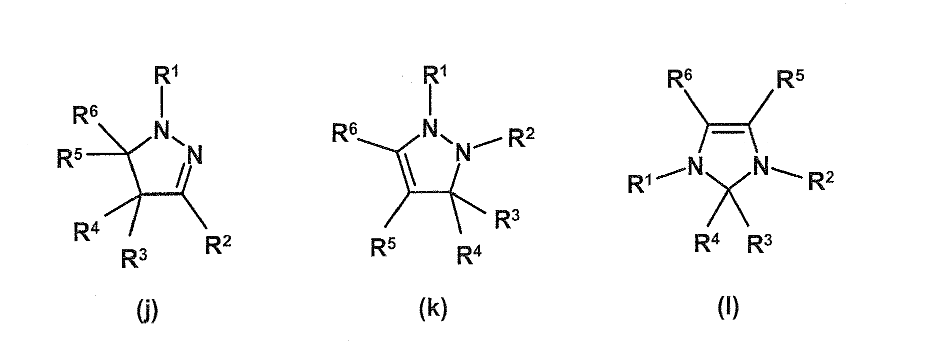 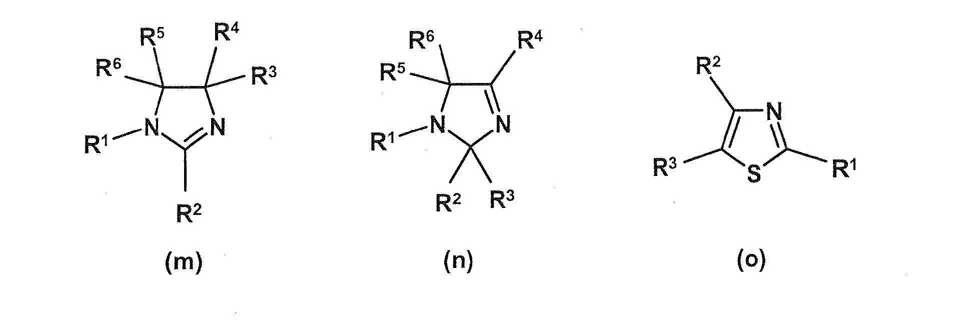 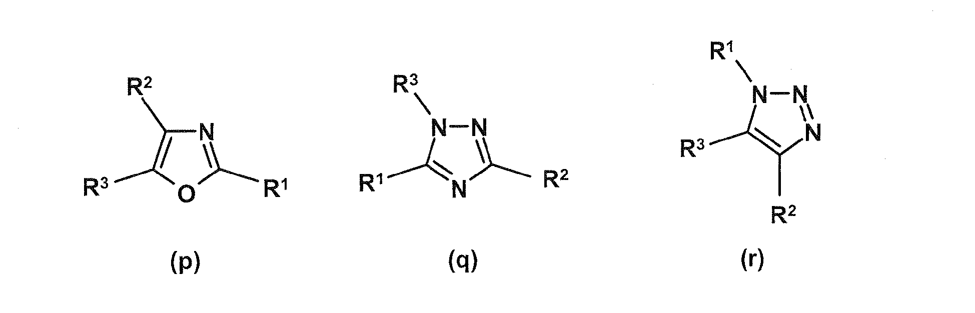 where
R¹, R², R³, R⁴, R⁵ and R⁶ are each, independently of one another, hydrogen, C₁-C₁₈-alkyl, C₂-C₁₈-alkyl which may optionally be interrupted by one or more oxygen and/or sulfur atoms and/or one or more substituted or unsubstituted imino groups, C₆-C₁₂-aryl, C₅-C₁₂-cycloalkyl or a five- to six-membered, oxygen-, nitrogen- and/or sulfur-containing heterocycle, where each of the abovementioned radicals may be substituted by functional groups, aryl, alkyl, aryloxy, alkyloxy, halogen, heteroatoms and/or heterocycles.

6. The process according to any of the preceding claims, wherein the auxiliary base is 1-n-butylimidazole, 1-methylimidazole, 2-methylpyridine or 2-ethylpyridine.

7. The process according to any of claims 1 to 4, wherein the auxiliary base is di-n-butyl-n-pentylamine or 1,5-diazabicyclo[4.3.0]non-5-ene (DBN).

8. The process according to any of the preceding claims, wherein the salt of the auxiliary base is soluble to an extent of less than 20% by weight in the desired product or in the solution of the desired product in a suitable solvent.

9. The process according to any of the preceding claims, wherein diphosphorous diester amides ([N](R'O)P-O-Z-O-P[N'](OR")),
diphosphorous ester diamides ([N][N']P-O-Z-O-P[N"][N"']),
bistriaminophosphines ([N][N']P-[N"]-Z-[N"']-P[N""][N""']),
or systems of the formula
[N](R'O)P-O-Z-O-P(OR")(OR"'),
[N][N']P-O-Z-O-P(OR")(OR"') or
[N][N']P-O-Z-O-P[N"](OR"')
or systems which are both nitrogen- and carbon-substituted on each phosphorus and have the formula
[N](R')P-O-Z-O-P[N'](R"') or
[N](R')P-[N"]-Z-[N'"]-P[N'](R'")
or systems of the formula
[N](R'O)P-O-Z-O-P[N'](R"')
are prepared,
where R, R', R" and R'" can be any organic radicals which may be identical or different, [N], [N'], [N"], [N"'], [N""] and [N""'] are unsubstituted, monosubstituted or disubstituted amino groups which may be identical or different and Z can be any divalent bridge.

10. A process for preparing phosphorus compounds from the appropriate starting materials as set forth in any of the preceding claims, wherein the preparation is carried out continuously at from 30°C to 190°C and a residence time of from 1 second to 1 hour.

11. A process for separating acids from reaction mixtures by means of an auxiliary base, wherein
b) the auxiliary base and the acid form a salt which is liquid at temperatures at which the desired product is not significantly decomposed during the process of separating off the liquid salt and
c) the salt of the auxiliary base forms two immiscible liquid phases with the desired product or the solution of the desired product in a suitable solvent, and the desired product is firstly distilled off from the reaction mixture in the presence of the auxiliary base in the protonated form and the auxiliary base is then set free by means of a strong base and the free auxiliary base is subsequently distilled.

12. A process for separating acids from reaction mixtures by means of an auxiliary base, wherein
b) the auxiliary base and the acid form a salt which is liquid at temperatures at which the desired product is not significantly decomposed during the process of separating off the liquid salt and
c) the salt of the auxiliary base forms two immiscible liquid phases with the desired product or the solution of the desired product in a suitable solvent, and the auxiliary base is firstly set free by means of a strong base and the free auxiliary base is subsequently distilled in the presence of the desired product and the desired product is then distilled.

13. A method of stopping acid-catalyzed reactions, which comprises neutralizing the acid catalyst in a chemical reaction with an auxiliary base, wherein
b) the auxiliary base and the acid form a salt which is liquid at temperatures at which the desired product is not significantly decomposed during the process of separating off the liquid salt and
c) the salt of the auxiliary base forms two immiscible liquid phases with the desired product or the solution of the desired product in a suitable solvent.

## Revendications

1. Procédé de préparation d'aminodihalogénophosphines de diaminohalogénophosphines, de triaminophosphines, de diamides d'ester d'acide phosphoreux, d'aminophosphines, de diaminophosphines, d'halogénures d'amide d'ester d'acide phosphoreux et d'halogénures d'aminophosphine, avec séparation d'un acide et en présence d'une base auxiliaire, **caractérisé en ce que**
b) la base auxiliaire forme avec l'acide un sel qui est liquide à des températures auxquelles le produit de valeur n'est pas décomposé de manière significative pendant l'isolement du sel liquide, et
c) le sel de la base auxiliaire forme avec le produit de valeur ou la solution du produit de valeur dans un solvant approprié deux phases liquides non miscibles.

2. Procédé suivant la revendication 1, **caractérisé en ce que** le sel de la base auxiliaire présente un point de fusion inférieur à 160°C.

3. Procédé suivant l'une des revendications précédentes, **caractérisé en ce que** le sel de la base auxiliaire présente une valeur E_{T}(30) supérieure à 35.

4. Procédé suivant l'une des revendications précédentes, **caractérisé en ce que** la base contient au moins un atome d'azote.

5. Procédé suivant l'une des revendications précédentes, **caractérisé en ce qu'**on met en oeuvre une base choisie parmi les formules (Ia) à (Ir) : 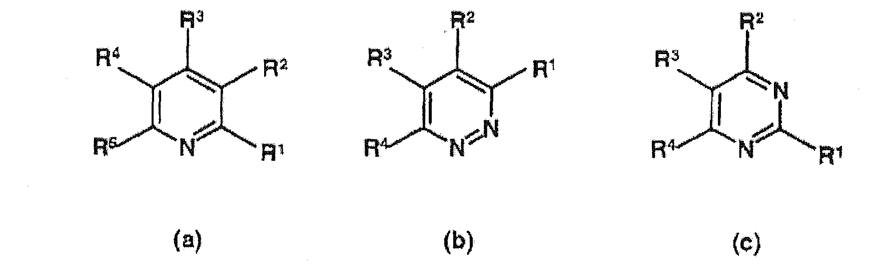 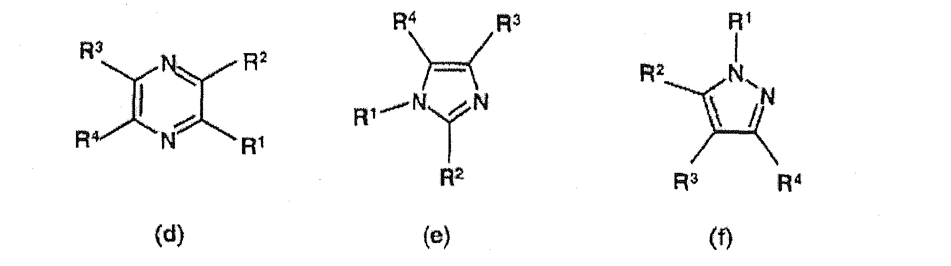 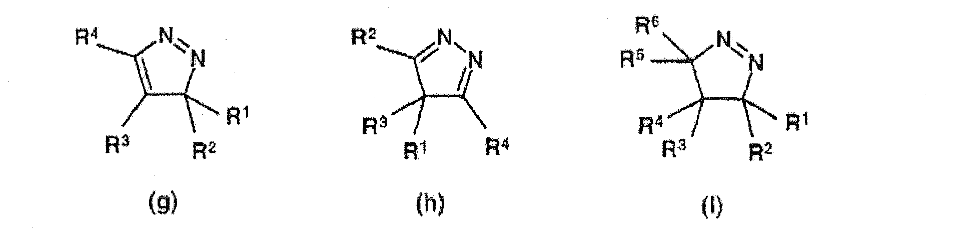 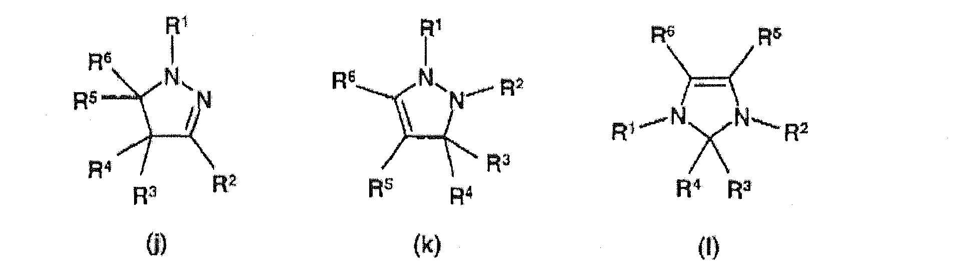 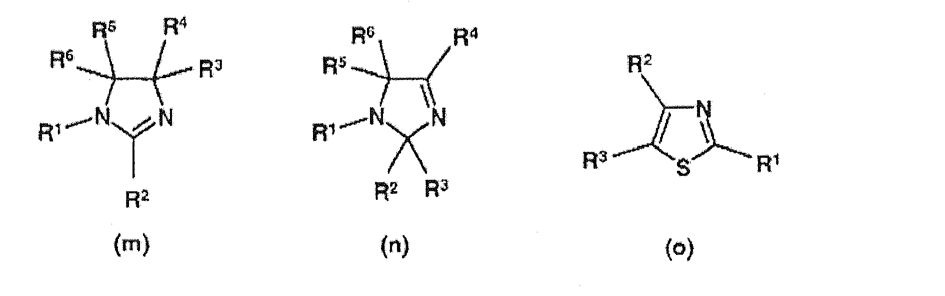 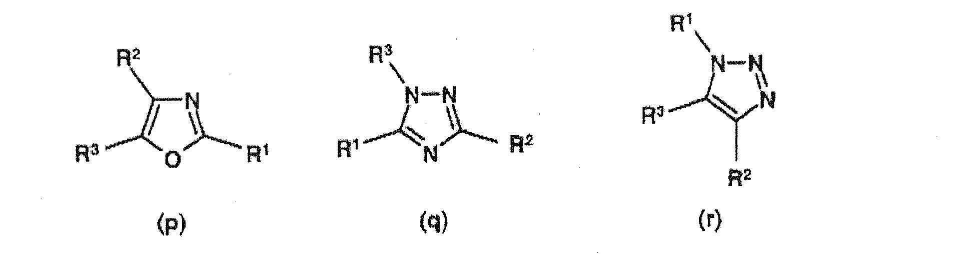 dans lesquelles
R¹, R², R³, R⁴, R⁵ et R⁶ représentent indépendamment l'un de l'autre de l'hydrogène, un groupe alkyle en C₁-C₁₈, alkyle en C₂-C₁₈ éventuellement interrompu par un ou plusieurs atomes d'oxygène et/ou de soufre et/ou un ou plusieurs groupes imino substitués ou non substitués, aryle en C₆-C₁₂, cycloalkyle en C₅-C₁₂ ou un hétérocycle pentagonal à hexagonal, présentant des atomes d'oxygène, d'azote et/ou de soufre, les radicaux cités pouvant être chacun substitués par des groupes fonctionnels, aryle, alkyle, aryloxy, alkyloxy, de l'halogène, des hétéroatomes et/ou des hétérocycles.

6. Procédé suivant l'une des revendications précédentes, **caractérisé en ce que** la base auxiliaire est du 1-n-butylimidazole, du 1-méthylimidazole, de la 2-méthylpyridine ou de la 2-éthylpyridine.

7. Procédé suivant l'une des revendications 1 à 4, **caractérisé en ce que** la base auxiliaire est de la di-n-butyl-n-pentylamine ou du 1,5-diazabicyclo [4 . 3 . 0] -non-5-ène (DBN).

8. Procédé suivant l'une des revendications précédentes, **caractérisé en ce que** le sel de la base auxiliaire est soluble à moins de 20 % en poids dans le produit de valeur ou dans la solution du produit de valeur dans un solvant approprié.

9. Procédé suivant l'une des revendications précédentes, **caractérisé en ce que**
des amides de diester d'acide diphosphoreux
([N] (R'O)P-O-Z-O-P[N'] (OR")),
des diamides d'ester d'acide diphosphoreux
([N] [N'] P-O-Z-O-P [N''] [N" ']),
des bistriaminophosphanes ( [N] [N'] P- [N"] -Z- [N" ']-P [N" "] [N" " ']),
ou des systèmes des formules
[N] (R'O) P-O-Z-O-P (OR") (OR" ')
[N] [N']P-O-Z-O-P(OR") (OR" ') ou
[N] [N'] P-O-Z-O-P [N"] (OR" ')
ou des systèmes des formules
[N](R')P-O-Z-O-P[N'] (R" ') ou
[N] (R') P- [N"] -Z- [N" '] -P [N' ] (R" ')
substitués respectivement par de l'azote et du carbone sur le phosphore
ou des systèmes de la formule
[N] (R'O) P-O-2-O-P [N'] (R" ')
sont préparés,
formules dans lesquelles R, R', R" et R"' peuvent être des radicaux organiques quelconques qui peuvent être chaque fois identiques ou différents, [N], [N'], [N"], [N"'], [N" "] et [N" " '] représentent des groupes amino non substitués, monosubstitués ou disubstitués, qui peuvent chacun être identiques ou différents, et Z peut être un pont bivalent quelconque.

10. Procédé de préparation de composés de phosphore à partir des produits respectifs suivant l'une des revendications précédentes, **caractérisé en ce qu'**on effectue la préparation de manière continue à une température de 30°C à 190°C et pendant une durée de séjour de 1 seconde à 1 heure.

11. Procédé d'isolement d'acides à partir de mélanges réactionnels au moyen d'une base auxiliaire, **caractérisé en ce que**
b) la base auxiliaire forme avec l'acide un sel qui est liquide à des températures auxquelles le produit de valeur n'est pas décomposé de manière significative pendant l'isolement du sel liquide, et
c) le sel de la base auxiliaire forme avec le produit de valeur ou la solution du produit de valeur dans un solvant approprié deux phases liquides non miscibles, le produit de valeur étant tout d'abord séparé du mélange réactionnel par distillation sous la forme protonée en présence de la base auxiliaire et la base auxiliaire étant ensuite libérée par une base forte et la base auxiliaire libérée étant ensuite distillée.

12. Procédé d'isolement d'acides à partir de mélanges réactionnels au moyen d'une base auxiliaire, **caractérisé en ce que**
b) la base auxiliaire forme avec l'acide un sel qui est liquide à des températures auxquelles le produit de valeur n'est pas décomposé de manière significative pendant l'isolement du sel liquide, et
c) le sel de la base auxiliaire forme avec le produit de valeur ou la solution du produit de valeur dans un solvant approprié deux phases liquides non miscibles, la base auxiliaire étant tout d'abord libérée par une base forte et la base auxiliaire libérée étant ensuite distillée en présence du produit de valeur et ensuite le produit de valeur étant distillé.

13. Procédé pour stopper des réactions à catalyse acide, dans lequel le catalyseur acide est neutralisé dans une réaction chimique par une base auxiliaire, **caractérisé en ce que**
b) la base auxiliaire forme avec l'acide un sel qui est liquide à des températures auxquelles le produit de valeur n'est pas décomposé de manière significative pendant l'isolement du sel liquide, et
c) le sel de la base auxiliaire forme avec le produit de valeur ou la solution du produit de valeur dans un solvant approprié deux phases liquides non miscibles.
